# EUROPEAN PATENT SPECIFICATION

(11) **EP 1 807 521 B1**
(45) Date of publication and mention of the grant of the patent: **29.12.2010**
(21) Application number: 05807978.1
(22) Date of filing: 27.10.2005
(51) Int. Cl.: C12N 15/82, C12N 15/29

(54) **PLANTS HAVING IMPROVED GROWTH CHARACTERISTICS AND METHOD FOR MAKING THE SAME**
PFLANZEN MIT VERÄNDERTEN WACHSTUMSEIGENSCHAFTEN UND VERFAHREN ZU DEREN HERSTELLUNG
PLANTES À CARACTÉRISTIQUES DE CROISSANCE AMÉLIORÉES ET PROCÉDÉ DE PRODUCTION DESDITES PLANTES

(30) Priority: 29.10.2004 EP 04105406; 04.11.2004 US 624892 P
(43) Date of publication of application: 18.07.2007
(73) Proprietor: CropDesign N.V., 9052 Zwijnaarde (BE)
(72) Inventor: SANZ MOLINERO, Ana Isabel, B-9050 Gentbrugge (BE)
(74) Representative: Wolf, Christian
(86) International application number: PCT/EP2005/055590
(87) International publication number: WO 2006/045829

(56) References cited:
- WO-A-96/05722
- RUS ANA ET AL: "AtHKT1 facilitates Na+ homeostasis and K+ nutrition in planta" PLANT PHYSIOLOGY (ROCKVILLE), vol. 136, no. 1, September 2004 (2004-09), pages 2500-2511, XP002327666 ISSN: 0032-0889
- LAURIE SOPHIE ET AL: "A role for HKT1 in sodium uptake by wheat roots" PLANT JOURNAL, vol. 32, no. 2, October 2002 (2002-10), pages 139-149, XP002327667 ISSN: 0960-7412 cited in the application
- RUS ANA ET AL: "AtHKT1 is a salt tolerance determinant that controls Na+ entry into plant roots" PROCEEDINGS OF THE NATIONAL ACADEMY OF SCIENCES OF THE UNITED STATES OF AMERICA, vol. 98, no. 24, 20 November 2001 (2001-11-20), pages 14150-14155, XP002327668 ISSN: 0027-8424
- HORIE TOMOAKI ET AL: "Two types of HKT transporters with different properties of Na+ and K+ transport in Oryza sativa" PLANT JOURNAL, vol. 27, no. 2, July 2001 (2001-07), pages 129-138, XP002327669 ISSN: 0960-7412
- SCHACHTMAN ET AL: "Structure and transport mechanism of a high-affinity potassium uptake transporter from higher plants" NATURE, MACMILLAN JOURNALS LTD. LONDON, GB, vol. 370, 25 August 1994 (1994-08-25), pages 655-658, XP002086588 ISSN: 0028-0836
- MASER P ET AL: "Altered shoot/root Na<+> distribution and bifurcating salt sensitivity in Arabidopsis by genetic disruption of the Na<+> transporter AtHKT1" FEBS LETTERS, ELSEVIER SCIENCE PUBLISHERS, AMSTERDAM, NL, vol. 531, no. 2, 6 November 2002 (2002-11-06), pages 157-161, XP004391623 ISSN: 0014-5793
- VÉRY ANNE-ALIÉNOR ET AL: "Molecular mechanisms and regulation of K+ transport in higher plants." ANNUAL REVIEW OF PLANT BIOLOGY. 2003, vol. 54, 2003, pages 575-603, XP002327670 ISSN: 1543-5008
- REN ZHONG-HAI ET AL: "A rice quantitative trait locus for salt tolerance encodes a sodium transporter" NATURE GENETICS, vol. 37, no. 10, October 2005 (2005-10), pages 1141-1146, XP002367318 ISSN: 1061-4036

### SEQUENCE LISTING

<110> CropDesign N.V.
<120> Plants having improved growth characteristics and method for making the same
<130> CD-125-PCT
<150> EP 04105406.5
   <151> 2004-10-29
<150> US 60/624,892
   <151> 2004-11-04
<160> 45
<170> PatentIn version 3.3
<210> 1
   <211> 1521
   <212> DNA
   <213> Arabidopsis thaliana
<400> 1
   atggacagag tggtggcaaa aatagcaaaa atccgttcgc agcttactaa attacgttca      60
   ctattcttcc tttacttcat ctacttcttg ttcttctcct ttttagggtt tttggcactc     120
   aagatcacaa agccaagaac cacttcacgt cctcatgact ttgacctttt cttcacttct     180
   gtctctgcca tcaccgtctc ttccatgtct accgtcgaca tggaagtctt ctccaacacc     240
   caacttatct tcctcactat cctcatgttc ctcggtggcg aaatcttcac ctcctttctc     300
   aacctctacg tctcctattt caccaagttc gtcttccctc ataacaagat tagacatatt     360
   ttgggatctt ataattcgga cagttccatc gaggatcgct gtgacgttga gactgttact     420
   gattatcgcg agggtcttat caagatcgat gaaagggcat ctaagtgctt gtactcggtg     480
   gttcttagtt accatcttgt tactaaccta gttggctctg tgttgcttct tgtgtacgta     540
   aattttgtta aaacggcgag agatgttctt agttccaaag aaatctcacc tctcactttc     600
   tccgtcttca caactgtttc cacgtttgca aactgcggat ttgtccccac gaatgagaac     660
   atgatcatct ttcgcaagaa ctctggtctc atctggctcc taatccctca agtactgatg     720
   ggaaacactt tgttcccttg cttcttggtt ttgctcatat ggggacttta taagatcaca     780
   aagcgtgacg agtatggtta cattctcaag aaccacaata agatgggata ctctcatcta     840
   ctctcggttc gtctatgtgt tcttcttgga gtgacggtgc tagggtttct gataatacag     900
   cttcttttct tctgcgcctt tgaatggacc tctgagtctc tagaaggaat gagttcgtac     960
   gagaagttgg ttggatcgtt gtttcaagtg gtgaattcgc gacacaccgg agaaactata    1020
   gtagacctct ctacactttc cccagctatc ttggtactct ttattcttat gatgtatctt    1080
   cctccataca ctttatttat gccgttgacg gaacaaaaga cgatagagaa agaaggagga    1140
   gatgatgatt ccgaaaatgg aaagaaagtt aaaaagagtg gactcatcgt gtcacaactt    1200
   tcctttttga cgatatgtat ctttctcatt tcaatcaccg aaaggcaaaa tctacaacgt    1260
   gatccgataa atttcaacgt ccttaacatc actctcgaag ttatcagtgc atatggaaac    1320
   gttggtttca ctaccgggta cagctgtgaa cggcgtgtgg acatcagcga tggtggctgc    1380
   aaagacgcga gttatgggtt tgcaggacga tggagtccaa tgggaaaatt cgtactaata    1440
   atagtaatgt tttatggtag gtttaagcag ttcacagcca aatctggccg cgcatggatt    1500
   ctttacccct cgtcttccta a                                              1521
<210> 2
   <211> 506
   <212> PRT
   <213> Arabidopsis thaliana
<400> 2
   Met Asp Arg Val Val Ala Lys Ile Ala Lys Ile Arg Ser Gln Leu Thr
   1               5                   10                  15
   Lys Leu Arg Ser Leu Phe Phe Leu Tyr Phe Ile Tyr Phe Leu Phe Phe
               20                  25                  30
   Ser Phe Leu Gly Phe Leu Ala Leu Lys Ile Thr Lys Pro Arg Thr Thr
           35                  40                  45
   Ser Arg Pro His Asp Phe Asp Leu Phe Phe Thr Ser Val Ser Ala Ile
       50                  55                  60
   Thr Val Ser Ser Met Ser Thr Val Asp Met Glu Val Phe Ser Asn Thr
   65                  70                  75                  80
   Gln Leu Ile Phe Leu Thr Ile Leu Met Phe Leu Gly Gly Glu Ile Phe
                   85                  90                  95
   Thr Ser Phe Leu Asn Leu Tyr Val Ser Tyr Phe Thr Lys Phe Val Phe
               100                 105                 110
   Pro His Asn Lys Ile Arg His Ile Leu Gly Ser Tyr Asn Ser Asp Ser
           115                 120                 125
   Ser Ile Glu Asp Arg Cys Asp Val Glu Thr Val Thr Asp Tyr Arg Glu
       130                 135                 140
   Gly Leu Ile Lys Ile Asp Glu Arg Ala Ser Lys Cys Leu Tyr Ser Val
   145                 150                 155                 160
   Val Leu Ser Tyr His Leu Val Thr Asn Leu Val Gly Ser Val Leu Leu
                   165                 170                 175
   Leu Val Tyr Val Asn Phe Val Lys Thr Ala Arg Asp Val Leu Ser Ser
               180                 185                 190
   Lys Glu Ile Ser Pro Leu Thr Phe Ser Val Phe Thr Thr Val Ser Thr
           195                 200                 205
   Phe Ala Asn Cys Gly Phe Val Pro Thr Asn Glu Asn Met Ile Ile Phe
       210                 215                 220
   Arg Lys Asn Ser Gly Leu Ile Trp Leu Leu Ile Pro Gln Val Leu Met
   225                 230                 235                 240
   Gly Asn Thr Leu Phe Pro Cys Phe Leu Val Leu Leu Ile Trp Gly Leu
                   245                 250                 255
   Tyr Lys Ile Thr Lys Arg Asp Glu Tyr Gly Tyr Ile Leu Lys Asn His
               260                 265                 270
   Asn Lys Met Gly Tyr Ser His Leu Leu Ser Val Arg Leu Cys Val Leu
           275                 280                 285
   Leu Gly Val Thr Val Leu Gly Phe Leu Ile Ile Gln Leu Leu Phe Phe
       290                 295                 300
   Cys Ala Phe Glu Trp Thr Ser Glu Ser Leu Glu Gly Met Ser Ser Tyr
   305                 310                 315                 320
   Glu Lys Leu Val Gly Ser Leu Phe Gln Val Val Asn Ser Arg His Thr
                   325                 330                 335
   Gly Glu Thr Ile Val Asp Leu Ser Thr Leu Ser Pro Ala Ile Leu Val
               340                 345                 350
   Leu Phe Ile Leu Met Met Tyr Leu Pro Pro Tyr Thr Leu Phe Met Pro
           355                 360                 365
   Leu Thr Glu Gln Lys Thr Ile Glu Lys Glu Gly Gly Asp Asp Asp Ser
       370                 375                 380
   Glu Asn Gly Lys Lys Val Lys Lys Ser Gly Leu Ile Val Ser Gln Leu
   385                 390                 395                 400
   Ser Phe Leu Thr Ile Cys Ile Phe Leu Ile Ser Ile Thr Glu Arg Gln
                   405                 410                 415
   Asn Leu Gln Arg Asp Pro Ile Asn Phe Asn Val Leu Asn Ile Thr Leu
               420                 425                 430
   Glu Val Ile Ser Ala Tyr Gly Asn Val Gly Phe Thr Thr Gly Tyr Ser
           435                 440                 445
   Cys Glu Arg Arg Val Asp Ile Ser Asp Gly Gly Cys Lys Asp Ala Ser
       450                 455                 460
   Tyr Gly Phe Ala Gly Arg Trp Ser Pro Met Gly Lys Phe Val Leu Ile
   465                 470                 475                 480
   Ile Val Met Phe Tyr Gly Arg Phe Lys Gln Phe Thr Ala Lys Ser Gly
                   485                 490                 495
   Arg Ala Trp Ile Leu Tyr Pro Ser Ser Ser
               500                 505
<210> 3
   <211> 3817
   <212> DNA
   <213> Arabidopsis thaliana
<400> 3
   gcttaaaccg actcgagaac taaaatggac agagtggtgg caaaaatagc aaaaatccgt     60
   tcgcagctta ctaaattacg ttcactattc ttcctttact tcatctactt cttgttcttc    120
   tcctttttag ggtttttggc actcaagatc acaaagccaa gaaccacttc acgtcctcat    180
   gactttgacc ttttcttcac ttctgtctct gccatcaccg tctcttccat gtctaccgtc    240
   gacatggaag tcttctccaa cacccaactt atcttcctca ctatcctcat gttcctcggt    300
   ggcgaaatct tcacctcctt tctcaacctc tacgtctcct atttcaccaa gttcgtcttc    360
   cctcataaca agattagaca tattttggga tcttataatt cggacagttc catcgaggat    420
   cgctgtgacg ttgagactgt tactgattat cgcgagggtc ttatcaagat cgatgaaagg    480
   gcatctaagt gcttgtactc ggtggttctt agttaccatc ttgttactaa cctagttggc    540
   tctgtgttgc ttcttgtgta cgtaaatttt gttaaaacgg cgagagatgt tcttagttcc    600
   aaagaaatct cacctctcac tttctccgtc ttcacaactg tttccacgtt tgcaaactgc    660
   ggatttgtcc ccacgaatga gaacatgatc atctttcgca agaactctgg tctcatctgg    720
   ctcctaatcc ctcaagtact gatgggaaac actttgttcc cttgcttctt ggttttgctc    780
   atatggggac tttataagat cacaaagcgt gacgagtatg gttacattct caagaaccac    840
   aataagatgg gatactctca tctactctcg gttcgtctat gtgttcttct tggagtgacg    900
   gtgctagggt ttctgataat acagcttctt ttcttctgcg cctttgaatg gacctctgag    960
   tctctagaag gaatgagttc gtacgagaag ttggttggat cgttgtttca agtggtgaat   1020
   tcgcgacaca ccggagaaac tatagtagac ctctctacac tttccccagc tatcttggta 1080
   ctctttattc ttatgatgta agtttctttc aatcctctcc tcatatatcg aatcatttgc 1140
   atgaactcga aatatttaga tcgaaaggtg cacttatttc gacataattt tcacatatga 1200
   ggtgatatgc atgcacacat aggtgcagtt ttacgaatac attaaactaa aacaattgag 1260
   atatagtgaa attaaatggt tgacatttca ctacagaaac aataaaaagt caccgcatga 1320
   attaaagttg gttgacattt cacaacagaa acaccaaaca tattattatc tcacgtaatg 1380
   tagtttggaa aattatcaaa tcatttacct aatttttttt tttatgaagc aaacattttc 1440
   ctaactatta ttgtttattc cataccaaaa aaaaaaacgt ttatgattta tttaaggatt 1500
   tttttaatta ctacgtaaag gccaatcatg catgagtggg gtggtagaac catagatttg 1560
   cattagtttt agtgctattc ttttggctga atccaccaat taatcagctt agcaattgaa 1620
   agcatatttt attaaccaaa attaatttta acatatctaa cgtggtccat ttggttaaag 1680
   tattcactcc catggattga ggaagtaaga tttaagttat aaccatgctt ggcaattata 1740
   catttaatga atagtttgaa taccaagcta atttataatt tttggagatt aattagtatt 1800
   tatttcggta aatcattctt gtaacaaaaa aaatactaat aatataaaaa attgagattg 1860
   tgaagtttat tttttcacca caaacacaaa tttcatagaa tttttcaagg tttccacaaa 1920
   ttagtttacg ctcttaaaat aacaaacaaa acaaaaagta gtatgcttgt caaactttcc 1980
   acaaaaacat taacgccgga aaacttaaaa caaatagtca taatacatct ttattaaata 2040
   aataaaaaga aggcataata catatataag ttgataaaac tctccatcac ttaagagtaa 2100
   ttttttgatg ttttcactta gaagaattta tatatatcta tattaaccat taattaagga 2160
   acctacaaga gaatgtggta gctagacaag atttgataga gtgaatcaga atatatgtcc 2220
   atggttttag attcagtaaa aaaaaaatgt cccttcaatg tagtggagac tggagaatgt 2280
   cccttcatca tattcacaaa gcattattct ctgtttttcc ataaaaactt ctttggacta 2340
   attaaaaagt aaaatataat tgcgtttcca aggtatcttc ctccatacac tttatttatg 2400
   ccgttgacgg aacaaaagac gatagagaaa gaaggaggag atgatgattc cgaaaatgga 2460
   aagaaagtta aaaagagtgg actcatcgtg tcacaacttt cctttttgac gatatgtatc 2520
   tttctcattt caatcaccga aaggcaaaat ctacaacgtg atccgataaa tttcaacgtc 2580
   cttaacatca ctctcgaagt tatcaggtat gtttctatat ctcaagattc tttaaccaaa 2640
   accaaataaa aaaattgttt cagcaatata taaatctcta tattacatat catgcatcca 2700
   taataactaa aaatttcaaa atattttcaa acaaaatacg tcatctaggt gatacattat 2760
   tctacaattt gacaacccaa gtattcgtaa ttcacctaac aaaaaaagag gacgtgaata 2820
   aaatcgtata agaaacatat atacagtaaa atctctataa attaataatg ttgggactgc 2880
   aaaattttat taatttagag agatttttta cttatcgata aattaataaa ttaatagaga 2940
   gatcttcata atttaataac tttgaaaaaa tttctcatta tagaaaatat ctttctaaaa 3000
   tcaattacaa gtaaaaaata atatcatgta tagaaaacaa caccaatatg ttttgatata 3060
   gtaaaatatt agaactaaat ttcaaataag aatatataca aatttaagca aaaaaatatc 3120
   agaaatatat ttttaagaaa tttcttacat ataatgtata tatatagttg atatatttgt 3180
   gcaattatta atttctgata ttgatggacc atatatttac ataggacttt caaaaaaatt 3240
   attatctaat taatttatcg atttatgtca ttttttatac tactcccaac tcgggaccgg 3300
   aagaatttat taatttatag aggttattaa tttatcgagt ataaatttat aaaataaaaa 3360
   gaaacatata tatatatata atatatatat atatatataa ttcactcatt atgaattgat 3420
   tgtgaattag aagttaagaa ttatcaacaa aataagtttt gtaatggcag tgcatatgga 3480
   aacgttggtt tcactaccgg gtacagctgt gaacggcgtg tggacatcag cgatggtggc 3540
   tgcaaagacg cgagttatgg gtttgcagga cgatggagtc caatgggaaa attcgtacta 3600
   ataatagtaa tgttttatgg taggtttaag cagttcacag ccaaatctgg ccgcgcatgg 3660
   attctttacc cctcgtcttc ctaacatatt tcgatcatat ataaatgtta tattaaaatt 3720
   agtcactttt agcattcctt ttctgtgagt gatgtacaat aagtttgtat gtagtttgca 3780
   tcatcgttaa tcctcgttaa tattatgata atgtatg 3817
<210> 4
   <211> 4308
   <212> DNA
   <213> Artificial sequence
<220>
   <223> expression cassette
<400> 4
   aatccgaaaa gtttctgcac cgttttcacc ccctaactaa caatataggg aacgtgtgct     60
   aaatataaaa tgagacctta tatatgtagc gctgataact agaactatgc aagaaaaact    120
   catccaccta ctttagtggc aatcgggcta aataaaaaag agtcgctaca ctagtttcgt    180
   tttccttagt aattaagtgg gaaaatgaaa tcattattgc ttagaatata cgttcacatc    240
   tctgtcatga agttaaatta ttcgaggtag ccataattgt catcaaactc ttcttgaata    300
   aaaaaatctt tctagctgaa ctcaatgggt aaagagagag atttttttta aaaaaataga    360
   atgaagatat tctgaacgta ttggcaaaga tttaaacata taattatata attttatagt    420
   ttgtgcattc gtcatatcgc acatcattaa ggacatgtct tactccatcc caatttttat    480
   ttagtaatta aagacaattg acttattttt attatttatc ttttttcgat tagatgcaag    540
   gtacttacgc acacactttg tgctcatgtg catgtgtgag tgcacctcct caatacacgt    600
   tcaactagca acacatctct aatatcactc gcctatttaa tacatttagg tagcaatatc    660
   tgaattcaag cactccacca tcaccagacc acttttaata atatctaaaa tacaaaaaat    720
   aattttacag aatagcatga aaagtatgaa acgaactatt taggtttttc acatacaaaa    780
   aaaaaaagaa ttttgctcgt gcgcgagcgc caatctccca tattgggcac acaggcaaca    840
   acagagtggc tgcccacaga acaacccaca aaaaacgatg atctaacgga ggacagcaag    900
   tccgcaacaa ccttttaaca gcaggctttg cggccaggag agaggaggag aggcaaagaa    960
   aaccaagcat cctcctcctc ccatctataa attcctcccc ccttttcccc tctctatata   1020
   ggaggcatcc aagccaagaa gagggagagc accaaggaca cgcgactagc agaagccgag   1080
   cgaccgcctt cttcgatcca tatcttccgg tcgagttctt ggtcgatctc ttccctcctc   1140
   cacctcctcc tcacagggta tgtgcccttc ggttgttctt ggatttattg ttctaggttg   1200
   tgtagtacgg gcgttgatgt taggaaaggg gatctgtatc tgtgatgatt cctgttcttg   1260
   gatttgggat agaggggttc ttgatgttgc atgttatcgg ttcggtttga ttagtagtat   1320
   ggttttcaat cgtctggaga gctctatgga aatgaaatgg tttagggtac ggaatcttgc   1380
   gattttgtga gtaccttttg tttgaggtaa aatcagagca ccggtgattt tgcttggtgt   1440
   aataaaagta cggttgtttg gtcctcgatt ctggtagtga tgcttctcga tttgacgaag   1500
   ctatcctttg tttattccct attgaacaaa aataatccaa ctttgaagac ggtcccgttg   1560
   atgagattga atgattgatt cttaagcctg tccaaaattt cgcagctggc ttgtttagat   1620
   acagtagtcc ccatcacgaa attcatggaa acagttataa tcctcaggaa caggggattc   1680
   cctgttcttc cgatttgctt tagtcccaga attttttttc ccaaatatct taaaaagtca   1740
   ctttctggtt cagttcaatg aattgattgc tacaaataat gcttttatag cgttatccta   1800
   gctgtagttc agttaatagg taatacccct atagtttagt caggagaaga acttatccga   1860
   tttctgatct ccatttttaa ttatatgaaa tgaactgtag cataagcagt attcatttgg   1920
   attatttttt ttattagctc tcaccccttc attattctga gctgaaagtc tggcatgaac   1980
   tgtcctcaat tttgttttca aattcacatc gattatctat gcattatcct cttgtatcta   2040
   cctgtagaag tttctttttg gttattcctt gactgcttga ttacagaaag aaatttatga   2100
   agctgtaatc gggatagtta tactgcttgt tcttatgatt catttccttt gtgcagttct   2160
   tggtgtagct tgccactttc accagcaaag ttcatttaaa tcaactaggg atatcacaag   2220
   tttgtacaaa aaagcaggct tcacaatgga cagagtggtg gcaaaaatag caaaaatccg   2280
   ttcgcagctt actaaattac gttcactatt cttcctttac ttcatctact tcttgttctt   2340
   ctccttttta gggtttttgg cactcaagat cacaaagcca agaaccactt cacgtcctca   2400
   tgactttgac cttttcttca cttctgtctc tgccatcacc gtctcttcca tgtctaccgt   2460
   cgacatggaa gtcttctcca acacccaact tatcttcctc actatcctca tgttcctcgg   2520
   tggcgaaatc ttcacctcct ttctcaacct ctacgtctcc tatttcacca agttcgtctt   2580
   ccctcataac aagattagac atattttggg atcttataat tcggacagtt ccatcgagga   2640
   tcgctgtgac gttgagactg ttactgatta tcgtgagggt cttatcaaga tcgatgaaag   2700
   ggcatctaag tgcttgtact cggtggttct tagttaccat cttgttacta acctagttgg   2760
   ctctgtgttg cttcttgtgt acgtaaattt tgttaaaacg gcgagagatg ttcttagttc   2820
   caaagaaatc tcacctctca ctttctccgt cttcacaaca gtttccacgt ttgcaaactg   2880
   cggatttgtc cccacgaatg agaacatgat catctttcgc aaaaactctg gtctcatctg   2940
   gctcctaatc cctcaagtac tgatgggaaa cactttgttc ccttgcttct tggttttgct   3000
   catatgggga ctttataaga tcacaaagcg tgacgagtat ggttacattc tcaagaacca   3060
   caataagatg ggatactctc atctactctc ggttcgtcta tgtgttcttc ttggagtgac   3120
   ggtgctaggg tttctgataa tacagcttct tttcttctgc gcctttgaat ggacctctga   3180
   gtctctagaa ggaatgagtt cgtacgagaa gttggttgga tcgttgtttc aagtggtgaa   3240
   ttcgcgacac accggagaaa ctatagtaga cctctctaca ctttccccag ctatcttggt   3300
   actctttatt cttatgatgt atcttcctcc atacacttta tttatgccgt tgacggaaca   3360
   aaagacgata gagaaagaag gaggagatga tgattccgaa aatggaaaga aagttaaaaa   3420
   gagtggactc atcgtgtcac aactttcctt tttgacgata tgtatctttc tcatttcaat   3480
   caccgaaagg caaaatctac aacgtgatcc gataaatttc aacgtcctta acatcactct   3540
   cgaagttatc agtgcatatg gaaacgtggg tttcactacc gggtacagct gtgaacggcg   3600
   tctggacatc agcgatggtg gctgcaaaga cgcgagttat gggtttgcag gacgatggag   3660
   tccaatggga aaattcgtac taataatagt aatgttttat ggtaggttta agcagttcac    3720
   agtcaaatct ggccgcgcat ggatacttta cccctcgtct tcctaacaac ccagctttct    3780
   tgtacaaagt ggtgatatca caagcccggg cggtcttcta gggataacag ggtaattata    3840
   tccctctaga tcacaagccc gggcggtctt ctacgatgat tgagtaataa tgtgtcacgc    3900
   atcaccatgg gtggcagtgt cagtgtgagc aatgacctga atgaacaatt gaaatgaaaa    3960
   gaaaaaaagt actccatctg ttccaaatta aaattcattt taacctttta ataggtttat    4020
   acaataattg atatatgttt tctgtatatg tctaatttgt tatcatccgg gcggtcttct    4080
   agggataaca gggtaattat atccctctag acaacacaca acaaataaga gaaaaaacaa    4140
   ataatattaa tttgagaatg aacaaaagga ccatatcatt cattaactct tctccatcca    4200
   tttccatttc acagttcgat agcgaaaacc gaataaaaaa cacagtaaat tacaagcaca    4260
   acaaatggta caagaaaaac agttttccca atgccataat actcgaac                 4308
<210> 5
   <211> 52
   <212> DNA
   <213> Artificial sequence
<220>
   <223> primer: prm3283
<400> 5
   ggggacaagt ttgtacaaaa aagcaggctt cacaatggac agagtggtgg ca 52
<210> 6
   <211> 52
   <212> DNA
   <213> Artificial sequence
<220>
   <223> primer: prm3286
<400> 6
   ggggaccact ttgtacaaga aagctgggtg aaatatgtta ggaagacgag gg 52
<210> 7
   <211> 112
   <212> PRT
   <213> Artificial sequence
<220>
   <223> domain
<400> 7
   Gly Leu Ile Val Ser Gln Leu Ser Phe Leu Thr Ile Cys Ile Phe Leu
   1               5                   10                  15
   Ile Ser Ile Thr Glu Arg Gln Asn Leu Gln Arg Asp Pro Ile Asn Phe
               20                  25                  30
   Asn Val Leu Asn Ile Thr Leu Glu Val Ile Ser Ala Tyr Gly Asn Val
           35                  40                  45
   Gly Phe Thr Thr Gly Tyr Ser Cys Glu Arg Arg Leu Asp Ile Ser Asp
       50                  55                  60
   Gly Gly Cys Lys Asp Ala Ser Tyr Gly Phe Ala Gly Arg Trp Ser Pro
   65                  70                  75                  80
   Met Gly Lys Phe Val Leu Ile Ile Val Met Phe Tyr Gly Arg Phe Lys
   85 90 95
   Gln Phe Thr Ala Lys Ser Gly Arg Ala Trp Ile Leu Tyr Pro Ser Ser
   100 105 110
<210> 8
   <211> 15
   <212> PRT
   <213> Artificial sequence
<220>
   <223> domain
<400> 8
   Glu Val Ile Ser Ala Tyr Gly Asn Val Gly Phe Thr Thr Gly Tyr
   1 5 10 15
<210> 9
   <211> 15
   <212> PRT
   <213> Artificial sequence
<220>
   <223> consensus sequence
<220>
   <221> MISC_FEATURE
   <222> (2)..(2)
   <223> Xaa can be Val or Ile
<220>
   <221> MISC_FEATURE
   <222> (3)..(3)
   <223> Xaa can be Ile or Val
<220>
   <221> MISC_FEATURE
   <222> (6)..(6)
   <223> Xaa can be Tyr or Phe
<220>
   <221> MISC_FEATURE
   <222> (8)..(8)
   <223> Xaa can be Asn or Thr
<220>
   <221> MISC_FEATURE
   <222> (9)..(9)
   <223> Xaa can be Val, Ala or Thr
<220>
   <221> MISC_FEATURE
   <222> (11)..(11)
   <223> Xaa can be Phe, Leu or Tyr
<220>
   <221> MISC_FEATURE
   <222> (12)..(12)
   <223> Xaa can be Thr or Ser
<220>
   <221> MISC_FEATURE
   <222> (13).. (13)
   <223> Xaa can be Thr, Ile, Leu, Val or Met
<400> 9
   Glu Xaa Xaa Ser Ala Xaa Gly Xaa Xaa Gly Xaa Xaa Xaa Gly Tyr
   1               5                   10                  15
<210> 10
   <211> 5
   <212> PRT
   <213> Artificial sequence
<220>
   <223> signature
<220>
   <221> MISC_FEATURE
   <222> (3)..(3)
   <223> Xaa can be Ile or Leu
<220>
   <221> MISC_FEATURE
   <222> (5)..(5)
   <223> Xaa can be Tyr, Phe or Leu
<400> 10
   Asp Pro Xaa Asn Xaa
   1               5
<210> 11
   <211> 1632
   <212> DNA
   <213> Mesembryanthemum crystallinum
<400> 11
   atggggagat ttgggtttct taaggagaaa gttcagcaac tttatggttg tctttgtgta     60
   ggcttatttt accttcttag tactttgttt tgggtatcca acaaaattta tgactttatt    120
   acctatcgta tgagtcattt ttccattgaa gtgtgttact tcatctttgt gtcgtgttta    180
   gggttcttaa tcttgaggaa tataaagcct aggacgtatc cggttaaacc tggagaatta    240
   gatatgttct ttacctcggt ctcggccgca acagtttcat ccatggctac tgttgaaatg    300
   gaggtatttt cagatgccca actccatatt atgaccattt tgatgttcat aggaggggag    360
   gttttcactt ccatggtagg ccttcatttt aaggcctcta ggcttgggaa tacaccctta    420
   ggtgtaaaaa gtagggctaa ttctgtggct agtttaccat gcccaccaga agattttgat    480
   cacattgagt taggcatcat cactaccaca accaccacaa caacaacaac cacaaccaca    540
   ttgcaaaaaa ctaaatcaga aattgatttc ctcttaaagt ctagatcaat tagggttttg    600
   ggttttgtag ttttggccta cctactaata gttcatgtct taggcacaat aatggtctat    660
   gcatacctta gaattgagcc tagtgcgaag agggttctag agactaaagg gttaaaatct    720
   atcacattcg cgattttcac tagtgtttca acattttcaa gctgtgggtt cgtccctacc    780
   aatgaaaaca tgatcatttt tcgacaaaat tccggcctcc tcttgatgct aatccctcaa    840
   gtcctcctag gaaacacatt gttaccatct ttcttaagat taacaatttg ggtcctggga    900
   aaatttacaa agaaggatga atctaagtat cttatgagga acacaaagga gattgcatac    960
   catcatttgc tccctaccaa gcactctaag tatgtggttg tgaccgtttt cgggttcatt   1020
   ttggcgtcac tcattatgtt ttgctcaatg gattggaact tgaagggatt aagtgatctt   1080
   aatgtttacc aaaaactggt gggtgcattg ttccaatgtg tgaatgcaag acatactggt   1140
   gaaactattg ttgatctctc aactattgcc tcagctgtct tggttgtttt cgtcataatg   1200
   atgtatcttc ccccttatac atcatttctg ccaacaaaag atggagaaga agaataccca   1260
   ctagtataca aaggagaaaa gacaaagggg aagttgattt tggacaatgt ggtattctca   1320
   caactctcct accttgtcat cttcatcatc cttgtttgca tcacagagag aaaaagcatg   1380
   aaagaagatc ccctcaattt caatgttctc aacattgttg ttgaagttat tagtgcatat   1440
   ggaaatgttg gattttcaac tggctacagt tgttcaaggc aattaaaacc agatgcaaat   1500
   tgtgtagata aatggtatgg atttgtagga aaatggagtg accaagggaa gatcatcctc   1560
   atttttgtca tgttctttgg aaggctcaag aaattcaaca tgaaaggagg cagagcatgg   1620
   aagctcctat aa                                                       1632
<210> 12
   <211> 543
   <212> PRT
   <213> Mesembryanthemum crystallinum
<400> 12
   Met Gly Arg Phe Gly Phe Leu Lys Glu Lys Val Gln Gln Leu Tyr Gly
   1               5                   10                  15
   Cys Leu Cys Val Gly Leu Phe Tyr Leu Leu Ser Thr Leu Phe Trp Val
               20                  25                  30
   Ser Asn Lys Ile Tyr Asp Phe Ile Thr Tyr Arg Met Ser His Phe Ser
           35                  40                  45
   Ile Glu Val Cys Tyr Phe Ile Phe Val Ser Cys Leu Gly Phe Leu Ile
       50                  55                  60
   Leu Arg Asn Ile Lys Pro Arg Thr Tyr Pro Val Lys Pro Gly Glu Leu
   65                  70                  75                  80
   Asp Met Phe Phe Thr Ser Val Ser Ala Ala Thr Val Ser Ser Met Ala
                   85                  90                  95
   Thr Val Glu Met Glu Val Phe Ser Asp Ala Gln Leu His Ile Met Thr
               100                 105                 110
   Ile Leu Met Phe Ile Gly Gly Glu Val Phe Thr Ser Met Val Gly Leu
           115                 120                 125
   His Phe Lys Ala Ser Arg Leu Gly Asn Thr Pro Leu Gly Val Lys Ser
       130                 135                 140
   Arg Ala Asn Ser Val Ala Ser Leu Pro Cys Pro Pro Glu Asp Phe Asp
   145                 150                 155                 160
   His Ile Glu Leu Gly Ile Ile Thr Thr Thr Thr Thr Thr Thr Thr Thr
                   165                 170                 175
   Thr Thr Thr Thr Leu Gln Lys Thr Lys Ser Glu Ile Asp Phe Leu Leu
               180                 185                 190
   Lys Ser Arg Ser Ile Arg Val Leu Gly Phe Val Val Leu Ala Tyr Leu
           195                 200                 205
   Leu Ile Val His Val Leu Gly Thr Ile Met Val Tyr Ala Tyr Leu Arg
       210                 215                 220
   Ile Glu Pro Ser Ala Lys Arg Val Leu Glu Thr Lys Gly Leu Lys Ser
   225                 230                 235                 240
   Ile Thr Phe Ala Ile Phe Thr Ser Val Ser Thr Phe Ser Ser Cys Gly
                   245                 250                 255
   Phe Val Pro Thr Asn Glu Asn Met Ile Ile Phe Arg Gln Asn Ser Gly
               260                 265                 270
   Leu Leu Leu Met Leu Ile Pro Gln Val Leu Leu Gly Asn Thr Leu Leu
           275                 280                 285
   Pro Ser Phe Leu Arg Leu Thr Ile Trp Val Leu Gly Lys Phe Thr Lys
       290                 295                 300
   Lys Asp Glu Ser Lys Tyr Leu Met Arg Asn Thr Lys Glu Ile Ala Tyr
   305                 310                 315                 320
   His His Leu Leu Pro Thr Lys His Ser Lys Tyr Val Val Val Thr Val
                   325                 330                 335
   Phe Gly Phe Ile Leu Ala Ser Leu Ile Met Phe Cys Ser Met Asp Trp
               340                 345                 350
   Asn Leu Lys Gly Leu Ser Asp Leu Asn Val Tyr Gln Lys Leu Val Gly
           355                 360                 365
   Ala Leu Phe Gln Cys Val Asn Ala Arg His Thr Gly Glu Thr Ile Val
       370                 375                 380
   Asp Leu Ser Thr Ile Ala Ser Ala Val Leu Val Val Phe Val Ile Met
   385                 390                 395                 400
   Met Tyr Leu Pro Pro Tyr Thr Ser Phe Leu Pro Thr Lys Asp Gly Glu
                   405                 410                 415
   Glu Glu Tyr Pro Leu Val Tyr Lys Gly Glu Lys Thr Lys Gly Lys Leu
               420                 425                 430
   Ile Leu Asp Asn Val Val Phe Ser Gln Leu Ser Tyr Leu Val Ile Phe
           435                 440                 445
   Ile Ile Leu Val Cys Ile Thr Glu Arg Lys Ser Met Lys Glu Asp Pro
       450                 455                 460
   Leu Asn Phe Asn Val Leu Asn Ile Val Val Glu Val Ile Ser Ala Tyr
   465                 470                 475                 480
   Gly Asn Val Gly Phe Ser Thr Gly Tyr Ser Cys Ser Arg Gln Leu Lys
                   485                 490                 495
   Pro Asp Ala Asn Cys Val Asp Lys Trp Tyr Gly Phe Val Gly Lys Trp
               500                 505                 510
   Ser Asp Gln Gly Lys Ile Ile Leu Ile Phe Val Met Phe Phe Gly Arg
           515                 520                 525
   Leu Lys Lys Phe Asn Met Lys Gly Gly Arg Ala Trp Lys Leu Leu
       530                 535                 540
<210> 13
   <211> 1518
   <212> DNA
   <213> Mesembryanthemum crystallinum
<400> 13
   atggagaaat atttggctct cttccacaag aaattagatc aattttttaa tttttgttat      60
   acaaaagctt tatcatatat tagctccttg tatgagtaca taatcttcca agttcaccca     120
   ttttggcatc acctctttta ttatatcata gtttctctct tgggttactt ctccttgaag     180
   gccacttcaa gaaagcagag gagctcccct gttttctctg atccccgcca cgatgacctc     240
   gacctcttct tcacctctgt ctccgccacc accatctcga gcatgtcgac aatcgagatg     300
   gaggatttct cgagtcccca gctcgtcgtt ctcatcatcc tcatgctttc tggtggggaa     360
   gtcttccttt ccctccttgg cctccagctc cgtaaatcca agcataggaa gagggagcaa     420
   aagaacatcc tcttgaactc gccctccgaa ggtatgaaat ataagtcact ggtggcacta     480
   ggacatgttg ttttgggcta cctcttggtg tgccatataa ttgggtacag tctagcttcc     540
   ttgtacataa gcatttctag aagtgcaagc aatgtgctcg agagaaaaca cattgagatg     600
   catctcttct ccctcttcgt caccgtctcc acattctcca actgtggatt catcccaaca     660
   aatgagaaca tgatggtctt taagggaaat tcaggattgc tattgatcct catcccacaa     720
   atcctcctag ggaacaaact gtacccatct tgcctaaggc tagttctttg ggtgctcgaa     780
   aagctaaccg gcaaagccga gttcagctac atattgaaga aaaatgatga gcttggttat     840
   gggttgtttt tctcgcaagt cgatgcgttg ctcttggctg tgactgcggt tggattggtt     900
   atggttcagt ttgtggtgtt ttgcatcttg gagtggaatt cggtagcgtt gatggatctt     960
   aattggcatc aaaaactggt cggatctctc tttcaaactg tcaattctag gcacagtgga    1020
   gaatctatca ttgatctctc ccttatttcc cctgccacca tggttctctt cgttgtcatg    1080
   atgtatcttc catcctatac cacatttata cctgttggat acgacaaaga aatatcgcca    1140
   gaaatgagca gcaagaagag caaaaagcaa ggttgcagca ttgcagaaaa tctcaaattc    1200
   tctcagcttt cttacttggc tatctttgtt atgattgttt gcatcactga aaggagaaat    1260
   ttagtggaag accctctcaa cttcaacgtc ttcaacatta tcgtagaagt cataagtgca    1320
   tatggaaatg ttggattctc catgggttat agttgtaaaa gaagacttgg agacattgga    1380
   acttgcaagg atgcaggata tggcttcgtg ggtcgatgga gtagacaagg gaaaatggtt    1440
   atcatcttgg tcatgctttt tggtaggctt aagagattta attttaaagg cggaaaagct    1500
   tggaaacttt ctctttaa                                                  1518
<210> 14
   <211> 505
   <212> PRT
   <213> Mesembryanthemum crystallinum
<400> 14
   Met Glu Lys Tyr Leu Ala Leu Phe His Lys Lys Leu Asp Gln Phe Phe
   1               5                   10                  15
   Asn Phe Cys Tyr Thr Lys Ala Leu Ser Tyr Ile Ser Ser Leu Tyr Glu
               20                  25                  30
   Tyr Ile Ile Phe Gln Val His Pro Phe Trp His His Leu Phe Tyr Tyr
           35                  40                  45
   Ile Ile Val Ser Leu Leu Gly Tyr Phe Ser Leu Lys Ala Thr Ser Arg
       50                  55                  60
   Lys Gln Arg Ser Ser Pro Val Phe Ser Asp Pro Arg His Asp Asp Leu
   65                  70                  75                  80
   Asp Leu Phe Phe Thr Ser Val Ser Ala Thr Thr Ile Ser Ser Met Ser
                   85                  90                  95
   Thr Ile Glu Met Glu Asp Phe Ser Ser Pro Gln Leu Val Val Leu Ile
               100                 105                 110
   Ile Leu Met Leu Ser Gly Gly Glu Val Phe Leu Ser Leu Leu Gly Leu
   115 120 125
   Gln Leu Arg Lys Ser Lys His Arg Lys Arg Glu Gln Lys Asn Ile Leu
   130 135 140
   Leu Asn Ser Pro Ser Glu Gly Met Lys Tyr Lys Ser Leu Val Ala Leu
   145 150 155 160
   Gly His Val Val Leu Gly Tyr Leu Leu Val Cys His Ile Ile Gly Tyr
   165 170 175
   Ser Leu Ala Ser Leu Tyr Ile Ser Ile Ser Arg Ser Ala Ser Asn Val
   180 185 190
   Leu Glu Arg Lys His Ile Glu Met His Leu Phe Ser Leu Phe Val Thr
   195 200 205
   Val Ser Thr Phe Ser Asn Cys Gly Phe Ile Pro Thr Asn Glu Asn Met
   210 215 220
   Met Val Phe Lys Gly Asn Ser Gly Leu Leu Leu Ile Leu Ile Pro Gln
   225 230 235 240
   Ile Leu Leu Gly Asn Lys Leu Tyr Pro Ser Cys Leu Arg Leu Val Leu
   245 250 255
   Trp Val Leu Glu Lys Leu Thr Gly Lys Ala Glu Phe Ser Tyr Ile Leu
   260 265 270
   Lys Lys Asn Asp Glu Leu Gly Tyr Gly Leu Phe Phe Ser Gln Val Asp
   275 280 285
   Ala Leu Leu Leu Ala Val Thr Ala Val Gly Leu Val Met Val Gln Phe
   290 295 300
   Val Val Phe Cys Ile Leu Glu Trp Asn Ser Val Ala Leu Met Asp Leu
   305 310 315 320
   Asn Trp His Gln Lys Leu Val Gly Ser Leu Phe Gln Thr Val Asn Ser
   325 330 335
   Arg His Ser Gly Glu Ser Ile Ile Asp Leu Ser Leu Ile Ser Pro Ala
   340 345 350
   Thr Met Val Leu Phe Val Val Met Met Tyr Leu Pro Ser Tyr Thr Thr
   355 360 365
   Phe Ile Pro Val Gly Tyr Asp Lys Glu Ile Ser Pro Glu Met Ser Ser
   370 375 380
   Lys Lys Ser Lys Lys Gln Gly Cys Ser Ile Ala Glu Asn Leu Lys Phe
   385 390 395 400
   Ser Gln Leu Ser Tyr Leu Ala Ile Phe Val Met Ile Val Cys Ile Thr
   405 410 415
   Glu Arg Arg Asn Leu Val Glu Asp Pro Leu Asn Phe Asn Val Phe Asn
   420 425 430
   Ile Ile Val Glu Val Ile Ser Ala Tyr Gly Asn Val Gly Phe Ser Met
           435                 440                 445
   Gly Tyr Ser Cys Lys Arg Arg Leu Gly Asp Ile Gly Thr Cys Lys Asp
       450                 455                 460
   Ala Gly Tyr Gly Phe Val Gly Arg Trp Ser Arg Gln Gly Lys Met Val
   465                 470                 475                 480
   Ile Ile Leu Val Met Leu Phe Gly Arg Leu Lys Arg Phe Asn Phe Lys
                   485                 490                 495
   Gly Gly Lys Ala Trp Lys Leu Ser Leu
               500                 505
<210> 15
   <211> 1653
   <212> DNA
   <213> Eucalyptus camaldulensis
<400> 15
   atgatgagct ttttttcgtc tttaggcaaa gaggttgcgt tcctttgtag cgcttcatgg      60
   atcaaactag cctgcatctg caggtccctt tgcttcctcc tttcttgctg tttccgcttc     120
   ttgctcctcc gagtcaattc cttctgcatc caagtttttt acttcgtctt cctctcgttc     180
   ctcggcttct gggtcctcaa ggccctaggg ccgcggaccg attcctttag gcctagggac     240
   ttggatctgt tcttcacttc cgtctctgcg acgaccgtct cgagcatgtc cacggtcgag     300
   atggaagtct tctccaactc acagcttgtc gtgatgaccg tcctgatgtt catcggagga     360
   gaggtcttca tttccctcgt tgggcttcat cttaggaaat ccaagctccg gtggcgcatc     420
   agaaccgaag acaaagtcgc ctctgccgat ggcaatctct gcccttccgc cccaacaaat     480
   gacattgttg accacataga gttaggtgta gtggcgaaaa cagattgcct gaactcgcag     540
   gtagagcctc aattttacag accgcaagac aagtcctcag atcttgacta tttgaaatat     600
   tgttcggtta ggtttttgtg ttatgtagtg ttaggttacc ttctggtagt ccaagttcta     660
   ggagttgccg cagtttcgct ctatatcaca ctagttccta gcgccagaga tgtgctaaag     720
   aagaaaggtc tcaaaatggt gaccttttcc gtcttcacca ccgtgtcgac ctttgccagt     780
   tgtgggtttg tcccgaccaa tgaaaacatg attatcttca gcaaaaactc tggcctcctc     840
   ctgattctca tccctcaggc ccttcttggg aacatgctgt tcccatcgag cctacgtttg     900
   acgctttggc tcatcgggag attcagcgag aaagatgaaa tcgggtactt gttgagcagg     960
   acaagtgaaa ttggttacaa gcacttgctt ccgagcctct actcgtcgct actgggggtc    1020
   acggtgttgg ggttcgtcgg tattcagttt ataatgtttt gctcgatgca gtgggattcg    1080
   gaatcgttga atggactaag ctcctgtgag aaaatcgtgg gcgcgctgtt tcagtgcgtg    1140
   aacagcagac ataccggcga gacggtcgtc gatctgtcca cagttgctcc cgccatcttg    1200
   gtgctattcg tggtcatgat gtaccttccg ccttacacgt cgttccttcc ggtgaaaggc    1260
   aatgagaggt tccctgaaaa cggcgaaaga agaaagccga aacagagtta tagactgctt    1320
   ttggagaatc tcaaattttc acagctttcc tacctggcca tctttatcat cgtgatttgc    1380
   atcaccgaga ggaaaaagat gaaggaagat cccctcaatt tcaacgtgtt gaacatcgtg    1440
   gtcgaagtgg taagcgcata tggaaatgtg gggtttacga caggatacag ttgcgaacgc    1500
   caactaaggc ctgtcaaagg ctgcgaggac aagtggtatg gattctctgg gaaatggagc    1560
   gatgaaagca aaatcatcct catcgtagtc atgtttttcg gaagattaaa gaagttcaac    1620
   atgaagggcg gccgagcttg gatactcttg tag                                 1653
<210> 16
   <211> 550
   <212> PRT
   <213> Eucalyptus camaldulensis
<400> 16
   Met Met Ser Phe Phe Ser Ser Leu Gly Lys Glu Val Ala Phe Leu Cys
   1 5 10 15
   Ser Ala Ser Trp Ile Lys Leu Ala Cys Ile Cys Arg Ser Leu Cys Phe
               20                  25                  30
   Leu Leu Ser Cys Cys Phe Arg Phe Leu Leu Leu Arg Val Asn Ser Phe
           35                  40                  45
   Cys Ile Gln Val Phe Tyr Phe Val Phe Leu Ser Phe Leu Gly Phe Trp
       50                  55                  60
   Val Leu Lys Ala Leu Gly Pro Arg Thr Asp Ser Phe Arg Pro Arg Asp
   65                  70                  75                  80
   Leu Asp Leu Phe Phe Thr Ser Val Ser Ala Thr Thr Val Ser Ser Met
                   85                  90                  95
   Ser Thr Val Glu Met Glu Val Phe Ser Asn Ser Gln Leu Val Val Met
               100                 105                 110
   Thr Val Leu Met Phe Ile Gly Gly Glu Val Phe Ile Ser Leu Val Gly
           115                 120                 125
   Leu His Leu Arg Lys Ser Lys Leu Arg Trp Arg Ile Arg Thr Glu Asp
       130                 135                 140
   Lys Val Ala Ser Ala Asp Gly Asn Leu Cys Pro Ser Ala Pro Thr Asn
   145                 150                 155                 160
   Asp Ile Val Asp His Ile Glu Leu Gly Val Val Ala Lys Thr Asp Cys
                   165                 170                 175
   Leu Asn Ser Gln Val Glu Pro Gln Phe Tyr Arg Pro Gln Asp Lys Ser
               180                 185                 190
   Ser Asp Leu Asp Tyr Leu Lys Tyr Cys Ser Val Arg Phe Leu Cys Tyr
           195                 200                 205
   Val Val Leu Gly Tyr Leu Leu Val Val Gln Val Leu Gly Val Ala Ala
       210                 215                 220
   Val Ser Leu Tyr Ile Thr Leu Val Pro Ser Ala Arg Asp Val Leu Lys
   225                 230                 235                 240
   Lys Lys Gly Leu Lys Met Val Thr Phe Ser Val Phe Thr Thr Val Ser
                   245                 250                 255
   Thr Phe Ala Ser Cys Gly Phe Val Pro Thr Asn Glu Asn Met Ile Ile
               260                 265                 270
   Phe Ser Lys Asn Ser Gly Leu Leu Leu Ile Leu Ile Pro Gln Ala Leu
           275                 280                 285
   Leu Gly Asn Met Leu Phe Pro Ser Ser Leu Arg Leu Thr Leu Trp Leu
       290                 295                 300
   Ile Gly Arg Phe Ser Glu Lys Asp Glu Ile Gly Tyr Leu Leu Ser Arg
   305                 310                 315                 320
   Thr Ser Glu Ile Gly Tyr Lys His Leu Leu Pro Ser Leu Tyr Ser Ser
                   325                 330                 335
   Leu Leu Gly Val Thr Val Leu Gly Phe Val Gly Ile Gln Phe Ile Met
               340                 345                 350
   Phe Cys Ser Met Gln Trp Asp Ser Glu Ser Leu Asn Gly Leu Ser Ser
           355                 360                 365
   Cys Glu Lys Ile Val Gly Ala Leu Phe Gln Cys Val Asn Ser Arg His
       370                 375                 380
   Thr Gly Glu Thr Val Val Asp Leu Ser Thr Val Ala Pro Ala Ile Leu
   385                 390                 395                 400
   Val Leu Phe Val Val Met Met Tyr Leu Pro Pro Tyr Thr Ser Phe Leu
                   405                 410                 415
   Pro Val Lys Gly Asn Glu Arg Phe Pro Glu Asn Gly Glu Arg Arg Lys
               420                 425                 430
   Pro Lys Gln Ser Tyr Arg Leu Leu Leu Glu Asn Leu Lys Phe Ser Gln
           435                 440                 445
   Leu Ser Tyr Leu Ala Ile Phe Ile Ile Val Ile Cys Ile Thr Glu Arg
       450                 455                 460
   Lys Lys Met Lys Glu Asp Pro Leu Asn Phe Asn Val Leu Asn Ile Val
   465                 470                 475                 480
   Val Glu Val Val Ser Ala Tyr Gly Asn Val Gly Phe Thr Thr Gly Tyr
                   485                 490                 495
   Ser Cys Glu Arg Gln Leu Arg Pro Val Lys Gly Cys Glu Asp Lys Trp
               500                 505                 510
   Tyr Gly Phe Ser Gly Lys Trp Ser Asp Glu Ser Lys Ile Ile Leu Ile
           515                 520                 525
   Val Val Met Phe Phe Gly Arg Leu Lys Lys Phe Asn Met Lys Gly Gly
       530                 535                 540
   Arg Ala Trp Ile Leu Leu
   545                 550
<210> 17
   <211> 1650
   <212> DNA
   <213> Eucalyptus camaldulensis
<400> 17
   atgatgcgct ttccttgttt aggcaaagaa gtcgtgtgtc tttgtattgc ttcatgcctc     60
   aaaatagccc gcctcttcag gtccttctgc ttcctctttt cttggtactt ccgcttcctg    120
   ctcctccgag tcaactcctt ttgtgtccag cttttgtact tcgtcttcct ctcgctcctt    180
   ggcttctggg tccttaaggc atcgaggccg ctaacccatt ccttcaggcc tagggacttg    240
   gatctgttct tcacctctgt ctccttggct actgtctcga gcatgtccac ggtggagatg    300
   gaggtcctct ccgactccca gctcgtcgtg atgaccgtcc tgatgttcgt cggaggggaa    360
   gtcttcacct ctcttgccgg gcttcacttc aggaaactga aactccagtc gctcctgaaa    420
   accgaagaaa cagtcgcctc cgtccatacc aatctttgcc cttcgaaccc aaccaacggt    480
   gttgttgacc acgtagagtt agctgtagta acgaattcag attgcctgaa ctctcgagta    540
   gaacctcagt tgcacggacc gcgagacgag tccccagatc tcgactattt gaaatatcgt    600
   tccgctaacg ttttgtgtct tgtagtgtta ggttaccttc tgggagtcaa tgttctcggt    660
   gtcgccgcag tttcactcta tatgatactt gtttccagcg cgagagacgt gctaaagcgg    720
   aagggcctca aaatgatgac attttccatc ttcaccactg tgtcgacctt cgccagttgc    780
   gggttcgtcc cgacgaacga aaacatggct gtcttcaaca agaactccgg cctcctccta    840
   attctcatcc ctcaggtcct tctcgggaac acgctgttcc cgtcgacctt acggctggta    900
   ctttggctct tgggaaaatt cagcaagaaa gcagaaatcg attatttgtt gagcagaaca    960
   ggtgaaattg gttacaagca cttgcttccg agcctctact cgtcgctcct ggtggtaacg   1020
   gtgttggggt ttattggtgt tcagttcata atgttttgct caatgcagtg ggattcagaa   1080
   tcgttagatg gactaaactc gtacgagaaa attgtggccg tgctctttca gtgcgtgaac   1140
   accagatata cgggcgagac gatcgttaat ctgtccaaag tatctcctgc catcttggtg   1200
   ttattcgtcg tcatgatgta ccttccgcca tacacgtcct tccttccggc gataggtggt   1260
   gaggagctcc tgggaaatgg ggaaagaaaa aaggcgaaac gaagtcataa actaatgttg   1320
   aagagtctca tattctcaca gccctcctac ttggccatct ttatcatcac gatttgcata   1380
   acggagaagg aaaagatgaa gaaagatcct ctcaacttta atgtgttcaa catcgtggtc   1440
   gaagtgataa gtgcatatgg gaatgtgggg ttcacgatag gttacaattg cgatcggcaa   1500
   ctaagacgta ttgaaggctg cgaggacaag tggtacggat tctctgggaa atggagcgat   1560
   gagggcaaaa ttatcctcat tgccgtcatg attttcggaa gattaaagaa gttcaacatg   1620
   aaaggtggcc gaaattggat actcttgtga                                     1650
<210> 18
   <211> 549
   <212> PRT
   <213> Eucalyptus camaldulensis
<400> 18
   Met Met Arg Phe Pro Cys Leu Gly Lys Glu Val Val Cys Leu Cys Ile
   1 5 10 15
   Ala Ser Cys Leu Lys Ile Ala Arg Leu Phe Arg Ser Phe Cys Phe Leu
   20 25 30
   Phe Ser Trp Tyr Phe Arg Phe Leu Leu Leu Arg Val Asn Ser Phe Cys
   35 40 45
   Val Gln Leu Leu Tyr Phe Val Phe Leu Ser Leu Leu Gly Phe Trp Val
   50 55 60
   Leu Lys Ala Ser Arg Pro Leu Thr His Ser Phe Arg Pro Arg Asp Leu
   65 70 75 80
   Asp Leu Phe Phe Thr Ser Val Ser Leu Ala Thr Val Ser Ser Met Ser
   85 90 95
   Thr Val Glu Met Glu Val Leu Ser Asp Ser Gln Leu Val Val Met Thr
   100 105 110
   Val Leu Met Phe Val Gly Gly Glu Val Phe Thr Ser Leu Ala Gly Leu
   115 120 125
   His Phe Arg Lys Leu Lys Leu Gln Ser Leu Leu Lys Thr Glu Glu Thr
   130 135 140
   Val Ala Ser Val His Thr Asn Leu Cys Pro Ser Asn Pro Thr Asn Gly
   145 150 155 160
   Val Val Asp His Val Glu Leu Ala Val Val Thr Asn Ser Asp Cys Leu
   165 170 175
   Asn Ser Arg Val Glu Pro Gln Leu His Gly Pro Arg Asp Glu Ser Pro
   180 185 190
   Asp Leu Asp Tyr Leu Lys Tyr Arg Ser Ala Asn Val Leu Cys Leu Val
   195 200 205
   Val Leu Gly Tyr Leu Leu Gly Val Asn Val Leu Gly Val Ala Ala Val
   210 215 220
   Ser Leu Tyr Met Ile Leu Val Ser Ser Ala Arg Asp Val Leu Lys Arg
   225 230 235 240
   Lys Gly Leu Lys Met Met Thr Phe Ser Ile Phe Thr Thr Val Ser Thr
   245 250 255
   Phe Ala Ser Cys Gly Phe Val Pro Thr Asn Glu Asn Met Ala Val Phe
   260 265 270
   Asn Lys Asn Ser Gly Leu Leu Leu Ile Leu Ile Pro Gln Val Leu Leu
   275 280 285
   Gly Asn Thr Leu Phe Pro Ser Thr Leu Arg Leu Val Leu Trp Leu Leu
   290 295 300
   Gly Lys Phe Ser Lys Lys Ala Glu Ile Asp Tyr Leu Leu Ser Arg Thr
   305 310 315 320
   Gly Glu Ile Gly Tyr Lys His Leu Leu Pro Ser Leu Tyr Ser Ser Leu
   325 330 335
   Leu Val Val Thr Val Leu Gly Phe Ile Gly Val Gln Phe Ile Met Phe
   340 345 350
   Cys Ser Met Gln Trp Asp Ser Glu Ser Leu Asp Gly Leu Asn Ser Tyr
   355 360 365
   Glu Lys Ile Val Ala Val Leu Phe Gln Cys Val Asn Thr Arg Tyr Thr
   370 375 380
   Gly Glu Thr Ile Val Asn Leu Ser Lys Val Ser Pro Ala Ile Leu Val
   385 390 395 400
   Leu Phe Val Val Met Met Tyr Leu Pro Pro Tyr Thr Ser Phe Leu Pro
   405 410 415
   Ala Ile Gly Gly Glu Glu Leu Leu Gly Asn Gly Glu Arg Lys Lys Ala
   420 425 430
   Lys Arg Ser His Lys Leu Met Leu Lys Ser Leu Ile Phe Ser Gln Pro
   435 440 445
   Ser Tyr Leu Ala Ile Phe Ile Ile Thr Ile Cys Ile Thr Glu Lys Glu
   450 455 460
   Lys Met Lys Lys Asp Pro Leu Asn Phe Asn Val Phe Asn Ile Val Val
   465 470 475 480
   Glu Val Ile Ser Ala Tyr Gly Asn Val Gly Phe Thr Ile Gly Tyr Asn
   485 490 495
   Cys Asp Arg Gln Leu Arg Arg Ile Glu Gly Cys Glu Asp Lys Trp Tyr
   500 505 510
   Gly Phe Ser Gly Lys Trp Ser Asp Glu Gly Lys Ile Ile Leu Ile Ala
   515 520 525
   Val Met Ile Phe Gly Arg Leu Lys Lys Phe Asn Met Lys Gly Gly Arg
   530 535 540
   Asn Trp Ile Leu Leu
   545
<210> 19
   <211> 1593
   <212> DNA
   <213> Oryza sativa
<400> 19
   atgacgagca tttaccatga tttcattcat aacaagctgc agagcttcgg aaggatcggt    60
   agatattttg ttaattttgt tgttctagcc catagattca ttgccttgca tattcaccca   120
   ttctggattc agttgtccta cttccttctc attagcatcc taggttcagt tctattgatg   180
   ttcctgaagc caagcaaccc agaattcaga ccaggttaca ttgacatgct gttcttgtca   240
   acctctgctc tgacactttc cagcctcatc accatcgaaa tggaggttct ctcaagctca   300
   caaattgtgg ttattacact gctgatgctt ctcgggggtg aagtctttgt ttctttcctg   360
   ggcctcatgc ttagactgaa ccataagcac aacccagagt tttcagggga caaggtcagt   420
   tcagttccta tcgagcttga tacaatcaac tcggcaagca ctgtgataag ttgtgaagag   480
   ctgcagctgg aagcagcaat tcctgaagtt ccatcttcca ccattaagga tttgaagagg   540
   agcaagcgcc tgaggtggtt cttaggattt gtagtcttca gctattttgt tgtgatccat   600
   gtcgctggct ttctgctggt tctctggtac ataagtcgtg tctcatctgc aaaagctcca   660
   ctgaagaaga aagggatcaa cattgcactc ttctcattct cggtcacggt ctcctcgttt   720
   gcgaatgtgg ggctcgtgcc gacaaatgag aacatggcaa tcttctccaa gaacccgggc   780
   ctcctcctcc tgttcatcgg ccagattctt gcaggcaata cactttaccc tctcttccta   840
   aggctattga tatggttcct ggggaaggtg accaagttga gagaactgaa gctcatgatc   900
   aagaaccctg aggagctaca gtatgattat ttgcttccta agttgccaac ggcgtttctg   960
   gcatcaactg tcattggcct tatggcttcc ttggtcacac tgttcggtgc tgtcgactgg  1020
   aattcttcag tctttgatgg actcagctct taccagaaga ttatcaatgc attgttcatg  1080
   gcagtgaacg caaggcactc gggggagaac tccatcgact gctcactcat cgcccctgct  1140
   gttctagtac tgttcatcat cttaatgtac ttaccaccat cgacaacatt tgcactgtcc  1200
   aatggagatg aaaaaactgc aaataagaaa gcgaaaagaa aattaggttt agtggtgcag  1260
   aacttggcat tttcacagct tgcctgcatc tcagtctttg tgatagttgc attcatcact  1320
   gagaggagtc gtctcagaaa tgatccactc aacttctctg ctctgaacat gatatttgaa  1380
   atcatcagtg catatgggaa tgtagggcta tccactggtt acagctgttc gaggttgcag  1440
   aaactgcacc cggggagcat ctgccaggac aagccgtaca gcttgtccgg atggtggagt  1500
   gatgaaggaa agttgctgct agtctttgtg atgctctatg gaaggctcaa ggccttcaca  1560
   aaaggtacag gtgaatattg gaggctatgg taa                                1593
<210> 20
   <211> 530
   <212> PRT
   <213> Oryza sativa
<400> 20
   Met Thr Ser Ile Tyr His Asp Phe Ile His Asn Lys Leu Gln Ser Phe
   1               5                   10                  15
   Gly Arg Ile Gly Arg Tyr Phe Val Asn Phe Val Val Leu Ala His Arg
               20                  25                  30
   Phe Ile Ala Leu His Ile His Pro Phe Trp Ile Gln Leu Ser Tyr Phe
   35 40 45
   Leu Leu Ile Ser Ile Leu Gly Ser Val Leu Leu Met Phe Leu Lys Pro
   50 55 60
   Ser Asn Pro Glu Phe Arg Pro Gly Tyr Ile Asp Met Leu Phe Leu Ser
   65 70 75 80
   Thr Ser Ala Leu Thr Leu Ser Ser Leu Ile Thr Ile Glu Met Glu Val
   85 90 95
   Leu Ser Ser Ser Gln Ile Val Val Ile Thr Leu Leu Met Leu Leu Gly
   100 105 110
   Gly Glu Val Phe Val Ser Phe Leu Gly Leu Met Leu Arg Leu Asn His
   115 120 125
   Lys His Asn Pro Glu Phe Ser Gly Asp Lys Val Ser Ser Val Pro Ile
   130 135 140
   Glu Leu Asp Thr Ile Asn Ser Ala Ser Thr Val Ile Ser Cys Glu Glu
   145 150 155 160
   Leu Gln Leu Glu Ala Ala Ile Pro Glu Val Pro Ser Ser Thr Ile Lys
   165 170 175
   Asp Leu Lys Arg Ser Lys Arg Leu Arg Trp Phe Leu Gly Phe Val Val
   180 185 190
   Phe Ser Tyr Phe Val Val Ile His Val Ala Gly Phe Leu Leu Val Leu
   195 200 205
   Trp Tyr Ile Ser Arg Val Ser Ser Ala Lys Ala Pro Leu Lys Lys Lys
   210 215 220
   Gly Ile Asn Ile Ala Leu Phe Ser Phe Ser Val Thr Val Ser Ser Phe
   225 230 235 240
   Ala Asn Val Gly Leu Val Pro Thr Asn Glu Asn Met Ala Ile Phe Ser
   245 250 255
   Lys Asn Pro Gly Leu Leu Leu Leu Phe Ile Gly Gln Ile Leu Ala Gly
   260 265 270
   Asn Thr Leu Tyr Pro Leu Phe Leu Arg Leu Leu Ile Trp Phe Leu Gly
   275 280 285
   Lys Val Thr Lys Leu Arg Glu Leu Lys Leu Met Ile Lys Asn Pro Glu
   290 295 300
   Glu Leu Gln Tyr Asp Tyr Leu Leu Pro Lys Leu Pro Thr Ala Phe Leu
   305 310 315 320
   Ala Ser Thr Val Ile Gly Leu Met Ala Ser Leu Val Thr Leu Phe Gly
   325 330 335
   Ala Val Asp Trp Asn Ser Ser Val Phe Asp Gly Leu Ser Ser Tyr Gln
   340 345 350
   Lys Ile Ile Asn Ala Leu Phe Met Ala Val Asn Ala Arg His Ser Gly
           355                 360                 365
   Glu Asn Ser Ile Asp Cys Ser Leu Ile Ala Pro Ala Val Leu Val Leu
       370                 375                 380
   Phe Ile Ile Leu Met Tyr Leu Pro Pro Ser Thr Thr Phe Ala Leu Ser
   385                 390                 395                 400
   Asn Gly Asp Glu Lys Thr Ala Asn Lys Lys Ala Lys Arg Lys Leu Gly
                   405                 410                 415
   Leu Val Val Gln Asn Leu Ala Phe Ser Gln Leu Ala Cys Ile Ser Val
               420                 425                 430
   Phe Val Ile Val Ala Phe Ile Thr Glu Arg Ser Arg Leu Arg Asn Asp
           435                 440                 445
   Pro Leu Asn Phe Ser Ala Leu Asn Met Ile Phe Glu Ile Ile Ser Ala
       450                 455                 460
   Tyr Gly Asn Val Gly Leu Ser Thr Gly Tyr Ser Cys Ser Arg Leu Gln
   465                 470                 475                 480
   Lys Leu His Pro Gly Ser Ile Cys Gln Asp Lys Pro Tyr Ser Leu Ser
                   485                 490                 495
   Gly Trp Trp Ser Asp Glu Gly Lys Leu Leu Leu Val Phe Val Met Leu
               500                 505                 510
   Tyr Gly Arg Leu Lys Ala Phe Thr Lys Gly Thr Gly Glu Tyr Trp Arg
           515                 520                 525
   Leu Trp
       530
<210> 21
   <211> 1593
   <212> DNA
   <213> Oryza sativa
<400> 21
   atgacgagca tttaccaaga attcattcat accaagtgtc agagcttcag aagcattggt     60
   agatatgttc ttcattcaat tgtcctcatc tatagattcg tttccttgca tgttcaccca    120
   ttctggatcc aactgtccta cttcctcctc attagcatcc taggttcagt tctattaatg    180
   tttctgaagc caagcagccc agagttcaag ccaggttaca ttgacatgtt gttcttgtca    240
   acctctgcta tgaccgtttc cggcctcagc accatcgaaa tggaggttct ctcaagctca    300
   caaatcgtgg tccttacact gctgatgctt gttgggggtg aagtctttgt ttctttcctt    360
   ggcctcatgc ttagactgaa gcataagcac aatccagaat tctcagggga cagggtcagt    420
   tcagttccta tcgagctcga tacgatcgag ccgacaagaa ctgtgatgag cagtgaagag    480
   ttgcagatag aagcagcagc tccagatgtt ccatcttcca ccattaagga tctgaagagg    540
   agcaagcgcc tgaggtggtt cttaggattt gtggtcttca gctattttgt tgtcatccat    600
   gtcgttggct ttctgctggt tctctggtac ataagccgtg tctcatctgc aaaggctcca    660
   ctgaagaaga aagggatcaa cattgcactc ttctcattct cggtcaccgt ctcctcgttt    720
   gcgaatgggg ggctcgtgcc gacgaatgag aacatggcaa tcttctccaa gaacccgggc    780
   ctcctcctcc tgttcatcgg ccagattctt gcaggcaaca cactttaccc tctcttccta    840
   aggatactga tatggttcct gggaaaggtg accaagttga aagacctgaa gctcatgatc    900
   aagaactctg atgagctaca gtatgattat ttgcttccta agttgccgac tgcgtttctc    960
   gcatcaactg tcattggcct tatggcttcc ttggtcacac tgttcggttc tgtcgactgg   1020
   aattcttcag tctttgatgg actcagctct taccagaaga ttatcaatgc attgttcatg   1080
   gcagtgaacg caaggcactc gggggagaac tccatcgact gctcactcat cgcccctgct   1140
   gttctagtac tgttcatcat cttaatgtac ttaccaccat cgacaacatt tgcactgtcc   1200
   aatggagatg aaaaaactgc aaataagaaa gcgaaaagaa aattaggttt agtggtgcgg   1260
   aacctggcat tttcacagct tgcctgcaac gcggtctttg tgatagttgc gctcatcact   1320
   gagaggagtc gtctcagaaa tgatccactc aacttctctg ctctgaacat gatatttgaa   1380
   gtcatcagtg catatgggaa tgtaggacta accactggtt acagctgttc gaggttgcaa   1440
   aaactgcacc cggggagcat ctgccaggac aagccgtaca gcttgtccgg atggtggagc   1500
   gatgaaggaa agttgctgct agtctctgtg atgctctatg gaaggctcaa ggccttcacc   1560
   aaaggtacgg gtgaatattg gaggctatgg tag                                1593
<210> 22
   <211> 530
   <212> PRT
   <213> Oryza sativa
<400> 22
   Met Thr Ser Ile Tyr Gln Glu Phe Ile His Thr Lys Cys Gln Ser Phe
   1               5                   10                  15
   Arg Ser Ile Gly Arg Tyr Val Leu His Ser Ile Val Leu Ile Tyr Arg
               20                  25                  30
   Phe Val Ser Leu His Val His Pro Phe Trp Ile Gln Leu Ser Tyr Phe
           35                  40                  45
   Leu Leu Ile Ser Ile Leu Gly Ser Val Leu Leu Met Phe Leu Lys Pro
       50                  55                  60
   Ser Ser Pro Glu Phe Lys Pro Gly Tyr Ile Asp Met Leu Phe Leu Ser
   65                  70                  75                  80
   Thr Ser Ala Met Thr Val Ser Gly Leu Ser Thr Ile Glu Met Glu Val
                   85                  90                  95
   Leu Ser Ser Ser Gln Ile Val Val Leu Thr Leu Leu Met Leu Val Gly
               100                 105                 110
   Gly Glu Val Phe Val Ser Phe Leu Gly Leu Met Leu Arg Leu Lys His
           115                 120                 125
   Lys His Asn Pro Glu Phe Ser Gly Asp Arg Val Ser Ser Val Pro Ile
       130                 135                 140
   Glu Leu Asp Thr Ile Glu Pro Thr Arg Thr Val Met Ser Ser Glu Glu
   145                 150                 155                 160
   Leu Gln Ile Glu Ala Ala Ala Pro Asp Val Pro Ser Ser Thr Ile Lys
                   165                 170                 175
   Asp Leu Lys Arg Ser Lys Arg Leu Arg Trp Phe Leu Gly Phe Val Val
               180                 185                 190
   Phe Ser Tyr Phe Val Val Ile His Val Val Gly Phe Leu Leu Val Leu
           195                 200                 205
   Trp Tyr Ile Ser Arg Val Ser Ser Ala Lys Ala Pro Leu Lys Lys Lys
       210                 215                 220
   Gly Ile Asn Ile Ala Leu Phe Ser Phe Ser Val Thr Val Ser Ser Phe
   225 230 235 240
   Ala Asn Gly Gly Leu Val Pro Thr Asn Glu Asn Met Ala Ile Phe Ser
   245 250 255
   Lys Asn Pro Gly Leu Leu Leu Leu Phe Ile Gly Gln Ile Leu Ala Gly
   260 265 270
   Asn Thr Leu Tyr Pro Leu Phe Leu Arg Ile Leu Ile Trp Phe Leu Gly
   275 280 285
   Lys Val Thr Lys Leu Lys Asp Leu Lys Leu Met Ile Lys Asn Ser Asp
   290 295 300
   Glu Leu Gln Tyr Asp Tyr Leu Leu Pro Lys Leu Pro Thr Ala Phe Leu
   305 310 315 320
   Ala Ser Thr Val Ile Gly Leu Met Ala Ser Leu Val Thr Leu Phe Gly
   325 330 335
   Ser Val Asp Trp Asn Ser Ser Val Phe Asp Gly Leu Ser Ser Tyr Gln
   340 345 350
   Lys Ile Ile Asn Ala Leu Phe Met Ala Val Asn Ala Arg His Ser Gly
   355 360 365
   Glu Asn Ser Ile Asp Cys Ser Leu Ile Ala Pro Ala Val Leu Val Leu
   370 375 380
   Phe Ile Ile Leu Met Tyr Leu Pro Pro Ser Thr Thr Phe Ala Leu Ser
   385 390 395 400
   Asn Gly Asp Glu Lys Thr Ala Asn Lys Lys Ala Lys Arg Lys Leu Gly
   405 410 415
   Leu Val Val Arg Asn Leu Ala Phe Ser Gln Leu Ala Cys Asn Ala Val
   420 425 430
   Phe Val Ile Val Ala Leu Ile Thr Glu Arg Ser Arg Leu Arg Asn Asp
   435 440 445
   Pro Leu Asn Phe Ser Ala Leu Asn Met Ile Phe Glu Val Ile Ser Ala
   450 455 460
   Tyr Gly Asn Val Gly Leu Thr Thr Gly Tyr Ser Cys Ser Arg Leu Gln
   465 470 475 480
   Lys Leu His Pro Gly Ser Ile Cys Gln Asp Lys Pro Tyr Ser Leu Ser
   485 490 495
   Gly Trp Trp Ser Asp Glu Gly Lys Leu Leu Leu Val Ser Val Met Leu
   500 505 510
   Tyr Gly Arg Leu Lys Ala Phe Thr Lys Gly Thr Gly Glu Tyr Trp Arg
   515 520 525
   Leu Trp
   530
<210> 23
   <211> 1530
   <212> DNA
   <213> Oryza sativa
<400> 23
   atgcctattc ggctgcatat ctttgtcaat tctgcaaggc atgccatcaa ctcatcggca     60
   ttcatttgtc ggttcatcgc ataccatctt agcccgcttt tgattcactt atcctatttt    120
   cttatcattg atatacttgg ttttgtttcc ttggtggtgc tcaggccaag caaccacaag    180
   tacaatcctc gctatgttga tatgtttttc ctatcgacat ctgcagttac agtcatagga    240
   ttagccacca tacaaatgga ggatctttcc agctctcaga tagctatcct gactctcttg    300
   atgttcttag acagtaagat gttcctttcc tttcttggcc ttgtccttga gtcaagcaag    360
   caaaacaagc atgatcctga aaaccgtaga gtcagttcag ttactgtgtg caagcaatca    420
   cagctagaag aggcgactcc ccaaactcca tccatgaact ccattgatat caagaagaga    480
   tgcctcaaat acttagtgtt tgtggtgctg gcatacatga tcattattct tgtcactggt    540
   tctctattgg tgttcatgta catagctcat gtttcaagtg ctagagatgt gctaacaagg    600
   aaaagcatca acaaagctct cttctcgata tcggtcacag tctcctcatt cacaaatgga    660
   gggttattgc cgacaaatga gagtatggtt gtattctcct caaacaatgg cctcctgttg    720
   ctactcattg gccagattct tgcaggcagc acactgttcc ctgtgtttct gaggttggtg    780
   atatgggcat tgagaggact aagattagca aaagctgaag agcctgactt catgatgaac    840
   aacagcagcg cagtaggttt cagtcacctg ctgcctaact tgcagacaat atttcttgca    900
   gttgtggagg ttgcttttgt agccatgaca gtcatcctct tctgctgctt gaactgggat    960
   tctgtagtgt ttgcagggct cagctccctc cagaagataa ccaatgcatt gttcatggca   1020
   gtgaatgcaa ggcaggcagg agaaaattcc attgattgtt cccttgttgc gccagcggct   1080
   ttagtactat tcatggtcat gatgtacact ccatccttga caaagttgtt ctcagcatgt   1140
   caagatcaca aacgaatcgg cccagaaagc gacgatagaa caagcaaggg gaaaccattc   1200
   ctgaagatga tggcattttc accactcggc ttcaacacca cagtgataat gctggtctgc   1260
   atcactgaaa ggagatcact ctccactgac cctctcaatt tatccacatt taacatcatc   1320
   tttgaggtga taagtgccta cgggaacatt gggttgtcca ctggctacag ctgctcgagg   1380
   cagctgcagc accaggaggg gattgcctgc catgagaagg cgtacaactt ctcggggtgg   1440
   tggagtgaac cagggaaact gattcttgtt cttgcaatgc tctgcgggag gctcaactca   1500
   aaggattcca caagcgcacg aactaggtga                                     1530
<210> 24
   <211> 509
   <212> PRT
   <213> Oryza sativa
<400> 24
   Met Pro Ile Arg Leu His Ile Phe Val Asn Ser Ala Arg His Ala Ile
   1               5                   10                  15
   Asn Ser Ser Ala Phe Ile Cys Arg Phe Ile Ala Tyr His Leu Ser Pro
               20                  25                  30
   Leu Leu Ile His Leu Ser Tyr Phe Leu Ile Ile Asp Ile Leu Gly Phe
           35                  40                  45
   Val Ser Leu Val Val Leu Arg Pro Ser Asn His Lys Tyr Asn Pro Arg
       50                  55                  60
   Tyr Val Asp Met Phe Phe Leu Ser Thr Ser Ala Val Thr Val Ile Gly
   65                  70                  75                  80
   Leu Ala Thr Ile Gln Met Glu Asp Leu Ser Ser Ser Gln Ile Ala Ile
                   85                  90                  95
   Leu Thr Leu Leu Met Phe Leu Asp Ser Lys Met Phe Leu Ser Phe Leu
                   100                 105                 110
   Gly Leu Val Leu Glu Ser Ser Lys Gln Asn Lys His Asp Pro Glu Asn
               115                 120                 125
   Arg Arg Val Ser Ser Val Thr Val Cys Lys Gln Ser Gln Leu Glu Glu
       130                 135                 140
   Ala Thr Pro Gln Thr Pro Ser Met Asn Ser Ile Asp Ile Lys Lys Arg
   145                 150                 155                 160
   Cys Leu Lys Tyr Leu Val Phe Val Val Leu Ala Tyr Met Ile Ile Ile
                   165                 170                 175
   Leu Val Thr Gly Ser Leu Leu Val Phe Met Tyr Ile Ala His Val Ser
               180                 185                 190
   Ser Ala Arg Asp Val Leu Thr Arg Lys Ser Ile Asn Lys Ala Leu Phe
           195                 200                 205
   Ser Ile Ser Val Thr Val Ser Ser Phe Thr Asn Gly Gly Leu Leu Pro
       210                 215                 220
   Thr Asn Glu Ser Met Val Val Phe Ser Ser Asn Asn Gly Leu Leu Leu
   225                 230                 235                 240
   Leu Leu Ile Gly Gln Ile Leu Ala Gly Ser Thr Leu Phe Pro Val Phe
                   245                 250                 255
   Leu Arg Leu Val Ile Trp Ala Leu Arg Gly Leu Arg Leu Ala Lys Ala
               260                 265                 270
   Glu Glu Pro Asp Phe Met Met Asn Asn Ser Ser Ala Val Gly Phe Ser
           275                 280                 285
   His Leu Leu Pro Asn Leu Gln Thr Ile Phe Leu Ala Val Val Glu Val
       290                 295                 300
   Ala Phe Val Ala Met Thr Val Ile Leu Phe Cys Cys Leu Asn Trp Asp
   305                 310                 315                 320
   Ser Val Val Phe Ala Gly Leu Ser Ser Leu Gln Lys Ile Thr Asn Ala
                   325                 330                 335
   Leu Phe Met Ala Val Asn Ala Arg Gln Ala Gly Glu Asn Ser Ile Asp
               340                 345                 350
   Cys Ser Leu Val Ala Pro Ala Ala Leu Val Leu Phe Met Val Met Met
           355                 360                 365
   Tyr Thr Pro Ser Leu Thr Lys Leu Phe Ser Ala Cys Gln Asp His Lys
       370                 375                 380
   Arg Ile Gly Pro Glu Ser Asp Asp Arg Thr Ser Lys Gly Lys Pro Phe
   385                 390                 395                 400
   Leu Lys Met Met Ala Phe Ser Pro Leu Gly Phe Asn Thr Thr Val Ile
                   405                 410                 415
   Met Leu Val Cys Ile Thr Glu Arg Arg Ser Leu Ser Thr Asp Pro Leu
               420                 425                 430
   Asn Leu Ser Thr Phe Asn Ile Ile Phe Glu Val Ile Ser Ala Tyr Gly
           435                 440                 445
   Asn Ile Gly Leu Ser Thr Gly Tyr Ser Cys Ser Arg Gln Leu Gln His
       450                 455                 460
   Gln Glu Gly Ile Ala Cys His Glu Lys Ala Tyr Asn Phe Ser Gly Trp
   465                 470                 475                 480
   Trp Ser Glu Pro Gly Lys Leu Ile Leu Val Leu Ala Met Leu Cys Gly
                   485                 490                 495
   Arg Leu Asn Ser Lys Asp Ser Thr Ser Ala Arg Thr Arg
               500                 505
<210> 25
   <211> 1659
   <212> DNA
   <213> Oryza sativa
<400> 25
   atgcatccac caagtttagt gctagatacc ttgaagcgta tcaaactata catagccatg      60
   aagctcctgt taccgaattc ggaggtgcct cggatctatt gggagaaagc tcagcatctc     120
   tgtgggttcc tgtccatgaa gctcatttcc agagccagat gtgtggcaag ttctgtcaaa     180
   caatcttata gttttctggt ttgcaaaagt aaccctttgg tagttcagct cgtttacttt     240
   gtgataatct catttgctgg tttccttgct ctgaagaatc tcaagcccca gggtaagcca     300
   ggtccaaagg atttggacct attgttcacc tctgtgtcta cacttactgt ctcgagcatg     360
   gcaacagtag agatggaaga cttatctgac aggcaactct gggttctgat ccttctgatg     420
   ctaatgggag gagaggtgtt cacatcaatg ctagggctct acttcaacaa tgccaatgcc     480
   aacagaaatg agaacagcca gagaagttta ccttcaatca gcttggacat tgaattcaac     540
   agtcctgcaa acaatgggga tcacaaaatt acggaatgtg gccaatcaga agaaactatg     600
   tcgcaaaacc aggtacagca aaacaaaagc ataacatata atccttgcgc tgtgttggtt     660
   cgcatagtga caggttattt cgtagctact gtcatttcca gttctgtcat cattattatt     720
   tacttttgga ttgattcaga tgcaagaaat gtactgaaaa gtaaggagat caatatgtat     780
   accttttgca tcttcacagc agtgtcctcg ttcgcaaact gtggcttcac gccactaaat     840
   agtaacatgc aacccttcag aaagaactgg gtccttttgc tcctagtgat cccgcagatt     900
   ctagcaggca acaccttgtt ttcaccactc ttgcggctat gcgtatgggt tttggggaag     960
   gtcagtggaa aagcagagta tgcttacatc cttcagcatc ctggggagac tggctacaaa    1020
   catttgcatg tgaggagaaa ttctgtttac attgttctga gtgttactgg actgattcta    1080
   ctgcaagtaa tgtttatttg ctcgtttgag tggaactcag agagcttaga gggaatgaat    1140
   tggttacaga aattggtagg attattattc cagagtgtta atacaagaca agccggtgaa    1200
   tctatccttg atatatcaac actttctcca tcaactctgt tgctatttgc agtcgtcatg    1260
   taccttcctt ccgatgcttc atttctcact gccaatgctg ataaccagcc cttgacagat    1320
   aaaaagacaa actcgattag cagagcactg tggaggaatt ttaccgttaa caagctttct    1380
   tgtttagcaa tgttcacatt cctggcatgc ataacagaaa ggaagtcaat ttcttctgat    1440
   ccactaaatt tcaacatctt cagcatagtt tttgagataa tcagcgcttt cggcaatgta    1500
   ggctactcac taggatacag ttgccagaag ttgttgaagc ctgatgccac ttgcaaagat    1560
   gcttcctacg ggttcgtcgg caggtggacc gaggagggta agctgatagt tatcctggtg    1620
   atgttcctgg ggaggctcaa ggagttcatc ctgaaatga                           1659
<210> 26
   <211> 552
   <212> PRT
   <213> Oryza sativa
<400> 26
   Met His Pro Pro Ser Leu Val Leu Asp Thr Leu Lys Arg Ile Lys Leu
   1               5                   10                  15
   Tyr Ile Ala Met Lys Leu Leu Leu Pro Asn Ser Glu Val Pro Arg Ile
               20                  25                  30
   Tyr Trp Glu Lys Ala Gln His Leu Cys Gly Phe Leu Ser Met Lys Leu
           35                  40                  45
   Ile Ser Arg Ala Arg Cys Val Ala Ser Ser Val Lys Gln Ser Tyr Ser
       50                  55                  60
   Phe Leu Val Cys Lys Ser Asn Pro Leu Val Val Gln Leu Val Tyr Phe
   65                  70                  75                  80
   Val Ile Ile Ser Phe Ala Gly Phe Leu Ala Leu Lys Asn Leu Lys Pro
                   85                  90                  95
   Gln Gly Lys Pro Gly Pro Lys Asp Leu Asp Leu Leu Phe Thr Ser Val
               100                 105                 110
   Ser Thr Leu Thr Val Ser Ser Met Ala Thr Val Glu Met Glu Asp Leu
           115                 120                 125
   Ser Asp Arg Gln Leu Trp Val Leu Ile Leu Leu Met Leu Met Gly Gly
       130                 135                 140
   Glu Val Phe Thr Ser Met Leu Gly Leu Tyr Phe Asn Asn Ala Asn Ala
   145                 150                 155                 160
   Asn Arg Asn Glu Asn Ser Gln Arg Ser Leu Pro Ser Ile Ser Leu Asp
                   165                 170                 175
   Ile Glu Phe Asn Ser Pro Ala Asn Asn Gly Asp His Lys Ile Thr Glu
               180                 185                 190
   Cys Gly Gln Ser Glu Glu Thr Met Ser Gln Asn Gln Val Gln Gln Asn
           195                 200                 205
   Lys Ser Ile Thr Tyr Asn Pro Cys Ala Val Leu Val Arg Ile Val Thr
       210                 215                 220
   Gly Tyr Phe Val Ala Thr Val Ile Ser Ser Ser Val Ile Ile Ile Ile
   225                 230                 235                 240
   Tyr Phe Trp Ile Asp Ser Asp Ala Arg Asn Val Leu Lys Ser Lys Glu
                   245                 250                 255
   Ile Asn Met Tyr Thr Phe Cys Ile Phe Thr Ala Val Ser Ser Phe Ala
               260                 265                 270
   Asn Cys Gly Phe Thr Pro Leu Asn Ser Asn Met Gln Pro Phe Arg Lys
           275                 280                 285
   Asn Trp Val Leu Leu Leu Leu Val Ile Pro Gln Ile Leu Ala Gly Asn
       290                 295                 300
   Thr Leu Phe Ser Pro Leu Leu Arg Leu Cys Val Trp Val Leu Gly Lys
   305                 310                 315                 320
   Val Ser Gly Lys Ala Glu Tyr Ala Tyr Ile Leu Gln His Pro Gly Glu
                   325                 330                 335
   Thr Gly Tyr Lys His Leu His Val Arg Arg Asn Ser Val Tyr Ile Val
               340                 345                 350
   Leu Ser Val Thr Gly Leu Ile Leu Leu Gln Val Met Phe Ile Cys Ser
           355                 360                 365
   Phe Glu Trp Asn Ser Glu Ser Leu Glu Gly Met Asn Trp Leu Gln Lys
       370                 375                 380
   Leu Val Gly Leu Leu Phe Gln Ser Val Asn Thr Arg Gln Ala Gly Glu
   385                 390                 395                 400
   Ser Ile Leu Asp Ile Ser Thr Leu Ser Pro Ser Thr Leu Leu Leu Phe
                   405                 410                 415
   Ala Val Val Met Tyr Leu Pro Ser Asp Ala Ser Phe Leu Thr Ala Asn
               420                 425                 430
   Ala Asp Asn Gln Pro Leu Thr Asp Lys Lys Thr Asn Ser Ile Ser Arg
           435                 440                 445
   Ala Leu Trp Arg Asn Phe Thr Val Asn Lys Leu Ser Cys Leu Ala Met
       450                 455                 460
   Phe Thr Phe Leu Ala Cys Ile Thr Glu Arg Lys Ser Ile Ser Ser Asp
   465                 470                 475                 480
   Pro Leu Asn Phe Asn Ile Phe Ser Ile Val Phe Glu Ile Ile Ser Ala
                   485                 490                 495
   Phe Gly Asn Val Gly Tyr Ser Leu Gly Tyr Ser Cys Gln Lys Leu Leu
               500                 505                 510
   Lys Pro Asp Ala Thr Cys Lys Asp Ala Ser Tyr Gly Phe Val Gly Arg
           515                 520                 525
   Trp Thr Glu Glu Gly Lys Leu Ile Val Ile Leu Val Met Phe Leu Gly
       530                 535                 540
   Arg Leu Lys Glu Phe Ile Leu Lys
   545                 550
<210> 27
   <211> 1596
   <212> DNA
   <213> Oryza sativa
<400> 27
   atgaatcatt gtcttgtagt atcccacaaa aaactccaaa ctttccgcac atttgcagct     60
   agcaagttct cttcttttac caaatctgca cagaagtcta taaaatactc cttccagttc    120
   atctaccaaa acaatccact ctttgtccat gtagcttact ttgccctgat ctcctttgct    180
   ggatatggat ctctaaaggt cctcaagcca cgagacaagt caaatactct gaaagacttg    240
   gacgtgctat ttacttccgt atctgcatca actgtttcaa gcatggctac tgttgaaatg    300
   gaggatttct caagcgctca actctgggtt ttgactattt taatgctgat tggtggtgag    360
   gtattcactt caatgcttgg cattcacttt atgagagccg aatttggtac aaaagagtca    420
   gtcagcacaa gggatcactc accttgcatt gatattgagt ctattacttc cacaaaattt    480
   ggtcccagca cccagggcac aaaagttaca gtttcatttt ctgaactccg catggaaaat    540
   ggaggacatg tagagcccaa gacgattaaa tttttaggtt ttgtagtgat gggatatctt    600
   ctaataacaa acttaggcgg ctccctactt atttacctct accttaacct ggtaccaagt    660
   gcacataaaa ttctaaagag aaaaggcatt gggatcatcg tattctcagt atttacagcc    720
   atctcctcag ttggaaattg tggcttcact ccagtaaatg agaatatgat tatctttcag    780
   aagaactcca ttcttctatt gctaattctt cctcagatac tagcaggaaa tacattattt    840
   gcaccatgct tgagattaat ggtgtggtca cttgagaaga ttaccggaaa aaaggattgt    900
   cgttacattc ttgaatatcc aaaggccatt ggatataaac atcttatgag taccagggaa    960
   agtgtttatt tgactttaac agttgtgagc ttgatcattc tgcaaaccgt attgttcctc   1020
   tctttggagt ggagctcggt agctttggat ggaatgagca actatcaaaa gatagtatcc   1080
   gctctatttc agtcggtcaa tgctaggcat gcaggtgaat ctgttacaga tctgtcaaac   1140
   ctctcttcag caatcctagt cctatacacc atcatgatgt atctccctgg ttacacttcg   1200
   tttttaccca gacatgatgg tgaggattct aagaccgaga agataaacaa aagaaaaggg   1260
   ctattggaga actggatctt ctcacatatg tcttatttgg ctatctttgt aatgctaatt   1320
   tgcatcacag aacgggactc gatggctaca gatccactta atttcaatgt tttcagcata   1380
   ttgtttgaag tcgtcagtgc atatggaaat gtggggttct cggttggcta cagctgcaag   1440
   aggctactga accatgatgc acgctgcaag gatgcctcgt acgggtttgc ggggaaatgg   1500
   agcgacaatg ggaaagcgat cctgatcatc gtcatgcttt tcgggaggct taaaacgttt   1560
   aacatgaagg gtggaagagc ctggaagctt agataa                             1596
<210> 28
   <211> 531
   <212> PRT
   <213> Oryza sativa
<400> 28
   Met Asn His Cys Leu Val Val Ser His Lys Lys Leu Gln Thr Phe Arg
   1               5                   10                  15
   Thr Phe Ala Ala Ser Lys Phe Ser Ser Phe Thr Lys Ser Ala Gln Lys
               20                  25                  30
   Ser Ile Lys Tyr Ser Phe Gln Phe Ile Tyr Gln Asn Asn Pro Leu Phe
           35                  40                  45
   Val His Val Ala Tyr Phe Ala Leu Ile Ser Phe Ala Gly Tyr Gly Ser
       50                  55                  60
   Leu Lys Val Leu Lys Pro Arg Asp Lys Ser Asn Thr Leu Lys Asp Leu
   65                  70                  75                  80
   Asp Val Leu Phe Thr Ser Val Ser Ala Ser Thr Val Ser Ser Met Ala
                   85                  90                  95
   Thr Val Glu Met Glu Asp Phe Ser Ser Ala Gln Leu Trp Val Leu Thr
               100                 105                 110
   Ile Leu Met Leu Ile Gly Gly Glu Val Phe Thr Ser Met Leu Gly Ile
           115                 120                 125
   His Phe Met Arg Ala Glu Phe Gly Thr Lys Glu Ser Val Ser Thr Arg
       130                 135                 140
   Asp His Ser Pro Cys Ile Asp Ile Glu Ser Ile Thr Ser Thr Lys Phe
   145                 150                 155                 160
   Gly Pro Ser Thr Gln Gly Thr Lys Val Thr Val Ser Phe Ser Glu Leu
                   165                 170                 175
   Arg Met Glu Asn Gly Gly His Val Glu Pro Lys Thr Ile Lys Phe Leu
               180                 185                 190
   Gly Phe Val Val Met Gly Tyr Leu Leu Ile Thr Asn Leu Gly Gly Ser
           195                 200                 205
   Leu Leu Ile Tyr Leu Tyr Leu Asn Leu Val Pro Ser Ala His Lys Ile
       210                 215                 220
   Leu Lys Arg Lys Gly Ile Gly Ile Ile Val Phe Ser Val Phe Thr Ala
   225                 230                 235                 240
   Ile Ser Ser Val Gly Asn Cys Gly Phe Thr Pro Val Asn Glu Asn Met
                   245                 250                 255
   Ile Ile Phe Gln Lys Asn Ser Ile Leu Leu Leu Leu Ile Leu Pro Gln
               260                 265                 270
   Ile Leu Ala Gly Asn Thr Leu Phe Ala Pro Cys Leu Arg Leu Met Val
           275                 280                 285
   Trp Ser Leu Glu Lys Ile Thr Gly Lys Lys Asp Cys Arg Tyr Ile Leu
       290                 295                 300
   Glu Tyr Pro Lys Ala Ile Gly Tyr Lys His Leu Met Ser Thr Arg Glu
   305                 310                 315                 320
   Ser Val Tyr Leu Thr Leu Thr Val Val Ser Leu Ile Ile Leu Gln Thr
                   325                 330                 335
   Val Leu Phe Leu Ser Leu Glu Trp Ser Ser Val Ala Leu Asp Gly Met
               340                 345                 350
   Ser Asn Tyr Gln Lys Ile Val Ser Ala Leu Phe Gln Ser Val Asn Ala
           355                 360                 365
   Arg His Ala Gly Glu Ser Val Thr Asp Leu Ser Asn Leu Ser Ser Ala
       370                 375                 380
   Ile Leu Val Leu Tyr Thr Ile Met Met Tyr Leu Pro Gly Tyr Thr Ser
   385                 390                 395                 400
   Phe Leu Pro Arg His Asp Gly Glu Asp Ser Lys Thr Glu Lys Ile Asn
                   405                 410                 415
   Lys Arg Lys Gly Leu Leu Glu Asn Trp Ile Phe Ser His Met Ser Tyr
               420                 425                 430
   Leu Ala Ile Phe Val Met Leu Ile Cys Ile Thr Glu Arg Asp Ser Met
           435                 440                 445
   Ala Thr Asp Pro Leu Asn Phe Asn Val Phe Ser Ile Leu Phe Glu Val
       450                 455                 460
   Val Ser Ala Tyr Gly Asn Val Gly Phe Ser Val Gly Tyr Ser Cys Lys
   465                 470                 475                 480
   Arg Leu Leu Asn His Asp Ala Arg Cys Lys Asp Ala Ser Tyr Gly Phe
   485 490 495
   Ala Gly Lys Trp Ser Asp Asn Gly Lys Ala Ile Leu Ile Ile Val Met
   500 505 510
   Leu Phe Gly Arg Leu Lys Thr Phe Asn Met Lys Gly Gly Arg Ala Trp
   515 520 525
   Lys Leu Arg
   530
<210> 29
   <211> 1503
   <212> DNA
   <213> Oryza sativa
<400> 29
   atgcccacgt cgcggcgcgc gctcgccggc ggcgctttgt ccatgcacgt cgcctacttc     60
   ctcgccatct cctgcctcgg ctacggcctc ctcggggtgc tcaaggtgcg ggagcccggc    120
   gccgcgccgc gccgcatcga ccgcttcttc accgccgtgt cggccgccac ggtgtcgagc    180
   atgtccaccg tcgagatgga ggtgttctcc aacggccagc tcgtggtgct caccgtcctg    240
   atgctcctcg gcggcgaggt gttcgtctcg ctcgtggggc tcgcgtccaa gtggtccaag    300
   ctgcggagcg acgccatgga cagatcccgg cgcgtcgaga gccacggcga cgtcgcgctc    360
   gccgacatcg acggcggcga cgtcgagaac ccgacgtcgt cgggagagga ggcggcgagc    420
   cgacgccgcc cgatggacgc ggacacgctg cggcacaatg cggtgcgcgc gctgttctac    480
   atcgtgctcg ccatcttcgc ggtggtgcac gtcgtcggcg ccgtggccgt cgcggcgtac    540
   gtgctcgcgt cgccgggcgc gaggcggacg ctgggggaca agtcgctgaa cacgtggacg    600
   ttcgcggtgt tcacgacggt gtcgacgttc tccaactgcg ggttcatgcc gacgaacgag    660
   aacatggtgg tgttcaagcg ggacgcgccg ctgcagctgc tgctggtgcc gcaggtgctg    720
   gcggggaaca cgctgttcgc gccgctgctg gcggcgtgcg tgtgggcggc ggcggcggcg    780
   acgcggcggg aggagctcgt ggagatggcg agggaggggg gcagggcggc ggcggccggg    840
   tacgcgcacc tgatgccggc gcggcggtgc tggatgctgg cggcgacggt ggcggcgttc    900
   gtcgccgtgc tgatggcgct ggtgtgcggc atggagtggg gcggggcgct gcaggggatg    960
   agcccgtggg agaaggtggt gaacgcgctg ttcctcgccg tgaacgcccg gcacaccggc   1020
   gagtccaccg tcgacctctc catcctcgcg ccggccatcc tcgtgctctt cgtcctcatg   1080
   atgtatctac ctccgtacac gacgtggttc ccatttgaag agaattccac tactaaggat   1140
   agtaatgcag agaaccaggg aatcagactg ctcgagagta cacttttgtc acaactctcc   1200
   tacctgacca tctttgtcat tgccatctgc atcaccgaga gaagaaagct caaagaagac   1260
   cccctcaact tcagtgtgct aagcattgtt gtcgaagttg tcagtgcata tggaaatgtg   1320
   gggttctcaa tgggctacag ttgcagtaga cagatcaatc cagaccatct ctgcacagat   1380
   aaatggactg gtttcgtagg gagatggagc gactctggca aactgatact catttttgtg    1440
   atgttctttg ggaggctcaa gaagttcagc atgaaaggag gcaaagcctg gaaacttagt   1500
   tag                                                                 1503
<210> 30
   <211> 500
   <212> PRT
   <213> Oryza sativa
<400> 30
   Met Pro Thr Ser Arg Arg Ala Leu Ala Gly Gly Ala Leu Ser Met His
   1               5                   10                  15
   Val Ala Tyr Phe Leu Ala Ile Ser Cys Leu Gly Tyr Gly Leu Leu Gly
               20                  25                  30
   Val Leu Lys Val Arg Glu Pro Gly Ala Ala Pro Arg Arg Ile Asp Arg
           35                  40                  45
   Phe Phe Thr Ala Val Ser Ala Ala Thr Val Ser Ser Met Ser Thr Val
       50                  55                  60
   Glu Met Glu Val Phe Ser Asn Gly Gln Leu Val Val Leu Thr Val Leu
   65                  70                  75                  80
   Met Leu Leu Gly Gly Glu Val Phe Val Ser Leu Val Gly Leu Ala Ser
                   85                  90                  95
   Lys Trp Ser Lys Leu Arg Ser Asp Ala Met Asp Arg Ser Arg Arg Val
               100                 105                 110
   Glu Ser His Gly Asp Val Ala Leu Ala Asp Ile Asp Gly Gly Asp Val
           115                 120                 125
   Glu Asn Pro Thr Ser Ser Gly Glu Glu Ala Ala Ser Arg Arg Arg Pro
       130                 135                 140
   Met Asp Ala Asp Thr Leu Arg His Asn Ala Val Arg Ala Leu Phe Tyr
   145                 150                 155                 160
   Ile Val Leu Ala Ile Phe Ala Val Val His Val Val Gly Ala Val Ala
                   165                 170                 175
   Val Ala Ala Tyr Val Leu Ala Ser Pro Gly Ala Arg Arg Thr Leu Gly
               180                 185                 190
   Asp Lys Ser Leu Asn Thr Trp Thr Phe Ala Val Phe Thr Thr Val Ser
           195                 200                 205
   Thr Phe Ser Asn Cys Gly Phe Met Pro Thr Asn Glu Asn Met Val Val
       210                 215                 220
   Phe Lys Arg Asp Ala Pro Leu Gln Leu Leu Leu Val Pro Gln Val Leu
   225                 230                 235                 240
   Ala Gly Asn Thr Leu Phe Ala Pro Leu Leu Ala Ala Cys Val Trp Ala
                   245                 250                 255
   Ala Ala Ala Ala Thr Arg Arg Glu Glu Leu Val Glu Met Ala Arg Glu
               260                 265                 270
   Gly Gly Arg Ala Ala Ala Ala Gly Tyr Ala His Leu Met Pro Ala Arg
           275                 280                 285
   Arg Cys Trp Met Leu Ala Ala Thr Val Ala Ala Phe Val Ala Val Leu
       290                 295                 300
   Met Ala Leu Val Cys Gly Met Glu Trp Gly Gly Ala Leu Gln Gly Met
   305                 310                 315                 320
   Ser Pro Trp Glu Lys Val Val Asn Ala Leu Phe Leu Ala Val Asn Ala
                   325                 330                 335
   Arg His Thr Gly Glu Ser Thr Val Asp Leu Ser Ile Leu Ala Pro Ala
               340                 345                 350
   Ile Leu Val Leu Phe Val Leu Met Met Tyr Leu Pro Pro Tyr Thr Thr
           355                 360                 365
   Trp Phe Pro Phe Glu Glu Asn Ser Thr Thr Lys Asp Ser Asn Ala Glu
       370                 375                 380
   Asn Gln Gly Ile Arg Leu Leu Glu Ser Thr Leu Leu Ser Gln Leu Ser
   385                 390                 395                 400
   Tyr Leu Thr Ile Phe Val Ile Ala Ile Cys Ile Thr Glu Arg Arg Lys
                   405                 410                 415
   Leu Lys Glu Asp Pro Leu Asn Phe Ser Val Leu Ser Ile Val Val Glu
               420                 425                 430
   Val Val Ser Ala Tyr Gly Asn Val Gly Phe Ser Met Gly Tyr Ser Cys
           435                 440                 445
   Ser Arg Gln Ile Asn Pro Asp His Leu Cys Thr Asp Lys Trp Thr Gly
       450                 455                 460
   Phe Val Gly Arg Trp Ser Asp Ser Gly Lys Leu Ile Leu Ile Phe Val
   465                 470                 475                 480
   Met Phe Phe Gly Arg Leu Lys Lys Phe Ser Met Lys Gly Gly Lys Ala
                   485                 490                 495
   Trp Lys Leu Ser
               500
<210> 31
   <211> 1665
   <212> DNA
   <213> Oryza sativa
<400> 31
   atgagttctc tggatgccac tactcctaga tatgacgagt ttaaaaggat ctaccacctt 60
   ttccttttcc atgcacaccc attctggctc caactgctgt acttcctctt catctccctc 120
   ttgggtttct tgatgctgaa agctctgccg atgaagacca gcatggtgcc gaggcccatg 180
   gacttggacc tgatcttcac gtcggtgtcg gcgacgacgg tgtcgagcat ggtcgccgtc 240
   gagatggagt ccttctccaa ctcccagctc ctcctcatca ccctcctcat gctgcttggt 300
   ggtgaggtct tcaccagcat ccttggcctc tacttcacca acgccaagta ctcctccaag 360
   atgatagcaa ccttacctga tgatgacgac catggtggca gtggcaaacc accaccacca 420
   acgacgtcac cttcgtctac cctagtggag ctcgagctcg ctcctcccat ggacgtcgtc 480
   gtcgtcaacc ctaccaccac tgcgacgacg cacgacgagg tagagctagg gttaggacgt 540
   cggaacaagc gcggctgcac ctgcactact actcacacgt cgtcgtcatc atcggcatcg 600
   aagacgacga cgacgaggct actgatgttc gtggtgatgg ggtaccacgc ggtggtgcac 660
   gtcgccgggt acacggccat cgtcgtgtac ctcagcgccg tcggcggcgc gggggcggtg 720
   gtcgccggga aggggatcag cgcgcacacg ttcgccatct tcaccgtcgt ctcgacgttc 780
   gccaactgcg ggttcgtgcc gacgaacgaa gggatggtgt cgttcaggtc gttcccgggg 840
   ctcctcctcc tcgtcatgcc gcacgtcctc ctcgggaaca cgctcttccc ggtcttcctc 900
   aggctggcca tcgccgcgct cgagagggtc accgggtggc cggagctcgg cgagctcctg 960
   atccggcggc ggaggggcgg cggcgagggc taccaccagc tgttgccgag ctcgcgcacg 1020
   cggttcctgg ccctcaccgt ggccgtgctc gtggtggcgc agctggcgct cttctgcgcc 1080
   atggagtggg gctccgacgg gctgcggggg ctcaccgcgg gccagaagct cgtcggcgcg 1140
   ctcttcatgg cggtcaactc gaggcactcc ggtgagatgg tgctcgacct ctccaccgtg 1200
   tcgtcggccg tcgtcgtgct ctacgtggtg atgatgtacc tgccacctta caccactttt 1260
   gtacctgtcc aagacaaaca ccagcaaacg ggagcacagt ccgggcagga gggcagcagc 1320
   agcagcagca tatggcagaa gctgctcatg tcgccgctct cgtgcctagc catcttcatc 1380
   gtcgtcatct gcatcacgga gcggcggcaa atcgccgacg accccatcaa ctacagcgtc 1440
   ctcaacatcg tcgtcgaggt tatcagtgcg tatggcaatg tggggttcag cacggggtac 1500
   agctgcgcga ggcaggtgag gcccgacggc agctgcagag acctgtgggt tggcttctca 1560
   gggaagtgga gcaaacaagg gaagctcact ctcatggccg tcatgttcta cggcaggctc 1620
   aagaagttca gcctgcacgg tggtcaggca tggaagatag aataa 1665
<210> 32
   <211> 554
   <212> PRT
   <213> Oryza sativa
<400> 32
   Met Ser Ser Leu Asp Ala Thr Thr Pro Arg Tyr Asp Glu Phe Lys Arg
   1               5                   10                  15
   Ile Tyr His Leu Phe Leu Phe His Ala His Pro Phe Trp Leu Gln Leu
               20                  25                  30
   Leu Tyr Phe Leu Phe Ile Ser Leu Leu Gly Phe Leu Met Leu Lys Ala
           35                  40                  45
   Leu Pro Met Lys Thr Ser Met Val Pro Arg Pro Met Asp Leu Asp Leu
       50                  55                  60
   Ile Phe Thr Ser Val Ser Ala Thr Thr Val Ser Ser Met Val Ala Val
   65                  70                  75                  80
   Glu Met Glu Ser Phe Ser Asn Ser Gln Leu Leu Leu Ile Thr Leu Leu
                   85                  90                  95
   Met Leu Leu Gly Gly Glu Val Phe Thr Ser Ile Leu Gly Leu Tyr Phe
               100                 105                 110
   Thr Asn Ala Lys Tyr Ser Ser Lys Met Ile Ala Thr Leu Pro Asp Asp
           115                 120                 125
   Asp Asp His Gly Gly Ser Gly Lys Pro Pro Pro Pro Thr Thr Ser Pro
       130                 135                 140
   Ser Ser Thr Leu Val Glu Leu Glu Leu Ala Pro Pro Met Asp Val Val
   145                 150                 155                 160
   Val Val Asn Pro Thr Thr Thr Ala Thr Thr His Asp Glu Val Glu Leu
                   165                 170                 175
   Gly Leu Gly Arg Arg Asn Lys Arg Gly Cys Thr Cys Thr Thr Thr His
               180                 185                 190
   Thr Ser Ser Ser Ser Ser Ala Ser Lys Thr Thr Thr Thr Arg Leu Leu
           195                 200                 205
   Met Phe Val Val Met Gly Tyr His Ala Val Val His Val Ala Gly Tyr
       210                 215                 220
   Thr Ala Ile Val Val Tyr Leu Ser Ala Val Gly Gly Ala Gly Ala Val
   225                 230                 235                 240
   Val Ala Gly Lys Gly Ile Ser Ala His Thr Phe Ala Ile Phe Thr Val
                   245                 250                 255
   Val Ser Thr Phe Ala Asn Cys Gly Phe Val Pro Thr Asn Glu Gly Met
   260 265 270
   Val Ser Phe Arg Ser Phe Pro Gly Leu Leu Leu Leu Val Met Pro His
   275 280 285
   Val Leu Leu Gly Asn Thr Leu Phe Pro Val Phe Leu Arg Leu Ala Ile
   290 295 300
   Ala Ala Leu Glu Arg Val Thr Gly Trp Pro Glu Leu Gly Glu Leu Leu
   305 310 315 320
   Ile Arg Arg Arg Arg Gly Gly Gly Glu Gly Tyr His His Leu Leu Pro
   325 330 335
   Ser Ser Arg Thr Arg Phe Leu Ala Leu Thr Val Ala Val Leu Val Val
   340 345 350
   Ala Gln Leu Ala Leu Phe Cys Ala Met Glu Trp Gly Ser Asp Gly Leu
   355 360 365
   Arg Gly Leu Thr Ala Gly Gln Lys Leu Val Gly Ala Leu Phe Met Ala
   370 375 380
   Val Asn Ser Arg His Ser Gly Glu Met Val Leu Asp Leu Ser Thr Val
   385 390 395 400
   Ser Ser Ala Val Val Val Leu Tyr Val Val Met Met Tyr Leu Pro Pro
   405 410 415
   Tyr Thr Thr Phe Val Pro Val Gln Asp Lys His Gln Gln Thr Gly Ala
   420 425 430
   Gln Ser Gly Gln Glu Gly Ser Ser Ser Ser Ser Ile Trp Gln Lys Leu
   435 440 445
   Leu Met Ser Pro Leu Ser Cys Leu Ala Ile Phe Ile Val Val Ile Cys
   450 455 460
   Ile Thr Glu Arg Arg Gln Ile Ala Asp Asp Pro Ile Asn Tyr Ser Val
   465 470 475 480
   Leu Asn Ile Val Val Glu Val Ile Ser Ala Tyr Gly Asn Val Gly Phe
   485 490 495
   Ser Thr Gly Tyr Ser Cys Ala Arg Gln Val Arg Pro Asp Gly Ser Cys
   500 505 510
   Arg Asp Leu Trp Val Gly Phe Ser Gly Lys Trp Ser Lys Gln Gly Lys
   515 520 525
   Leu Thr Leu Met Ala Val Met Phe Tyr Gly Arg Leu Lys Lys Phe Ser
   530 535 540
   Leu His Gly Gly Gln Ala Trp Lys Ile Glu
   545 550
<210> 33
   <211> 1530
   <212> DNA
   <213> Oryza sativa
<400> 33
   atgcctattc ggctgcatat ctttgtcagt tctgcaaggc atgccatcaa ctcgtcggca      60
   ttgatttgtc ggttcatcgc attccatctt agcccgcttt tgattcacct gtcctatttt     120
   cttatcattg atgtacttgg ttttgttgcc ttggtggtgc tcaggccaag caaccacaag     180
   tacaatcctc gctatatcga tatgtttttc ctatcgacat ctgcagttac agtcacagga     240
   ttagccacca cacaaatgga ggatctttct agctctcaga tagctgtcct gactctcttg     300
   atgttcttag gaagtgagat gttcctttcc tttcttggcc ttgtccttga gtcgagcaag     360
   caaaacaagc atgatcctga aaaccgtaga gtaagttcag ttaccgtgtg tgagcagtca     420
   catctagaag aggcaattcc acaaactcca tccatgaact ccactgatat caagaggagc     480
   tgccacaaat acttagtgtt tgtggtgttg gcatacatga ttattattct tgtcactggt     540
   tctctattgg tgttcatgta catagctcat gtttcaagtg ctagagatgt gctaacaagg     600
   aaaagcatca acaaagctct cttctcgata tcggtcacag tctcctcatt tacaaatgga     660
   gggttattgc cgacaaatga gagtatggct gtattctcct caaacaatgg cctcctgttg     720
   ctactcatcg gccagattct tgcaggcagc acactgctcc ctatgtttct gaggttggtg     780
   atatgggcat tgagaggact aagattagca aaagctgaag agccagactt catgatgaac     840
   aacagcagct cagtaggttt cagtcacctg ctacctaact tgcagacaat atttcttgca     900
   gctgtggagg ttgcttttgt aggcatgaca gtcatcctct tctgctgctt gaactgggat     960
   tctgcagtgt ttgcagggct aacctccctc cagaagataa ccaatgcatt gttcatggca    1020
   gtgagtgcaa ggcaggcagg agaaaattcc attgattgtt cccttgttgc gccagcggct    1080
   ttagtactat tcatggtcat gatgtacact ccatccttga caaagttgtt ctcagcatgt    1140
   caagatcaca aacaaatcgg cccggaaagc gacgatagaa caagcaaggg gaaaccattc    1200
   ctgaagacga tggcattttc accactagcc ttcaacacca cagtgataat gctggtctgc    1260
   atcactgaaa ggagatcgat ctccactgac cctctcaatt tctccacatt taacatcatc    1320
   tttgaggtga taagtgccta cgggaacatt gggttgtcca ctggctacag ctgctcgaga    1380
   cagctacagc accaggatgg gattgcttgc catgagaagc catacagctt ctcggggtgg    1440
   tggagtgaac cagggaagct gattcttgtt cttgcgatgc tctacgggag gctcaactca    1500
   aaggattcca caagcgcacg aactaggtga                                     1530
<210> 34
   <211> 509
   <212> PRT
   <213> Oryza sativa
<400> 34
   Met Pro Ile Arg Leu His Ile Phe Val Ser Ser Ala Arg His Ala Ile
   1               5                   10                  15
   Asn Ser Ser Ala Leu Ile Cys Arg Phe Ile Ala Phe His Leu Ser Pro
               20                  25                  30
   Leu Leu Ile His Leu Ser Tyr Phe Leu Ile Ile Asp Val Leu Gly Phe
           35                  40                  45
   Val Ala Leu Val Val Leu Arg Pro Ser Asn His Lys Tyr Asn Pro Arg
       50                  55                  60
   Tyr Ile Asp Met Phe Phe Leu Ser Thr Ser Ala Val Thr Val Thr Gly
   65                  70                  75                  80
   Leu Ala Thr Thr Gln Met Glu Asp Leu Ser Ser Ser Gln Ile Ala Val
                   85                  90                  95
   Leu Thr Leu Leu Met Phe Leu Gly Ser Glu Met Phe Leu Ser Phe Leu
               100                 105                 110
   Gly Leu Val Leu Glu Ser Ser Lys Gln Asn Lys His Asp Pro Glu Asn
           115                 120                 125
   Arg Arg Val Ser Ser Val Thr Val Cys Glu Gln Ser His Leu Glu Glu
       130                 135                 140
   Ala Ile Pro Gln Thr Pro Ser Met Asn Ser Thr Asp Ile Lys Arg Ser
   145                 150                 155                 160
   Cys His Lys Tyr Leu Val Phe Val Val Leu Ala Tyr Met Ile Ile Ile
                   165                 170                 175
   Leu Val Thr Gly Ser Leu Leu Val Phe Met Tyr Ile Ala His Val Ser
               180                 185                 190
   Ser Ala Arg Asp Val Leu Thr Arg Lys Ser Ile Asn Lys Ala Leu Phe
           195                 200                 205
   Ser Ile Ser Val Thr Val Ser Ser Phe Thr Asn Gly Gly Leu Leu Pro
       210                 215                 220
   Thr Asn Glu Ser Met Ala Val Phe Ser Ser Asn Asn Gly Leu Leu Leu
   225                 230                 235                 240
   Leu Leu Ile Gly Gln Ile Leu Ala Gly Ser Thr Leu Leu Pro Met Phe
                   245                 250                 255
   Leu Arg Leu Val Ile Trp Ala Leu Arg Gly Leu Arg Leu Ala Lys Ala
               260                 265                 270
   Glu Glu Pro Asp Phe Met Met Asn Asn Ser Ser Ser Val Gly Phe Ser
           275                 280                 285
   His Leu Leu Pro Asn Leu Gln Thr Ile Phe Leu Ala Ala Val Glu Val
       290                 295                 300
   Ala Phe Val Gly Met Thr Val Ile Leu Phe Cys Cys Leu Asn Trp Asp
   305                 310                 315                 320
   Ser Ala Val Phe Ala Gly Leu Thr Ser Leu Gln Lys Ile Thr Asn Ala
                   325                 330                 335
   Leu Phe Met Ala Val Ser Ala Arg Gln Ala Gly Glu Asn Ser Ile Asp
               340                 345                 350
   Cys Ser Leu Val Ala Pro Ala Ala Leu Val Leu Phe Met Val Met Met
           355                 360                 365
   Tyr Thr Pro Ser Leu Thr Lys Leu Phe Ser Ala Cys Gln Asp His Lys
       370                 375                 380
   Gln Ile Gly Pro Glu Ser Asp Asp Arg Thr Ser Lys Gly Lys Pro Phe
   385                 390                 395                 400
   Leu Lys Thr Met Ala Phe Ser Pro Leu Ala Phe Asn Thr Thr Val Ile
                   405                 410                 415
   Met Leu Val Cys Ile Thr Glu Arg Arg Ser Ile Ser Thr Asp Pro Leu
               420                 425                 430
   Asn Phe Ser Thr Phe Asn Ile Ile Phe Glu Val Ile Ser Ala Tyr Gly
   435 440 445
   Asn Ile Gly Leu Ser Thr Gly Tyr Ser Cys Ser Arg Gln Leu Gln His
   450 455 460
   Gln Asp Gly Ile Ala Cys His Glu Lys Pro Tyr Ser Phe Ser Gly Trp
   465 470 475 480
   Trp Ser Glu Pro Gly Lys Leu Ile Leu Val Leu Ala Met Leu Tyr Gly
   485 490 495
   Arg Leu Asn Ser Lys Asp Ser Thr Ser Ala Arg Thr Arg
   500 505
<210> 35
   <211> 1602
   <212> DNA
   <213> Triticum aestivum
<400> 35
   atgggccggg tgaaaagatt ttaccaggat ttcatccata tcaagctgca tagcttctgc 60
   cgtatcagtg gatatgttgt cgattcaata gcttttgtct atagatttgt tgcattgcat 120
   gttcacccct tctggatcca actgtcctac ttccttgcca ttgctatact tggttcagtc 180
   ctcttgatgt cgctgaaacc aagcaaccct gacttcagcc ctccttacat tgacatgtta 240
   ttcttgtcaa cttctgctct aacagtttct ggcctcagca ccatcacgat ggaggatctc 300
   tcaagctctc aaattgtggt tttgacattg ctcatgctta taggagggga gatctttgtt 360
   tcactcttag ggctcatgct tagagtgaac catcaagaca tgcaagatct tccaagcgtg 420
   aagatcagct cggttcctgt cgagcttgaa gagctagact tgcccaacag catggcacta 480
   tgtgatgagt cgcagcttga agaagcagct catgcaattc cacccaagaa atgtacagag 540
   ttgaagagga gtaggtctgt caagtgctta ggatatgtgg tctttgggta ctttgccatg 600
   atccatgtct tgggctttct gctggttttt ctgtatataa ctcatgtgcc aactgcaagt 660
   gccccactga acaagaaagg gatcaacatc gtgctcttct cactatcagt caccgttgcc 720
   tcctgtgcga atgcaggact cgtgcccaca aatgagaaca tggtcatctt ctcaaagaat 780
   tcaggcctct tgttgctgct gagtggccag atgctcgcag gcaatacatt gttccctctc 840
   ttcctgaggc tactggtgtg gttcctgggg aggatcacaa aggtgaagga gctgaggctc 900
   atgatcaata accccgagga agtgcgtttt gctaatttgc ttgctaggtt gccaactgtg 960
   tttctctcct caacggtcgt tggccttgta gcagctgggg tcacgatgtt ctgcgctgtt 1020
   gattggaatt cttcagtctt tgatgggctc agctcttatc agaagactgt caatgcattc 1080
   ttcatggtgg tgaatgcgag gcactcaggg gagaattcca tcgactgctc gctcatgtcc 1140
   cctgccatta tagtactatt catcgtcatg atgtatttgc catcatcagc aacatttgca 1200
   ccacccagtg gagatactaa aaccaccaat gagaacacga aagggaaagt caagagaggg 1260
   tcgttggtgc agaatttggc attctcaccg ctcgggtgta acatcatctt tgtgatggtt 1320
   gcctgcatca cggaaaggag aaggctcaga aacgatccac tcaacttctc caccttgaac 1380
   atgatatttg aggtcatcag cgcatatggc aatgcagggt tatccactgg ttacagttgt 1440
   tctagactgc atcagctgca cccagagatc atctgccagg acaaaccata cagcttttct 1500
   ggatggtgga gtgacggagg aaagtttgtg ctaatattgg tcatgctcta tggaaggctt 1560
   aaggctttca cactggccac gggtaaatcc tggaaagtat ga 1602
<210> 36
   <211> 533
   <212> PRT
   <213> Triticum aestivum
<400> 36
   Met Gly Arg Val Lys Arg Phe Tyr Gln Asp Phe Ile His Ile Lys Leu
   1               5                   10                  15
   His Ser Phe Cys Arg Ile Ser Gly Tyr Val Val Asp Ser Ile Ala Phe
               20                  25                  30
   Val Tyr Arg Phe Val Ala Leu His Val His Pro Phe Trp Ile Gln Leu
   35 40 45
   Ser Tyr Phe Leu Ala Ile Ala Ile Leu Gly Ser Val Leu Leu Met Ser
   50 55 60
   Leu Lys Pro Ser Asn Pro Asp Phe Ser Pro Pro Tyr Ile Asp Met Leu
   65 70 75 80
   Phe Leu Ser Thr Ser Ala Leu Thr Val Ser Gly Leu Ser Thr Ile Thr
   85 90 95
   Met Glu Asp Leu Ser Ser Ser Gln Ile Val Val Leu Thr Leu Leu Met
   100 105 110
   Leu Ile Gly Gly Glu Ile Phe Val Ser Leu Leu Gly Leu Met Leu Arg
   115 120 125
   Val Asn His Gln Asp Met Gln Asp Leu Pro Ser Val Lys Ile Ser Ser
   130 135 140
   Val Pro Val Glu Leu Glu Glu Leu Asp Leu Pro Asn Ser Met Ala Leu
   145 150 155 160
   Cys Asp Glu Ser Gln Leu Glu Glu Ala Ala His Ala Ile Pro Pro Lys
   165 170 175
   Lys Cys Thr Glu Leu Lys Arg Ser Arg Ser Val Lys Cys Leu Gly Tyr
   180 185 190
   Val Val Phe Gly Tyr Phe Ala Met Ile His Val Leu Gly Phe Leu Leu
   195 200 205
   Val Phe Leu Tyr Ile Thr His Val Pro Thr Ala Ser Ala Pro Leu Asn
   210 215 220
   Lys Lys Gly Ile Asn Ile Val Leu Phe Ser Leu Ser Val Thr Val Ala
   225 230 235 240
   Ser Cys Ala Asn Ala Gly Leu Val Pro Thr Asn Glu Asn Met Val Ile
   245 250 255
   Phe Ser Lys Asn Ser Gly Leu Leu Leu Leu Leu Ser Gly Gln Met Leu
   260 265 270
   Ala Gly Asn Thr Leu Phe Pro Leu Phe Leu Arg Leu Leu Val Trp Phe
   275 280 285
   Leu Gly Arg Ile Thr Lys Val Lys Glu Leu Arg Leu Met Ile Asn Asn
   290 295 300
   Pro Glu Glu Val Arg Phe Ala Asn Leu Leu Ala Arg Leu Pro Thr Val
   305 310 315 320
   Phe Leu Ser Ser Thr Val Val Gly Leu Val Ala Ala Gly Val Thr Met
   325 330 335
   Phe Cys Ala Val Asp Trp Asn Ser Ser Val Phe Asp Gly Leu Ser Ser
   340 345 350
   Tyr Gln Lys Thr Val Asn Ala Phe Phe Met Val Val Asn Ala Arg His
   355 360 365
   Ser Gly Glu Asn Ser Ile Asp Cys Ser Leu Met Ser Pro Ala Ile Ile
   370 375 380
   Val Leu Phe Ile Val Met Met Tyr Leu Pro Ser Ser Ala Thr Phe Ala
   385 390 395 400
   Pro Pro Ser Gly Asp Thr Lys Thr Thr Asn Glu Asn Thr Lys Gly Lys
   405 410 415
   Val Lys Arg Gly Ser Leu Val Gln Asn Leu Ala Phe Ser Pro Leu Gly
   420 425 430
   Cys Asn Ile Ile Phe Val Met Val Ala Cys Ile Thr Glu Arg Arg Arg
   435 440 445
   Leu Arg Asn Asp Pro Leu Asn Phe Ser Thr Leu Asn Met Ile Phe Glu
   450 455 460
   Val Ile Ser Ala Tyr Gly Asn Ala Gly Leu Ser Thr Gly Tyr Ser Cys
   465 470 475 480
   Ser Arg Leu His Gln Leu His Pro Glu Ile Ile Cys Gln Asp Lys Pro
   485 490 495
   Tyr Ser Phe Ser Gly Trp Trp Ser Asp Gly Gly Lys Phe Val Leu Ile
   500 505 510
   Leu Val Met Leu Tyr Gly Arg Leu Lys Ala Phe Thr Leu Ala Thr Gly
   515 520 525
   Lys Ser Trp Lys Val
   530
<210> 37
   <211> 1458
   <212> DNA
   <213> Suaeda maritima subsp. salsa
<400> 37
   atgttgagtt tcaaatttat agtagaaaaa tgcaaacatt tttatacttc tctttatcta     60
   cttcttgtct atgtttttac atccttatat tggttaattt caaaaatcta tgattttatt    120
   atggtttatg tttgccactt gattattgag ttatgttact tcattcttgt atcttctttt    180
   ggattcttgt ttttaaaaac cctaattcca aaatcatcta ataatagtaa taacaataat    240
   aattcaataa atggtttaga tttgtttttc acctcggttt ccgccacaac cgtctcaagc    300
   atgtcaaccc tagagatgga ggtattctca aattcccaac tcattgtttt aaccattttg    360
   atgttcatag gaggtgaggt ctttacctcc atggtaggtc tccatttttc ggcctcgaaa    420
   cttgtgtaca ctcccctgca agcaaggagt agagtaaact cagtggctag cttaccactt    480
   cctcctgaaa gtattgagtt aggaatcatt attccatcat caacaacaac aacacaagaa    540
   attagggttt catcaaccat tgaaaaaaca aaatcagaaa tagatttcct aataaaatca    600
   aaatcaatta gggttttagg ctttgtggtt ttgtcttact taatcatagt tcatttccta    660
   ggaatttcaa tggtattaac atacattaat acaatcccaa atgctaaaaa tgttttagac    720
   aaaaaaggtc ttaaaacatt cactttttca attttttcag ttgtttcaac ttttgcaagt    780
   tgtggtttca cccctacaaa tgaaaacatg caagttttta gcaaaaactc tggcctcttg    840
   ttgatgctaa tccctcaaat cctccttggg aacacattgt ttccttcgtt tcttcgattt    900
   tcgatatgga tgctaggaaa atttgtcaaa aaagacgaaa ccaagtatct aatgagaaac    960
   tcaaaggaaa ttgagtacca tcatttgctc tctagcaaac actctaggtt tttaattgtg   1020
   acggttttag ggttcatttt ggtgcaattt ataatgtttt gttcaatgga atggaatttc   1080
   gatggtctga ataatgatca taatatttat caaaaattag tgggaatatt atttcaatgt   1140
   gttaattcaa gacatacagg tgaaagcatt gttgatcttt cctccattaa ttcagctatg   1200
   ttggtgattt tcatcgttat gatgtacctt ccaccatata cttcctttct gccaataaag   1260
   gatgaagaaa aagaatatcc aaacatggta ttatttcaca aaggagaaaa gaaacggaag   1320
   aagattttga agaatttcct attttcacag cttggccata ttgccatttc atcattatta   1380
   tttgcatcac agagaagcaa aagattaaag atgatcccct caatttcaac gttttcaaca   1440
   ttgcctttga ggttctaa                                                 1458
<210> 38
   <211> 485
   <212> PRT
   <213> Suaeda maritima subsp. salsa
<400> 38
   Met Leu Ser Phe Lys Phe Ile Val Glu Lys Cys Lys His Phe Tyr Thr
   1               5                   10                  15
   Ser Leu Tyr Leu Leu Leu Val Tyr Val Phe Thr Ser Leu Tyr Trp Leu
               20                  25                  30
   Ile Ser Lys Ile Tyr Asp Phe Ile Met Val Tyr Val Cys His Leu Ile
           35                  40                  45
   Ile Glu Leu Cys Tyr Phe Ile Leu Val Ser Ser Phe Gly Phe Leu Phe
       50                  55                  60
   Leu Lys Thr Leu Ile Pro Lys Ser Ser Asn Asn Ser Asn Asn Asn Asn
   65                  70                  75                  80
   Asn Ser Ile Asn Gly Leu Asp Leu Phe Phe Thr Ser Val Ser Ala Thr
                   85                  90                  95
   Thr Val Ser Ser Met Ser Thr Leu Glu Met Glu Val Phe Ser Asn Ser
               100                 105                 110
   Gln Leu Ile Val Leu Thr Ile Leu Met Phe Ile Gly Gly Glu Val Phe
           115                 120                 125
   Thr Ser Met Val Gly Leu His Phe Ser Ala Ser Lys Leu Val Tyr Thr
       130                 135                 140
   Pro Leu Gln Ala Arg Ser Arg Val Asn Ser Val Ala Ser Leu Pro Leu
   145                 150                 155                 160
   Pro Pro Glu Ser Ile Glu Leu Gly Ile Ile Ile Pro Ser Ser Thr Thr
                   165                 170                 175
   Thr Thr Gln Glu Ile Arg Val Ser Ser Thr Ile Glu Lys Thr Lys Ser
               180                 185                 190
   Glu Ile Asp Phe Leu Ile Lys Ser Lys Ser Ile Arg Val Leu Gly Phe
           195                 200                 205
   Val Val Leu Ser Tyr Leu Ile Ile Val His Phe Leu Gly Ile Ser Met
       210                 215                 220
   Val Leu Thr Tyr Ile Asn Thr Ile Pro Asn Ala Lys Asn Val Leu Asp
   225 230 235 240
   Lys Lys Gly Leu Lys Thr Phe Thr Phe Ser Ile Phe Ser Val Val Ser
   245 250 255
   Thr Phe Ala Ser Cys Gly Phe Thr Pro Thr Asn Glu Asn Met Gln Val
   260 265 270
   Phe Ser Lys Asn Ser Gly Leu Leu Leu Met Leu Ile Pro Gln Ile Leu
   275 280 285
   Leu Gly Asn Thr Leu Phe Pro Ser Phe Leu Arg Phe Ser Ile Trp Met
   290 295 300
   Leu Gly Lys Phe Val Lys Lys Asp Glu Thr Lys Tyr Leu Met Arg Asn
   305 310 315 320
   Ser Lys Glu Ile Glu Tyr His His Leu Leu Ser Ser Lys His Ser Arg
   325 330 335
   Phe Leu Ile Val Thr Val Leu Gly Phe Ile Leu Val Gln Phe Ile Met
   340 345 350
   Phe Cys Ser Met Glu Trp Asn Phe Asp Gly Leu Asn Asn Asp His Asn
   355 360 365
   Ile Tyr Gln Lys Leu Val Gly Ile Leu Phe Gln Cys Val Asn Ser Arg
   370 375 380
   His Thr Gly Glu Ser Ile Val Asp Leu Ser Ser Ile Asn Ser Ala Met
   385 390 395 400
   Leu Val Ile Phe Ile Val Met Met Tyr Leu Pro Pro Tyr Thr Ser Phe
   405 410 415
   Leu Pro Ile Lys Asp Glu Glu Lys Glu Tyr Pro Asn Met Val Leu Phe
   420 425 430
   His Lys Gly Glu Lys Lys Arg Lys Lys Ile Leu Lys Asn Phe Leu Phe
   435 440 445
   Ser Gln Leu Gly His Ile Ala Ile Ser Ser Leu Leu Phe Ala Ser Gln
   450 455 460
   Arg Ser Lys Arg Leu Lys Met Ile Pro Ser Ile Ser Thr Phe Ser Thr
   465 470 475 480
   Leu Pro Leu Arg Phe
   485
<210> 39
   <211> 3708
   <212> DNA
   <213> Saccharomyces cerevisiae
<400> 39
   atgcatttta gaagaacgat gagtagagtg cccacattgg catctcttga aatacgatat     60
   aaaaaatctt tcggccataa atttcgtgat tttattgctc tatgtggtca ctattttgct    120
   ccagttaaaa aatatatctt ccccagtttt atcgcggttc actacttcta cacgatatcc    180
   ctgacattaa taacttcaat cctgctatat cccattaaga ataccagata cattgataca    240
   ttgtttttag cagcgggcgc agttacacaa ggtggcttaa atactgtgga tatcaacaat    300
   ctaagcttat accaacaaat tgttctgtat atcgtatgct gcatatcaac accaattgca    360
   gttcatagtt gcttggcatt tgtacggctt tactggtttg agcgctactt cgatggtatt    420
   agagactctt ctagacgaaa ttttaagatg agaagaacga aaacaatctt agaaagggaa    480
   ctaacagcaa gaaccatgac caagaataga acaggtaccc aaagaacgtc ttatcctagg    540
   aaacaagcta aaacagatga tttccaagaa aaattgttca gcggagaaat ggttaataga    600
   gatgagcagg actcagttca cagcgaccag aattctcatg acattagtag ggacagcagc    660
   aataataata cgaatcacaa tggtagcagt ggcagtttag atgatttcgt taaggaagac    720
   gaaacggatg acaatggaga atatcaggag aacaactcct actcgacggt aggtagttcg    780
   tctaacacag ttgcagacga aagtttaaat cagaagccca agccaagcag tcttcggttt    840
   gatgagccac acagcaaaca aagacccgca agagttccct cagagaaatt tgcaaaaaga    900
   aggggttcaa gagatattag cccagccgat atgtatcgat ccattatgat gctacaaggt    960
   aagcatgaag caactgctga agatgaaggt ccccctttag tcatcgggtc ccctgcggat   1020
   ggcacaagat ataaaagtaa tgtcaataag ctaaagaagg ccaccggcat aaatggtaac   1080
   aaaatcaaga ttcgagataa gggaaatgaa agtaacactg atcaaaattc cgtgtcaagt   1140
   gaagcaaaca gtacggcgag cgtttcggac gaaagctcgt tacacacaaa ttttggtaac   1200
   aaagtacctt cattaagaac aaatactcat agatcaaatt cgggcccgat agccattact   1260
   gataacgcag aaacagacaa aaagcatggg ccatcaattc aattcgatat aactaaacct   1320
   cctagaaaaa tttcaaaaag agtttcaacc ttcgatgatt tgaacccaaa atcttccgtt   1380
   ctttatcgaa aaaaagcatc gaagaagtac ctcatgaaac attttcctaa agcgcggcga   1440
   atacggcaac aaattaagag aaggctttct actggttcaa ttgagaaaaa cagcagtaac   1500
   aatgtttcag atagaaaacc tattactgat atggatgatg atgatgatga cgatgacaac   1560
   gacggcgata acaacgaaga atactttgct gacaacgaaa gcggcgatga agatgaacga   1620
   gtacagcagt ctgaaccaca ttctgattca gaactcaaat cgcaccaaca acagcaagaa   1680
   aaacaccaac tgcagcagaa cctgcaccgc atgtataaaa ccaaatcatt tgatgataat   1740
   cgttcaagag cagttcctat ggaacgttcc aggaccatcg atatggcaga ggctaaggat   1800
   ctaaatgagc tcgcaaggac gcctgatttt caaaaaatgg tctatcaaaa ttggaaagcc   1860
   catcatagaa aaaaaccgaa ctttaggaag aggggatgga ataacaagat atttgaacat   1920
   ggtccctatg catctgacag cgatcgcaat tatcctgata atagtaatac tggaaacagt   1980
   attcttcatt acgcagagtc tattttacat catgatggct ctcataaaaa tggaagcgaa   2040
   gaagcctctt ccgactctaa tgagaatatc tattccacga atggaggaag cgaccacaat   2100
   ggtcttaaca actatcctac ttacaacgac gatgaagaag gctattatgg tttacatttc   2160
   gataccgatt atgacctaga tcctcgtcat gatttatcta aaggcagtgg taaaacgtat   2220
   ctatcatggc aaccaactat tggacgtaac tcaaacttcc ttggattaac aagagcccag   2280
   aaagatgaat taggtggtgt cgagtacaga gcaatcaaac ttttatgcac catattggtt   2340
   gtctactacg ttggatggca tattgttgct tttgttatgt tagtaccttg gattattttg   2400
   aaaaagcatt atagtgaagt tgttagagat gatggtgttt cacctacatg gtggggattt   2460
   tggacagcaa tgagtgcatt taatgattta ggtttgacat taactccaaa ttcaatgatg   2520
   tcgtttaaca aagctgtata cccattgatc gttatgattt ggtttatcat tatcggaaat   2580
   acagggtttc ccatccttct tagatgcatc atttggataa tgtttaaaat ttctcctgat   2640
   ttatcacaga tgagagaaag tttaggtttt ctcttagacc atccacgtcg ttgtttcacc   2700
   ttgctatttc ctaaggcagc tacatggtgg ctacttttaa cgcttgcagg attgaatata   2760
   actgattgga ttttatttat tattctagat tttggctcaa cagttgtgaa atcattatcg   2820
   aaaggctata gagtccttgt cggcctgttt caatctgtta gcacaagaac tgctggattc   2880
   agcgttgtcg atttaagtca actgcatcct tctatccaag tctcctatat gctaatgatg   2940
   tatgtctccg tattaccatt ggccatctct attcgacgga caaatgttta cgaggagcaa   3000
   tctttaggac tatatggaga tatgggggga gaaccagaag atacggatac tgaagacgat   3060
   ggtaacgatg aagatgacga cgaggaaaac gagagtcacg aaggtcaaag tagtcaaaga   3120
   agtagttcga acaacaacaa caataacaac aggaaaaaga aaaagaaaaa gaaaactgaa   3180
   aatccaaatg aaatatctac aaaatccttt atcggtgccc atttaaggaa acagctttca   3240
   tttgacttgt ggtttctatt tttagggtta tttatcattt gcatttgtga aggggacaag   3300
   ataaaggacg tacaagaacc aaactttaat atatttgcaa ttctttttga aattgttagc   3360
   gcttacggta cagttgggct atcgctaggt tatccggaca ccaaccaatc gttttcaaga   3420
   cagtttacta cattatctaa gttggtgatc atagctatgc tgatcagagg caagaataga   3480
   ggtctaccat actcactgga tcgtgcaatt atcttgccta gtgatagact tgaacatatt   3540
   gaccaccttg agggcatgaa attgaagaga caggctagaa ccaatacaga agacccaatg   3600
   acggaacatt tcaagagaag tttcactgat gtgaaacatc gttggggagc tcttaagcgt   3660
   aagaccacac attcccgaaa tcctaaaagg agcagcacaa cgctctaa 3708
<210> 40
   <211> 1235
   <212> PRT
   <213> Saccharomyces cerevisiae
<400> 40
   Met His Phe Arg Arg Thr Met Ser Arg Val Pro Thr Leu Ala Ser Leu
   1               5                   10                  15
   Glu Ile Arg Tyr Lys Lys Ser Phe Gly His Lys Phe Arg Asp Phe Ile
               20                  25                  30
   Ala Leu Cys Gly His Tyr Phe Ala Pro Val Lys Lys Tyr Ile Phe Pro
           35                  40                  45
   Ser Phe Ile Ala Val His Tyr Phe Tyr Thr Ile Ser Leu Thr Leu Ile
       50                  55                  60
   Thr Ser Ile Leu Leu Tyr Pro Ile Lys Asn Thr Arg Tyr Ile Asp Thr
   65                  70                  75                  80
   Leu Phe Leu Ala Ala Gly Ala Val Thr Gln Gly Gly Leu Asn Thr Val
                   85                  90                  95
   Asp Ile Asn Asn Leu Ser Leu Tyr Gln Gln Ile Val Leu Tyr Ile Val
               100                 105                 110
   Cys Cys Ile Ser Thr Pro Ile Ala Val His Ser Cys Leu Ala Phe Val
           115                 120                 125
   Arg Leu Tyr Trp Phe Glu Arg Tyr Phe Asp Gly Ile Arg Asp Ser Ser
       130                 135                 140
   Arg Arg Asn Phe Lys Met Arg Arg Thr Lys Thr Ile Leu Glu Arg Glu
   145                 150                 155                 160
   Leu Thr Ala Arg Thr Met Thr Lys Asn Arg Thr Gly Thr Gln Arg Thr
                   165                 170                 175
   Ser Tyr Pro Arg Lys Gln Ala Lys Thr Asp Asp Phe Gln Glu Lys Leu
               180                 185                 190
   Phe Ser Gly Glu Met Val Asn Arg Asp Glu Gln Asp Ser Val His Ser
           195                 200                 205
   Asp Gln Asn Ser His Asp Ile Ser Arg Asp Ser Ser Asn Asn Asn Thr
       210                 215                 220
   Asn His Asn Gly Ser Ser Gly Ser Leu Asp Asp Phe Val Lys Glu Asp
   225                 230                 235                 240
   Glu Thr Asp Asp Asn Gly Glu Tyr Gln Glu Asn Asn Ser Tyr Ser Thr
                   245                 250                 255
   Val Gly Ser Ser Ser Asn Thr Val Ala Asp Glu Ser Leu Asn Gln Lys
               260                 265                 270
   Pro Lys Pro Ser Ser Leu Arg Phe Asp Glu Pro His Ser Lys Gln Arg
   275 280 285
   Pro Ala Arg Val Pro Ser Glu Lys Phe Ala Lys Arg Arg Gly Ser Arg
   290 295 300
   Asp Ile Ser Pro Ala Asp Met Tyr Arg Ser Ile Met Met Leu Gln Gly
   305 310 315 320
   Lys His Glu Ala Thr Ala Glu Asp Glu Gly Pro Pro Leu Val Ile Gly
   325 330 335
   Ser Pro Ala Asp Gly Thr Arg Tyr Lys Ser Asn Val Asn Lys Leu Lys
   340 345 350
   Lys Ala Thr Gly Ile Asn Gly Asn Lys Ile Lys Ile Arg Asp Lys Gly
   355 360 365
   Asn Glu Ser Asn Thr Asp Gln Asn Ser Val Ser Ser Glu Ala Asn Ser
   370 375 380
   Thr Ala Ser Val Ser Asp Glu Ser Ser Leu His Thr Asn Phe Gly Asn
   385 390 395 400
   Lys Val Pro Ser Leu Arg Thr Asn Thr His Arg Ser Asn Ser Gly Pro
   405 410 415
   Ile Ala Ile Thr Asp Asn Ala Glu Thr Asp Lys Lys His Gly Pro Ser
   420 425 430
   Ile Gln Phe Asp Ile Thr Lys Pro Pro Arg Lys Ile Ser Lys Arg Val
   435 440 445
   Ser Thr Phe Asp Asp Leu Asn Pro Lys Ser Ser Val Leu Tyr Arg Lys
   450 455 460
   Lys Ala Ser Lys Lys Tyr Leu Met Lys His Phe Pro Lys Ala Arg Arg
   465 470 475 480
   Ile Arg Gln Gln Ile Lys Arg Arg Leu Ser Thr Gly Ser Ile Glu Lys
   485 490 495
   Asn Ser Ser Asn Asn Val Ser Asp Arg Lys Pro Ile Thr Asp Met Asp
   500 505 510
   Asp Asp Asp Asp Asp Asp Asp Asn Asp Gly Asp Asn Asn Glu Glu Tyr
   515 520 525
   Phe Ala Asp Asn Glu Ser Gly Asp Glu Asp Glu Arg Val Gln Gln Ser
   530 535 540
   Glu Pro His Ser Asp Ser Glu Leu Lys Ser His Gln Gln Gln Gln Glu
   545 550 555 560
   Lys His Gln Leu Gln Gln Asn Leu His Arg Met Tyr Lys Thr Lys Ser
   565 570 575
   Phe Asp Asp Asn Arg Ser Arg Ala Val Pro Met Glu Arg Ser Arg Thr
   580 585 590
   Ile Asp Met Ala Glu Ala Lys Asp Leu Asn Glu Leu Ala Arg Thr Pro
           595                 600                 605
   Asp Phe Gln Lys Met Val Tyr Gln Asn Trp Lys Ala His His Arg Lys
       610                 615                 620
   Lys Pro Asn Phe Arg Lys Arg Gly Trp Asn Asn Lys Ile Phe Glu His
   625                 630                 635                 640
   Gly Pro Tyr Ala Ser Asp Ser Asp Arg Asn Tyr Pro Asp Asn Ser Asn
                   645                 650                 655
   Thr Gly Asn Ser Ile Leu His Tyr Ala Glu Ser Ile Leu His His Asp
               660                 665                 670
   Gly Ser His Lys Asn Gly Ser Glu Glu Ala Ser Ser Asp Ser Asn Glu
           675                 680                 685
   Asn Ile Tyr Ser Thr Asn Gly Gly Ser Asp His Asn Gly Leu Asn Asn
       690                 695                 700
   Tyr Pro Thr Tyr Asn Asp Asp Glu Glu Gly Tyr Tyr Gly Leu His Phe
   705                 710                 715                 720
   Asp Thr Asp Tyr Asp Leu Asp Pro Arg His Asp Leu Ser Lys Gly Ser
                   725                 730                 735
   Gly Lys Thr Tyr Leu Ser Trp Gln Pro Thr Ile Gly Arg Asn Ser Asn
               740                 745                 750
   Phe Leu Gly Leu Thr Arg Ala Gln Lys Asp Glu Leu Gly Gly Val Glu
           755                 760                 765
   Tyr Arg Ala Ile Lys Leu Leu Cys Thr Ile Leu Val Val Tyr Tyr Val
       770                 775                 780
   Gly Trp His Ile Val Ala Phe Val Met Leu Val Pro Trp Ile Ile Leu
   785                 790                 795                 800
   Lys Lys His Tyr Ser Glu Val Val Arg Asp Asp Gly Val Ser Pro Thr
                   805                 810                 815
   Trp Trp Gly Phe Trp Thr Ala Met Ser Ala Phe Asn Asp Leu Gly Leu
               820                 825                 830
   Thr Leu Thr Pro Asn Ser Met Met Ser Phe Asn Lys Ala Val Tyr Pro
           835                 840                 845
   Leu Ile Val Met Ile Trp Phe Ile Ile Ile Gly Asn Thr Gly Phe Pro
       850                 855                 860
   Ile Leu Leu Arg Cys Ile Ile Trp Ile Met Phe Lys Ile Ser Pro Asp
   865                 870                 875                 880
   Leu Ser Gln Met Arg Glu Ser Leu Gly Phe Leu Leu Asp His Pro Arg
                   885                 890                 895
   Arg Cys Phe Thr Leu Leu Phe Pro Lys Ala Ala Thr Trp Trp Leu Leu
   900 905 910
   Leu Thr Leu Ala Gly Leu Asn Ile Thr Asp Trp Ile Leu Phe Ile Ile
   915 920 925
   Leu Asp Phe Gly Ser Thr Val Val Lys Ser Leu Ser Lys Gly Tyr Arg
   930 935 940
   Val Leu Val Gly Leu Phe Gln Ser Val Ser Thr Arg Thr Ala Gly Phe
   945 950 955 960
   Ser Val Val Asp Leu Ser Gln Leu His Pro Ser Ile Gln Val Ser Tyr
   965 970 975
   Met Leu Met Met Tyr Val Ser Val Leu Pro Leu Ala Ile Ser Ile Arg
   980 985 990
   Arg Thr Asn Val Tyr Glu Glu Gln Ser Leu Gly Leu Tyr Gly Asp Met
   995 1000 1005
   Gly Gly Glu Pro Glu Asp Thr Asp Thr Glu Asp Asp Gly Asn Asp
   1010 1015 1020
   Glu Asp Asp Asp Glu Glu Asn Glu Ser His Glu Gly Gln Ser Ser
   1025 1030 1035
   Gln Arg Ser Ser Ser Asn Asn Asn Asn Asn Asn Asn Arg Lys Lys
   1040 1045 1050
   Lys Lys Lys Lys Lys Thr Glu Asn Pro Asn Glu Ile Ser Thr Lys
   1055 1060 1065
   Ser Phe Ile Gly Ala His Leu Arg Lys Gln Leu Ser Phe Asp Leu
   1070 1075 1080
   Trp Phe Leu Phe Leu Gly Leu Phe Ile Ile Cys Ile Cys Glu Gly
   1085 1090 1095
   Asp Lys Ile Lys Asp Val Gln Glu Pro Asn Phe Asn Ile Phe Ala
   1100 1105 1110
   Ile Leu Phe Glu Ile Val Ser Ala Tyr Gly Thr Val Gly Leu Ser
   1115 1120 1125
   Leu Gly Tyr Pro Asp Thr Asn Gln Ser Phe Ser Arg Gln Phe Thr
   1130 1135 1140
   Thr Leu Ser Lys Leu Val Ile Ile Ala Met Leu Ile Arg Gly Lys
   1145 1150 1155
   Asn Arg Gly Leu Pro Tyr Ser Leu Asp Arg Ala Ile Ile Leu Pro
   1160 1165 1170
   Ser Asp Arg Leu Glu His Ile Asp His Leu Glu Gly Met Lys Leu
   1175 1180 1185
   Lys Arg Gln Ala Arg Thr Asn Thr Glu Asp Pro Met Thr Glu His
   1190 1195 1200
   Phe Lys Arg Ser Phe Thr Asp Val Lys His Arg Trp Gly Ala Leu
   1205 1210 1215
   Lys Arg Lys Thr Thr His Ser Arg Asn Pro Lys Arg Ser Ser Thr
   1220 1225 1230
   Thr Leu
   1235
<210> 41
   <211> 2670
   <212> DNA
   <213> Saccharomyces cerevisiae
<400> 41
   atgccaacag ctaagaggac gtcatccagg gcttcgttgg cactgccctt ccagttacgg     60
   ttggtgcaca agaaatcatg gggccatcgg ctaagagact tcatttccgg gttcttaaaa    120
   tcatgcagac ccattgctaa atacgttttc cccaacttca tcgtggtgca ctatatctac    180
   ctgatcacgc tgtcgattat cgggtccatt ctgttatatc cgtgcaagaa cacggcgttc    240
   atcgatgtgc tatttctggc tgctggagcg tctacacagg gcgggctggc caccaagagc    300
   actaacgatt tcaacctgta ccagcagata gtggtgtacg tcattacatt gctgtccacg    360
   cctatactta ttcatgggtt tttggccttt gtcaggctgt attggtttga aaggtacttc    420
   gacaacatta gggatatctc caaacagaat tttaaactaa gaaggaccat gacgttgcaa    480
   caaagggaac tatcgggcag cagtggcaat gccgctcgaa gtaggagttt caaggacaac    540
   ctgttccgtg ggaagtttgt ttccagagaa gacccacgac aatccgcttc agatgtgccg    600
   atggactctc ctgacacgtc cgcattgtcc tcaatctcac cgttgaatgt ttcctcctct    660
   aaggaggaat ccagtgacac gcaaagctcg cctccaaact tctcaagtaa gcgccaaccc    720
   tcagacgttg acccaagaga catttacaaa tcgataatga tgctacaaaa acaacaagag    780
   aagagcaacg caaactccac ggattctttt tcgagcgaga ccaatggacc cgctttcatt    840
   gtgcaggaac gtcatgagag aagagccccc cactgctcac tgaaacgcca ttctgtcctg    900
   ccatcttctc aggaattgaa caagctagcc cagacgaaaa gtttccagaa attgcttggc    960
   ttgcggagag atgaaggtga ccatgactac tttgacggtg ctcctcacaa atatatggtc   1020
   accaagaaga aaaaaatatc tagaacgcaa tcatgtaaca tcccaacgta tactgcttca   1080
   ccgagtccta aaacctcagg ccaagtagtt gaaaatcata gaaacttggc caagtcggcg    1140
   ccttcatctt ttgttgatga ggagatgagc ttttcaccgc aagagtcttt gaatttacag   1200
   ttccaagcgc acccgcccaa accaaaacga cgtgaaggtg atataggcca ccccttcacc  1260
   agaacaatga gcaccaacta tctatcgtgg cagccaacct ttggcagaaa ctccgtcttc   1320
   attggactca caaagcaaca aaaggaggaa ctcggcggtg tcgaatatcg tgctttgaga   1380
   ttgctgtgct gcattctcat ggtatactac atcggattca acattttggc gtttgtgacc   1440
   atcgttccat gggcctgtac gaggcaccac tactcagaga ttattagacg aaatggagtt   1500
   tctccaacct ggtgggggtt tttcactgca atgagtgcat tcagcaactt gggtctgtct   1560
   ttgaccgctg attcaatggt ttcctttgat actgcgccgt atccgctgat tttcatgatg   1620
   ttcttcatca tcataggcaa tacaggcttc ccaattatgt tacgatttat catttggatc   1680
   atgttcaaga cctcgagaga cctatctcag tttaaggaaa gtcttgggtt tctcttggat   1740
   catccgcgca ggtgttttac gttgctgttc cccagcggcc ccacatggtg gctgtttaca   1800
   actttagtcg tcttaaacgc tacggattgg attcttttca taattctgga tttcaactcc   1860
   gctgtagtaa ggcaggttgc taaaggttat cgagctctca tgggcctctt ccagtctgta   1920
   tgcacaagaa ctgctggatt caacgttgtt gacttaagta aattacaccc gtccattcag   1980
   gtgtcttata tgctaatgat gtacgtttcg gtcctgccgc tggcgatttc cattagaaga   2040
   acgaatgttt atgaggagca atcgttggga ctatacgata gtggacaaga tgacgaaaat   2100
   atcacccacg aagacgatat aaaggaaaca gaccatgatg gcgaatccga agagcgagac   2160
   actgtatcta caaagtccaa gccgaagaaa cagtccccaa aatcgtttgt tggtgctcat   2220
   ttgaggaggc aactctcttt tgatttatgg tacctattcc ttggattatt tataatatgc   2280
   atatgcgagg gcagaaaaat cgaagacgtt aataaacctg atttcaatgt ctttgctata   2340
   ttgtttgaag ttgttagcgc ttatggtaca gtgggtttgt cattgggtta cccaaacacc   2400
   aacacatcac tatctgccca gttcaccgta ttatcgaagc tagtcataat tgccatgcta   2460
   ataagaggaa gaaatagagg tttaccatac actttggatc gtgccatcat gctgccaagt   2520
   gacaaactgg aacaaattga tcgtttacaa gatatgaaag ctaagggtaa gttgttagcc   2580
   aaagttggtg aggatccaat gactacttac gtcaaaaaga gatcccacaa actgaaaaaa   2640
   atagcaacaa agttttgggg gaagcattaa                                    2670
<210> 42
   <211> 889
   <212> PRT
   <213> Saccharomyces cerevisiae
<400> 42
   Met Pro Thr Ala Lys Arg Thr Ser Ser Arg Ala Ser Leu Ala Leu Pro
   1               5                   10                  15
   Phe Gln Leu Arg Leu Val His Lys Lys Ser Trp Gly His Arg Leu Arg
               20                  25                  30
   Asp Phe Ile Ser Gly Phe Leu Lys Ser Cys Arg Pro Ile Ala Lys Tyr
           35                  40                  45
   Val Phe Pro Asn Phe Ile Val Val His Tyr Ile Tyr Leu Ile Thr Leu
       50                  55                  60
   Ser Ile Ile Gly Ser Ile Leu Leu Tyr Pro Cys Lys Asn Thr Ala Phe
   65                  70                  75                  80
   Ile Asp Val Leu Phe Leu Ala Ala Gly Ala Ser Thr Gln Gly Gly Leu
                   85                  90                  95
   Ala Thr Lys Ser Thr Asn Asp Phe Asn Leu Tyr Gln Gln Ile Val Val
               100                 105                 110
   Tyr Val Ile Thr Leu Leu Ser Thr Pro Ile Leu Ile His Gly Phe Leu
           115                 120                 125
   Ala Phe Val Arg Leu Tyr Trp Phe Glu Arg Tyr Phe Asp Asn Ile Arg
       130                 135                 140
   Asp Ile Ser Lys Gln Asn Phe Lys Leu Arg Arg Thr Met Thr Leu Gln
   145                 150                 155                 160
   Gln Arg Glu Leu Ser Gly Ser Ser Gly Asn Ala Ala Arg Ser Arg Ser
                   165                 170                 175
   Phe Lys Asp Asn Leu Phe Arg Gly Lys Phe Val Ser Arg Glu Asp Pro
               180                 185                 190
   Arg Gln Ser Ala Ser Asp Val Pro Met Asp Ser Pro Asp Thr Ser Ala
           195                 200                 205
   Leu Ser Ser Ile Ser Pro Leu Asn Val Ser Ser Ser Lys Glu Glu Ser
       210                 215                 220
   Ser Asp Thr Gln Ser Ser Pro Pro Asn Phe Ser Ser Lys Arg Gln Pro
   225                 230                 235                 240
   Ser Asp Val Asp Pro Arg Asp Ile Tyr Lys Ser Ile Met Met Leu Gln
                   245                 250                 255
   Lys Gln Gln Glu Lys Ser Asn Ala Asn Ser Thr Asp Ser Phe Ser Ser
               260                 265                 270
   Glu Thr Asn Gly Pro Ala Phe Ile Val Gln Glu Arg His Glu Arg Arg
   275 280 285
   Ala Pro His Cys Ser Leu Lys Arg His Ser Val Leu Pro Ser Ser Gln
   290 295 300
   Glu Leu Asn Lys Leu Ala Gln Thr Lys Ser Phe Gln Lys Leu Leu Gly
   305 310 315 320
   Leu Arg Arg Asp Glu Gly Asp His Asp Tyr Phe Asp Gly Ala Pro His
   325 330 335
   Lys Tyr Met Val Thr Lys Lys Lys Lys Ile Ser Arg Thr Gln Ser Cys
   340 345 350
   Asn Ile Pro Thr Tyr Thr Ala Ser Pro Ser Pro Lys Thr Ser Gly Gln
   355 360 365
   Val Val Glu Asn His Arg Asn Leu Ala Lys Ser Ala Pro Ser Ser Phe
   370 375 380
   Val Asp Glu Glu Met Ser Phe Ser Pro Gln Glu Ser Leu Asn Leu Gln
   385 390 395 400
   Phe Gln Ala His Pro Pro Lys Pro Lys Arg Arg Glu Gly Asp Ile Gly
   405 410 415
   His Pro Phe Thr Arg Thr Met Ser Thr Asn Tyr Leu Ser Trp Gln Pro
   420 425 430
   Thr Phe Gly Arg Asn Ser Val Phe Ile Gly Leu Thr Lys Gln Gln Lys
   435 440 445
   Glu Glu Leu Gly Gly Val Glu Tyr Arg Ala Leu Arg Leu Leu Cys Cys
   450 455 460
   Ile Leu Met Val Tyr Tyr Ile Gly Phe Asn Ile Leu Ala Phe Val Thr
   465 470 475 480
   Ile Val Pro Trp Ala Cys Thr Arg His His Tyr Ser Glu Ile Ile Arg
   485 490 495
   Arg Asn Gly Val Ser Pro Thr Trp Trp Gly Phe Phe Thr Ala Met Ser
   500 505 510
   Ala Phe Ser Asn Leu Gly Leu Ser Leu Thr Ala Asp Ser Met Val Ser
   515 520 525
   Phe Asp Thr Ala Pro Tyr Pro Leu Ile Phe Met Met Phe Phe Ile Ile
   530 535 540
   Ile Gly Asn Thr Gly Phe Pro Ile Met Leu Arg Phe Ile Ile Trp Ile
   545 550 555 560
   Met Phe Lys Thr Ser Arg Asp Leu Ser Gln Phe Lys Glu Ser Leu Gly
   565 570 575
   Phe Leu Leu Asp His Pro Arg Arg Cys Phe Thr Leu Leu Phe Pro Ser
   580 585 590
   Gly Pro Thr Trp Trp Leu Phe Thr Thr Leu Val Val Leu Asn Ala Thr
   595 600 605
   Asp Trp Ile Leu Phe Ile Ile Leu Asp Phe Asn Ser Ala Val Val Arg
   610 615 620
   Gln Val Ala Lys Gly Tyr Arg Ala Leu Met Gly Leu Phe Gln Ser Val
   625 630 635 640
   Cys Thr Arg Thr Ala Gly Phe Asn Val Val Asp Leu Ser Lys Leu His
   645 650 655
   Pro Ser Ile Gln Val Ser Tyr Met Leu Met Met Tyr Val Ser Val Leu
   660 665 670
   Pro Leu Ala Ile Ser Ile Arg Arg Thr Asn Val Tyr Glu Glu Gln Ser
   675 680 685
   Leu Gly Leu Tyr Asp Ser Gly Gln Asp Asp Glu Asn Ile Thr His Glu
   690 695 700
   Asp Asp Ile Lys Glu Thr Asp His Asp Gly Glu Ser Glu Glu Arg Asp
   705 710 715 720
   Thr Val Ser Thr Lys Ser Lys Pro Lys Lys Gln Ser Pro Lys Ser Phe
   725 730 735
   Val Gly Ala His Leu Arg Arg Gln Leu Ser Phe Asp Leu Trp Tyr Leu
   740 745 750
   Phe Leu Gly Leu Phe Ile Ile Cys Ile Cys Glu Gly Arg Lys Ile Glu
   755 760 765
   Asp Val Asn Lys Pro Asp Phe Asn Val Phe Ala Ile Leu Phe Glu Val
   770 775 780
   Val Ser Ala Tyr Gly Thr Val Gly Leu Ser Leu Gly Tyr Pro Asn Thr
   785 790 795 800
   Asn Thr Ser Leu Ser Ala Gln Phe Thr Val Leu Ser Lys Leu Val Ile
   805 810 815
   Ile Ala Met Leu Ile Arg Gly Arg Asn Arg Gly Leu Pro Tyr Thr Leu
   820 825 830
   Asp Arg Ala Ile Met Leu Pro Ser Asp Lys Leu Glu Gln Ile Asp Arg
   835 840 845
   Leu Gln Asp Met Lys Ala Lys Gly Lys Leu Leu Ala Lys Val Gly Glu
   850 855 860
   Asp Pro Met Thr Thr Tyr Val Lys Lys Arg Ser His Lys Leu Lys Lys
   865 870 875 880
   Ile Ala Thr Lys Phe Trp Gly Lys His
   885
<210> 43
   <211> 3726
   <212> DNA
   <213> Saccharomyces uvarum
<400> 43
   atgcacatta gagggaccat gagcagagtg cccacattag catcgttcga agtacgatac    60
   aaaaaatcct tcggccacaa atttcgtgat ttcattgctc tatgtggcca ctattgctcc   120
   ccaattaaaa aatatatttt ccctaatttt atcgcagtcc attatttcta tacgatagtc   180
   ctcacgctaa taacctctat tctgttgtat ccagttaaga acatcagata tatcgacgct   240
   ttgtttttgg cagcaggtgc ggtcactcaa ggtggtttga atactgtgga tgtcaacaat   300
   ctaaccttat atcaacagat tattttgtac attatatgct gcatatcaac gcccattgcc   360
   gttcatagtt gtttggcatt cgtaagactc tattggtttg aacgttattt tgacggtatt   420
   agagactcgt ccaggctaaa tttcaaaatg agaagaacaa agacaatctt ggaaagggaa   480
   ctaaccgcta gaaccatgac caaaagtaag acaggaggga ctcaaagagt gtcacgtccc   540
   gggaaatcag acaagagaga cgatttccaa gaaaaattgt ttaatggaga aatggttaat   600
   agagatgagc aggactcggt tcatagtagc cacaattctc gtgatagtaa tagcaatgcc   660
   aataccaata gtagcaacaa taatagcatc aaccacaatg gtagtagtgg cagtttggat   720
   gattatgtta gggaagacaa agcggatgag gtcgaaaaat accacggaaa taagtcctat   780
   tcaagtgtag gtagttcgtc caatacagcc acagatgaaa atataagtca aaaattaaag   840
   ccaagtagtc ttcgattcga tgagtcgcaa aataaaagaa aacgtacggg agccccttca   900
   gaaaaatttg ccaaaaggag aggttcaaga gatatcactc ctgaagatat gtatcgatcc   960
   attatgatgc tacaaggtga gcacgagggt actgcagaag atgaaggtcc acctttagtt  1020
   attggatcgc ctacggatgg tacaagaaat atggacaacg gtagtgagtc aaagtctgct  1080
   cccacgatga atgagagtaa aatcaggatt caggataaag gagctaaaat tagccttgac  1140
   caggactccg tattacatag ttcaaattct tccgcatgca cttcggatga ggattctcta  1200
   cctacaaatt ttggaggcac aacaccttca ttgagcgcaa agccacggga atcaagttca  1260
   ggcccaatag cgttcactga aggggaaaat gctgatagaa aacaaggccc atcgattcag  1320
   ttcaatatca ctaaaccgcc aaggaaggct tctaaatcga agagggtttc tactatggat  1380
   gacttgaacc caagatcaat ttttccgcat caaaaaaaat catcgaaagg atacataatg  1440
   aaacatttgc cgaaagcccg tcgaatacgg cagcagatta aaagaagact ttccacgggt  1500
   tcgatcgata aaaacagtag cagtgacgct ctcgatagag ggctcatttc tggtctaaat  1560
   gatgatgatg atggcaacga aggtgataac atggaagagt actttgccga caacgaaagt  1620
   ggggatgaag atgaccgaat gcagcaatct gaaccacagt ctggatcaga acttaaattg  1680
   aaacaacagc aacagcacca attgcagcaa aacctgcatc gtatgtacaa gacgaaatct  1740
   tttgacgata atcgttcaaa acctgtcctc atggaaaggt ctaagactat cgatatggct  1800
   gaagctagag atttaaatga acttgccagg actcctgact ttcaaaaaat ggtttataag  1860
   aattggaaag cccatcatag aaataaacct aacttcaaaa gaaggggctg gaatggcaag  1920
   atgtttgaac atggtcctta tacatctgac agcgaccata attatcagga taatggaaat  1980
   aatagtaata gtatagttca ctacgcagaa tctattctac atcgtgataa ttctcataga  2040
   aatggaagcg aagatgtttc ttcagattct aatgagacca cctatccttt gaatggaaac  2100
   aatgatcaca gccaaaacga tgctaatggc tatcccacct acaacgatga agaggaaggc  2160
   tattacggtt tacatttcga ttctgattat aacttggatc ctcaccatgc tctatccagt  2220
   aacgctagca aaaactactt gtcatggcaa ccaaccattg gacgtaattc aaactttctt  2280
   gggttgacaa gagcccaaaa ggacgaattg ggtggtgtcg agtacagagc catcaaacta  2340
   ctatgtacca tattggttgt ctattatgtt ggatggcaca tcgtctcttt tgttatgttg  2400
   gtgccttgga ttaacttgaa aaagcactat agcgatattg tcagaagcga tggcgtttca  2460
   cctacatggt ggggtttttg gacagcaatg agtgccttca acgatttggg attaacatta  2520
   accccggact caatgatgtc atttgataaa gctgtatatc ccttgatcgt tatgatttgg  2580
   tttatcatta ttggaaatac agggtttccc atccttctta gatgcattat ttggataatg  2640
   ttcaaacttt cccctgattt gtcacagatg agagagagtt taggctttct tttagatcac  2700
   ccgcgtcgtt gttttactct attatttcca aaggctgcca catggtggct gcttttgacg  2760
   cttgtgggac taaattttac agattggatc ttatttatta ttttggattt tggatcaact  2820
   gtagttaaat cgttatcaaa aggttataga gttcttgttg gtttattcca atctgtcagt  2880
   acgaggaccg ccggtttcag tgtcgttgat ttgagtcagt tacacccctc tatccaagtc  2940
   tcctatatgc ttatgatgta tgtctccgtg ttgccattag ctatttctat tagacggaca  3000
   aatgtttacg aggagcaatc tttgggactg tatggagaaa tgggaggtaa accagaagat  3060
   actgatactg aagaggacgg cgactgcgat gatgaagatg acgacaacga agaagaggag  3120
   agccatgaag gtggaagtag ccaaaggggc aaatcaaaga aagaaacaaa aaagaaaaaa  3180
   aagagaaaag aaaatgagaa ccccaacgaa gaatcaacca agtccttcat tggtgcgcat  3240
   ttaagaagac agctatcttt tgatttatgg tttttgtttc ttgggctatt tatcatttgt   3300
   atctgcgaac gtgacaagat aaaagacatc cagagaccga acttcaatgt atttacaatt   3360
   ctttttgaga ttgtaagcgc ttatggtaca gtcggactat cattgggcta tccgaacact   3420
   aaccaatcat tttcaagaca gttaaccaca ttatctaaat taatcatcat agctatgctg   3480
   attagaggta agaatagagg tctgccctat tctttggacc gtgctattat cctgccaagt   3540
   gatagacttg aacatattga tcacattgag gacttgaaat tgaagagaca ggccagaacc   3600
   gatacggatg atccgatgac ggagcatctc aagagaagca ttagcgatgc caaacatcgt   3660
   tgggacgaac ttaaacacaa gagaagcctt tcccggagtt cgaaaagaag caccaaaacc   3720
   aactaa                                                              3726
<210> 44
   <211> 1241
   <212> PRT
   <213> Saccharomyces uvarum
<400> 44
   Met His Ile Arg Gly Thr Met Ser Arg Val Pro Thr Leu Ala Ser Phe
   1 5 10 15
   Glu Val Arg Tyr Lys Lys Ser Phe Gly His Lys Phe Arg Asp Phe Ile
   20 25 30
   Ala Leu Cys Gly His Tyr Cys Ser Pro Ile Lys Lys Tyr Ile Phe Pro
   35 40 45
   Asn Phe Ile Ala Val His Tyr Phe Tyr Thr Ile Val Leu Thr Leu Ile
   50 55 60
   Thr Ser Ile Leu Leu Tyr Pro Val Lys Asn Ile Arg Tyr Ile Asp Ala
   65 70 75 80
   Leu Phe Leu Ala Ala Gly Ala Val Thr Gln Gly Gly Leu Asn Thr Val
   85 90 95
   Asp Val Asn Asn Leu Thr Leu Tyr Gln Gln Ile Ile Leu Tyr Ile Ile
   100 105 110
   Cys Cys Ile Ser Thr Pro Ile Ala Val His Ser Cys Leu Ala Phe Val
   115 120 125
   Arg Leu Tyr Trp Phe Glu Arg Tyr Phe Asp Gly Ile Arg Asp Ser Ser
   130 135 140
   Arg Leu Asn Phe Lys Met Arg Arg Thr Lys Thr Ile Leu Glu Arg Glu
   145 150 155 160
   Leu Thr Ala Arg Thr Met Thr Lys Ser Lys Thr Gly Gly Thr Gln Arg
   165 170 175
   Val Ser Arg Pro Gly Lys Ser Asp Lys Arg Asp Asp Phe Gln Glu Lys
   180 185 190
   Leu Phe Asn Gly Glu Met Val Asn Arg Asp Glu Gln Asp Ser Val His
   195 200 205
   Ser Ser His Asn Ser Arg Asp Ser Asn Ser Asn Ala Asn Thr Asn Ser
   210 215 220
   Ser Asn Asn Asn Ser Ile Asn His Asn Gly Ser Ser Gly Ser Leu Asp
   225 230 235 240
   Asp Tyr Val Arg Glu Asp Lys Ala Asp Glu Val Glu Lys Tyr His Gly
   245 250 255
   Asn Lys Ser Tyr Ser Ser Val Gly Ser Ser Ser Asn Thr Ala Thr Asp
   260 265 270
   Glu Asn Ile Ser Gln Lys Leu Lys Pro Ser Ser Leu Arg Phe Asp Glu
   275 280 285
   Ser Gln Asn Lys Arg Lys Arg Thr Gly Ala Pro Ser Glu Lys Phe Ala
   290 295 300
   Lys Arg Arg Gly Ser Arg Asp Ile Thr Pro Glu Asp Met Tyr Arg Ser
   305 310 315 320
   Ile Met Met Leu Gln Gly Glu His Glu Gly Thr Ala Glu Asp Glu Gly
   325 330 335
   Pro Pro Leu Val Ile Gly Ser Pro Thr Asp Gly Thr Arg Asn Met Asp
   340 345 350
   Asn Gly Ser Glu Ser Lys Ser Ala Pro Thr Met Asn Glu Ser Lys Ile
   355 360 365
   Arg Ile Gln Asp Lys Gly Ala Lys Ile Ser Leu Asp Gln Asp Ser Val
   370 375 380
   Leu His Ser Ser Asn Ser Ser Ala Cys Thr Ser Asp Glu Asp Ser Leu
   385 390 395 400
   Pro Thr Asn Phe Gly Gly Thr Thr Pro Ser Leu Ser Ala Lys Pro Arg
   405 410 415
   Glu Ser Ser Ser Gly Pro Ile Ala Phe Thr Glu Gly Glu Asn Ala Asp
   420 425 430
   Arg Lys Gln Gly Pro Ser Ile Gln Phe Asn Ile Thr Lys Pro Pro Arg
   435 440 445
   Lys Ala Ser Lys Ser Lys Arg Val Ser Thr Met Asp Asp Leu Asn Pro
   450 455 460
   Arg Ser Ile Phe Pro His Gln Lys Lys Ser Ser Lys Gly Tyr Ile Met
   465 470 475 480
   Lys His Leu Pro Lys Ala Arg Arg Ile Arg Gln Gln Ile Lys Arg Arg
   485 490 495
   Leu Ser Thr Gly Ser Ile Asp Lys Asn Ser Ser Ser Asp Ala Leu Asp
   500 505 510
   Arg Gly Leu Ile Ser Gly Leu Asn Asp Asp Asp Asp Gly Asn Glu Gly
   515 520 525
   Asp Asn Met Glu Glu Tyr Phe Ala Asp Asn Glu Ser Gly Asp Glu Asp
   530 535 540
   Asp Arg Met Gln Gln Ser Glu Pro Gln Ser Gly Ser Glu Leu Lys Leu
   545 550 555 560
   Lys Gln Gln Gln Gln His Gln Leu Gln Gln Asn Leu His Arg Met Tyr
   565 570 575
   Lys Thr Lys Ser Phe Asp Asp Asn Arg Ser Lys Pro Val Leu Met Glu
   580 585 590
   Arg Ser Lys Thr Ile Asp Met Ala Glu Ala Arg Asp Leu Asn Glu Leu
   595 600 605
   Ala Arg Thr Pro Asp Phe Gln Lys Met Val Tyr Lys Asn Trp Lys Ala
   610 615 620
   His His Arg Asn Lys Pro Asn Phe Lys Arg Arg Gly Trp Asn Gly Lys
   625 630 635 640
   Met Phe Glu His Gly Pro Tyr Thr Ser Asp Ser Asp His Asn Tyr Gln
   645 650 655
   Asp Asn Gly Asn Asn Ser Asn Ser Ile Val His Tyr Ala Glu Ser Ile
   660 665 670
   Leu His Arg Asp Asn Ser His Arg Asn Gly Ser Glu Asp Val Ser Ser
   675 680 685
   Asp Ser Asn Glu Thr Thr Tyr Pro Leu Asn Gly Asn Asn Asp His Ser
   690 695 700
   Gln Asn Asp Ala Asn Gly Tyr Pro Thr Tyr Asn Asp Glu Glu Glu Gly
   705 710 715 720
   Tyr Tyr Gly Leu His Phe Asp Ser Asp Tyr Asn Leu Asp Pro His His
   725 730 735
   Ala Leu Ser Ser Asn Ala Ser Lys Asn Tyr Leu Ser Trp Gln Pro Thr
   740 745 750
   Ile Gly Arg Asn Ser Asn Phe Leu Gly Leu Thr Arg Ala Gln Lys Asp
   755 760 765
   Glu Leu Gly Gly Val Glu Tyr Arg Ala Ile Lys Leu Leu Cys Thr Ile
   770 775 780
   Leu Val Val Tyr Tyr Val Gly Trp His Ile Val Ser Phe Val Met Leu
   785 790 795 800
   Val Pro Trp Ile Asn Leu Lys Lys His Tyr Ser Asp Ile Val Arg Ser
   805 810 815
   Asp Gly Val Ser Pro Thr Trp Trp Gly Phe Trp Thr Ala Met Ser Ala
   820 825 830
   Phe Asn Asp Leu Gly Leu Thr Leu Thr Pro Asp Ser Met Met Ser Phe
   835 840 845
   Asp Lys Ala Val Tyr Pro Leu Ile Val Met Ile Trp Phe Ile Ile Ile
   850 855 860
   Gly Asn Thr Gly Phe Pro Ile Leu Leu Arg Cys Ile Ile Trp Ile Met
   865 870 875 880
   Phe Lys Leu Ser Pro Asp Leu Ser Gln Met Arg Glu Ser Leu Gly Phe
   885 890 895
   Leu Leu Asp His Pro Arg Arg Cys Phe Thr Leu Leu Phe Pro Lys Ala
   900 905 910
   Ala Thr Trp Trp Leu Leu Leu Thr Leu Val Gly Leu Asn Phe Thr Asp
   915 920 925
   Trp Ile Leu Phe Ile Ile Leu Asp Phe Gly Ser Thr Val Val Lys Ser
   930 935 940
   Leu Ser Lys Gly Tyr Arg Val Leu Val Gly Leu Phe Gln Ser Val Ser
   945 950 955 960
   Thr Arg Thr Ala Gly Phe Ser Val Val Asp Leu Ser Gln Leu His Pro
   965 970 975
   Ser Ile Gln Val Ser Tyr Met Leu Met Met Tyr Val Ser Val Leu Pro
   980 985 990
   Leu Ala Ile Ser Ile Arg Arg Thr Asn Val Tyr Glu Glu Gln Ser Leu
   995 1000 1005
   Gly Leu Tyr Gly Glu Met Gly Gly Lys Pro Glu Asp Thr Asp Thr
   1010 1015 1020
   Glu Glu Asp Gly Asp Cys Asp Asp Glu Asp Asp Asp Asn Glu Glu
   1025 1030 1035
   Glu Glu Ser His Glu Gly Gly Ser Ser Gln Arg Gly Lys Ser Lys
   1040 1045 1050
   Lys Glu Thr Lys Lys Lys Lys Lys Arg Lys Glu Asn Glu Asn Pro
   1055 1060 1065
   Asn Glu Glu Ser Thr Lys Ser Phe Ile Gly Ala His Leu Arg Arg
   1070 1075 1080
   Gln Leu Ser Phe Asp Leu Trp Phe Leu Phe Leu Gly Leu Phe Ile
   1085 1090 1095
   Ile Cys Ile Cys Glu Arg Asp Lys Ile Lys Asp Ile Gln Arg Pro
   1100 1105 1110
   Asn Phe Asn Val Phe Thr Ile Leu Phe Glu Ile Val Ser Ala Tyr
   1115 1120 1125
   Gly Thr Val Gly Leu Ser Leu Gly Tyr Pro Asn Thr Asn Gln Ser
   1130 1135 1140
   Phe Ser Arg Gln Leu Thr Thr Leu Ser Lys Leu Ile Ile Ile Ala
   1145 1150 1155
   Met Leu Ile Arg Gly Lys Asn Arg Gly Leu Pro Tyr Ser Leu Asp
   1160 1165 1170
   Arg Ala Ile Ile Leu Pro Ser Asp Arg Leu Glu His Ile Asp His
   1175 1180 1185
   Ile Glu Asp Leu Lys Leu Lys Arg Gln Ala Arg Thr Asp Thr Asp
   1190 1195 1200
   Asp Pro Met Thr Glu His Leu Lys Arg Ser Ile Ser Asp Ala Lys
   1205 1210 1215
   His Arg Trp Asp Glu Leu Lys His Lys Arg Ser Leu Ser Arg Ser
   1220 1225 1230
   Ser Lys Arg Ser Thr Lys Thr Asn
   1235 1240
<210> 45
   <211> 1828
   <212> DNA
   <213> Oryza sativa
<400> 45
   gcttgagtca tagggagaaa acaaatcgat catatttgac tcttttccct ccatctctct      60
   taccggcaaa aaaagtagta ctggtttata tgtaaagtaa gattctttaa ttatgtgaga     120
   tccggcttaa tgcttttctt ttgtcacata tactgcattg caacaattgc catatattca     180
   cttctgccat cccattatat agcaactcaa gaatggattg atatatcccc tattactaat     240
   ctagacatgt taaggctgag ttgggcagtc catcttccca acccaccacc ttcgtttttc     300
   gcgcacatac ttttcaaact actaaatggt gtgtttttta aaaatatttt caatacaaaa     360
   gttgctttaa aaaattatat tgatccattt ttttaaaaaa aatagctaat acttaattaa     420
   tcacgtgtta aaagaccgct ccgttttgcg tgcaggaggg ataggttcac atcctgcatt     480
   accgaacaca gcctaaatct tgttgtctag attcgtagta ctggatatat taaatcatgt     540
   tctaagttac tatatactga gatgaataga ataagtaaaa ttagacccac cttaagtctt     600
   gatgaagtta ctactagctg cgtttgggag gacttcccaa aaaaaaaagt attagccatt     660
   agcacgtgat taattaagta ctagtttaaa aaacttaaaa aataaattaa tatgattctc     720
   ttaagtaact ctcctataga aaacttttac aaaattacac cgtttaatag tttggaaaat     780
   atgtcagtaa aaaataagag agtagaagtt atgaaagtta gaaaaagaat tgttttagta     840
   gtatacagtt ataaactatt ccctctgttc taaaacataa gggattatgg atggattcga     900
   catgtaccag taccatgaat cgaatccaga caagtttttt atgcatattt attctactat     960
   aatatatcac atctgctcta aatatcttat atttcgaggt ggagactgtc gctatgtttt    1020
   tctgcccgtt gctaagcaca cgccaccccc gatgcgggga cgcctctggc cttcttgcca    1080
   cgataattga atggaacttc cacattcaga ttcgataggt gaccgtcgac tccaagtgct    1140
   ttgcacaaaa caactccggc ctcccggcca ccagtcacac gactcacggc actaccaccc    1200
   ctgactccct gaggcggacc tgccactgtt ctgcatgcga agctatctaa aattctgaag    1260
   caaagaaagc acagcacatg ctccgggaca cgcgccaccc ggcggaaaag ggctcggtgt    1320
   ggcgatctca cagccgcata tcgcatttca caagccgccc atctccaccg gcttcacgag    1380
   gctcatcgcg gcacgaccgc gcacggaacg cacgcggccg acccgcgcgc ctcgatgcgc    1440
   gagcccatcc gccgcgtcct ccctttgcct ttgccgctat cctctcggtc gtatcccgtt    1500
   tctctgtctt ttgctccccg gcgcgcgcca gttcggagta ccagcgaaac ccggacacct    1560
   ggtacacctc cgccggccac aacgcgtgtc ccccctacgt ggccgcgcag cacatgccca    1620
   tgcgcgacac gtgcacctcc tcatccaaac tctcaagtct caacggtcct ataaatgcac    1680
   ggatagcctc aagctgctcg tcacaaggca agaggcaaga ggcaagagca tccgtattaa    1740
   ccagcctttt gagacttgag agtgtgtgtg actcgatcca gcgtagtttc agttcgtgtg    1800
   ttggtgagtg attccagcca agtttgcg                                       1828

## Description

The present invention relates generally to the field of molecular biology and concerns a method for improving plant growth characteristics. More specifically, the present invention concerns a method for improving plant growth characteristics, in particular for increasing yield, by increasing activity in a plant of a Na⁺-K⁺ cotransporter protein (HKT) or a homologue thereof. The present disclosure also concerns plants having increased HKT activity, which plants have improved growth characteristics relative to corresponding wild type plants. The disclosure also provides constructs useful in the methods of the invention.

Given the ever-increasing world population, and the dwindling area of land available for agriculture, it remains a major goal of agricultural research to improve the efficiency of agriculture and to increase the diversity of plants in horticulture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic complements that may not always result in the desirable trait being passed on from parent plants. Advances in molecular biology have allowed mankind to manipulate the germplasm of animals and plants. Genetic engineering of plants entails the isolation and manipulation of genetic material (typically in the form of DNA or RNA) and the subsequent introduction of that genetic material into a plant Such technology has led to the development of plants having various improved economic, agronomic or horticultural traits. Traits of particular economic interest are growth characteristics such as high yield. Yield is normally defined as the measurable produce of economic value from a crop. This may be defined in terms of quantity and/or quality. Yield is directly dependent on several factors, for example, the number and size of the organs, plant architecture (for example, the number of branches), seed production and more. Root development, nutrient uptake and stress tolerance may also be important factors in determining yield. Crop yield may therefore be increased by optimising one of the above-mentioned factors.

A major abiotic stress factor for plants is salinity. Salt stress, and in particular Na⁺ stress, may negatively influence several cellular processes. Besides hyperosmotic damage, which comprises membrane dysfunction, high sodium concentrations may interfere with Na⁺ sensitive enzymes and may result in distorted ion transport (for an overview of cellular and molecular responses, see Hasegawa et al., 2000. Annu. Rev. Plant Physiol. Plant Mol. Biol. 51, 463-499). On the other hand, potassium is an important nutrient, necessary for neutralizing negative charges on proteins, activation of K⁺-ependent enzymes, maintenance of cell turgor and osmotic homeostasis. Na⁺ and K⁺ transport are linked to each other: experimental data indicate that both ions use the same transport proteins and plants may compensate shortage of potassium by taking up sodium (Pitman, 1967. Nature 216,1343-1344; Pitman et al., 1968. Aust. J. Biol. Sci 21, 871-881). Voltage-insensitive monovalent-cation channels (VIC) play an important role in the uptake of Na⁺ and K⁺ in plant cells (White, 1999. Trends Plant Sci. 4, 245-246). In addition, several K⁺ up taking proteins have been described in *Arabidopsis* (Maser et al., 2001. Plant Physiol. 126:1646-1667): AKT/KAT-type channel proteins, HAK/AT/KUP-like transporter proteins and HKT-type transporter proteins. Plant HKT proteins are part of a superfamily of cation transporters that also comprise the yeast Trk proteins and prokaryotic KdpA, KrkH and KtrB proteins (Schachtman & Liu, 1999. Trends Plant Sci. 4, 281-286).

The transmembrane protein AtHKT1 was shown to be also involved in Na⁺ uptake in roots and in salt tolerance in *Arabidopsis* (Uozumi et al., 2000. Plant Physiol. 122, 1249-1259; Rus et al., 2001. Proc. Natl. Acad. Sci. USA 98, 14150-14155); its rice counterpart (OsHKT1) fulfilled a similar function under conditions of potassium deprivation (Garciadeblás et al, 2003. Plant J. 34, 788-801). AtHKT1 was also reported to be important in recirculation of Na⁺ from the shoots to the roots, thereby contributing to plant salt tolerance (Berthomieu et al, 2003. EMBO J. 22, 2004-2014). In rice, 9 types of HKT transporters have been identified and characterised (Garciadeblás et al, 2003. Plant J. 34, 788-801, Horie et al, 2001. Plant J. 27, 129-138). In addition, rice HKT1 not only mediated Na⁺ and K⁺ transport, but also mediated transport of other alkali cations (Golldack et al., 2002. Plant J. 31, 529-542). The effect of high and low HKT1 protein levels was studied in rice (Golldack et al., 2002): the salt tolerant line Pokkali and the salt sensitive line IR29 differed significantly from each other in their ability to take up or exclude sodium. High concentrations of alkali ions repressed OsHKT1 expression in both lines, but this repression was less pronounced in root and leaf vascular tissues of the IR29 line than in the salt tolerant line Pokkali.

Few data are available with respect to heterologous expression of *HKT1* genes in plants. Schroeder and Schachtman (WO96/05722) disclose an HKT1 protein from wheat and suggested use of this HKT1 protein to modulate salt uptake from the environment by manipulating its expression, resulting in plants that can be used for desalinization (increased sodium uptake upon enhanced *HKT1* expression) or in plants that are resistant to toxic alkali metals (decreased or inhibited uptake by repressing *HKT1* expression). However as shown by Laurie et al. (Plant J. 32, 139-149, 2002), plants overexpressing *HKT1* had a phenotype that was not much different from the control plants: under NaCl stress, the fresh weight of the HKT1 overexpressing line was reduced to a similar degree as for the control plants and there was no significant increase in the Na⁺ content of roots, compared to the control. Laurie et al. have also shown that the conditions for obtaining downregulated expression of HKT1 are quite complex. So far, the prior art relating to HKT1 was mainly focused on cation transport and ion homeostasis, and many studies were performed in yeast or *Xenopus* oocytes, which results may not be fully reflective for HKT1 function in plants.

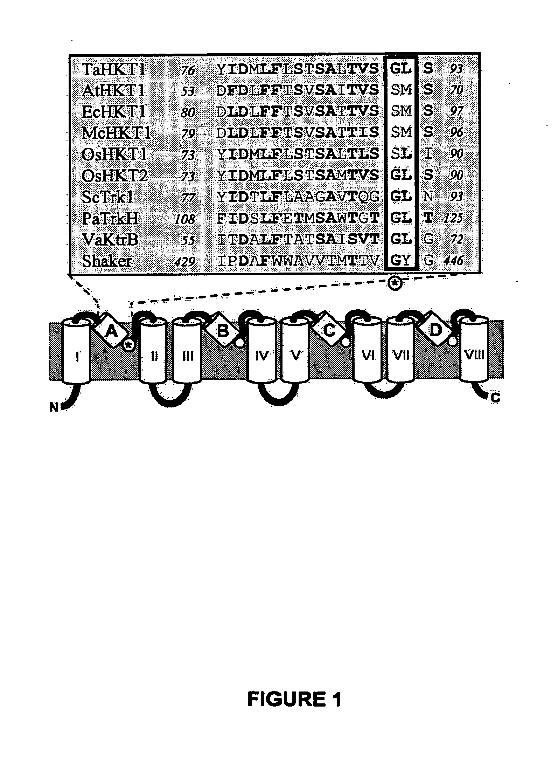

AtHKT1 is a protein encoded by a single gene in *Arabidopsis thaliana.* The gene is thought to be present in all plant genomes, and may sometimes occur as a small gene family, for example as in rice. The *AtHKT1* gene is expressed in roots and to a lesser extent in other tissues (Uozumi et al., 2000). A three dimensional model of an HKT type protein was constructed using sequence and hydrophobicity analysis (Durell et al., Biophys. J. 77, 775-788, 1999; Durell and Guy, Biophys. J. 77, 789-807, 1999): the protein was shown to be hydrophobic and comprised a core structure of eight transmembrane domains flanking four loops that formed a pore (in other words: four units of a transmembrane domain-pore forming domain-transmembrane domain), each pore forming domain was predicted to be located partly inside the membrane (see Figure 1) and comprised a conserved Glycine or Serine residue. Heterologous expression of wild type *AtHKT1* revealed this protein to selectively mediate Na⁺ transport while K⁺ transport occurred to a lesser degree (Uozumi et al., 2000). Other HKT proteins from different plant species show a Na⁺/K⁺ symporter activity. The ion selectivity is believed to be determined by the first pore forming domain (or P-loop). Mutating Ser-68 to Glycine in the *AtHKT1* gene restored the permeability for K⁺. Glycine residues corresponding to the mutated Gly-68 of AtHKT1 were found in other HKT proteins; it was shown that this is the first Gly in a conserved "GYG" motif that functions as an ion selectivity filter (Mäser et al., Proc. Natl. Acad. Sci. USA 99, 6428-6433, 2002). In addition to Na⁺ and K⁺, HKT type proteins were also shown to transport other cations like Rb⁺, Li⁺ and Cs⁺ (Golldack et al., 2002). Besides *Arabidopsis,* HKT proteins were isolated from various other plant species, including rice, wheat, *Eucalyptus camaldulensis, Hordeum vulgare* and *Mesembryanthemum crystallinum.*

It has now surprisingly been found that increasing activity of an HKT protein or a homologue thereof in a plant gives rise to plants having improved growth characteristics compared to wild type plants, in particular under non-stress conditions.

According to one embodiment of the present invention, there is provided a method for increasing yield of a plant as described in claim 1. Optionally there may follow a step for selecting plants having increased yield. Advantageously, performance of the methods according to the present invention results in plants having increased yield, particularly seed yield.

The term "increased yield" as defined herein is taken to mean an increase in any one or more of the following, each relative to corresponding wild type plants: (i) increased biomass (weight) of one or more parts of a plant, particularly aboveground (harvestable) parts, increased root biomass or increased biomass of any other harvestable part; (ii) increased total seed yield, which includes an increase in seed biomass (seed weight) and which may be an increase in the seed weight per plant or on an individual seed basis; (iii) increased number of flowers ("florets") per panicle (iv) increased number of (filled) seeds; (v) increased seed size, which may also influence the composition of seeds; (vi) increased seed volume, which may also influence the composition of seeds (including oil, protein and carbohydrate total content and composition); (vii) increased individual seed area; (viii) increased individual seed length and/or width; (ix) increased harvest index, which is expressed as a ratio of the yield of harvestable parts, such as seeds, over the total biomass; and (x) increased thousand kernel weight (TKW), which is extrapolated from the number of filled seeds counted and their total weight An increased TKW may result from an increased seed size and/or seed weight. An increased TKW may result from an increase in embryo size and/or endosperm size.

Taking corn as an example, a yield increase may be manifested as one or more of the following: increase in the number of plants per hectare or acre, an increase in the number of ears per plant, an increase in the number of rows, number of kernels per row, kernel weight, TKW, ear length/diameter, among others. Taking rice as an example, a yield increase may be manifested by an increase in one or more of the following: number of plants per hectare or acre, number of panicles per plant, number of spikelets per panicle, number of flowers per panicle, increase in the seed filling rate, increase in TKW, among others. An increase in yield may also result in modified architecture, or may occur as a result of modified architecture.

Performance of the methods according to the present invention results in plants having increased yield and more particularly, increased biomass and/or increased seed yield.

Since the improved plants according to the present invention have increased yield, it is likely that these plants exhibit an increased growth rate (during at least part of their life cycle), relative to the growth rate of corresponding wild type plants at a corresponding stage in their life cycle. The increased growth rate may be specific to one or more parts or cell types of a plant (including seeds), or may be throughout substantially the whole plant. Plants having an increased growth rate may have a shorter life cycle. The life cyde of a plant is taken to mean the time needed to grow from a dry mature seed up to the stage where the plant has produced dry mature seeds similar to the starting material. This life cycle may be influenced by factors such as early vigour, growth rate, flowering time and speed of seed maturation. An increase in growth rate may take place at one or more stages in the life cyde of a plant or during substantially the whole plant life cycle. Increased growth rate during the early stages in the life cyde of a plant may reflect enhanced vigour. The increase in growth rate may alter the harvest cyde of a plant allowing plants to be sown later and/or harvested sooner than would otherwise be possible. If the growth rate is sufficiently increased, it may allow for the sowing of further seeds of the same plant species (for example sowing and harvesting of rice plants followed by sowing and harvesting of further rice plants all within one conventional growing period). Similarly, if the growth rate is sufficiently increased, it may allow for the sowing of further seeds of different plants species (for example the sowing and harvesting of rice plants followed by, for example, the sowing and optional harvesting of soy bean, potatoes or any other suitable plant). Harvesting additional times from the same rootstock in the case of some plants may also be possible. Altering the harvest cyde of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves plotting growth experiments, such parameters may be: T-Mid (the time taken for plants to reach 50% of their maximal size) and T-90 (time taken for plants to reach 90% of their maximal size), amongst others.

An increase in yield and/or growth rate occurs whether the plant is under non-stress conditions or whether the plant is exposed to various stresses compared to control plants. It should thus be understood that in the present invention the increase in yield and/or growth rate is not dependent on the growth conditions, whether they be stress conditions or non-stress conditions. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the typical stresses to which a plant may be exposed. These stresses may be the everyday biotic and/or abiotic (environmental) stresses to which a plant is exposed. Typical abiotic or environmental stresses include temperature stresses caused by atypical hot or cold/freezing temperatures; salt stress; water stress (drought or excess water). Abiotic stresses may also be caused by chemicals. Biotic stresses are typically those stresses caused by pathogens, such as bacteria, viruses, fungi and insects. The term "non-stress conditions" as used herein are those environmental conditions that do not significantly go beyond the everyday climatic and other abiotic stress conditions that plants may encounter. Persons skilled in the art are aware of normal soil conditions and climatic conditions for a given geographic location.

The abovementioned growth characteristics may advantageously be improved in any plant.

The term "plant" as used herein encompasses whole plants, ancestors and progeny of the plants and plant parts, including seeds, shoots, stems, leaves, roots (including tubers), flowers, and tissues and organs. The term plant" also encompasses plant cells, suspension cultures, callus tissue, embryos, meristematic regions, gametophytes, sporophytes, pollen, and microspores. The term "plant" furthermore encompasses plants, plant parts or plant cells modified by human intervention, such as mutated or transformed plants or plant parts, which mutated or transformed plants or plant parts comprise the gene/nucleic acid of interest or the specific modification in the gene/nucleic acid of interest.

Plants that are particularly useful in the methods of the invention include algae, ferns, and all plants which belong to the superfamily Viridiplantae, in particular monocotyledonous and dicotyledonous plants, including fodder or forage legumes, ornamental plants, food crops, trees, or shrubs selected from the list comprising *Abelmoschus* spp., Acerspp., *Actinidia* spp., *Agropyron* spp., *Allium* spp., *Amaranthus* spp., *Ananas comosus, Annona* spp., *Apium graveolens, Arabidopsis thaliana, Arachis* spp, *Artocarpus* spp., *Asparagus officinalis, Avena sativa, Averrhoa carambola, Benincasa hispida, Bertholletia excelsea, Beta vulgaris, Brassica* spp., *Cadaba farinosa, Camellia sinensis, Canna indica, Capsicum* spp., *Carica papaya, Carissa macrocarpa, Carthamus tinctorius, Carya* spp., *Castanea* spp., *Cichorium endivia, Cinnamomum spp., Citrullus lanatus, Citrus* spp., Cocos spp., *Coffea* spp., *Cola* spp., *Colocasia esculenta, Corylus* spp., *Crataegus* spp., *Cucumis* spp., *Cucurbita* spp., *Cynara* spp., *Daucus carota, Desmodium* spp., *Dimocarpus longan, Dioscorea* spp., *Diospyros* spp., *Echinochloa* spp., *Eleusine coracana, Eriobotrya japonica, Eugenia uniflora, Fagopyrum* spp., *Fagus* spp., *Ficus carica, Fortunella spp., Fragaria* spp., *Ginkgo biloba, Glycine* spp., *Gossypium hirsutum, Helianthus* spp., *Hibiscus* spp., *Hordeum* spp., *Ipomoea batatas, Juglans* spp., *Lactuca sativa, Lathyrus* spp., *Lemna spp., Lens culinaris, Linum usitatissimum, Litchi chinensis, Lotus* spp., *Luffa acutangula, Lupinus* spp., *Macrotyloma* spp., *Malpighia emarginata, Malus* spp., Mammea *americana, Mangifera indica, Manihot* spp., *Manilkara zapota, Medicago sativa, Melilotus* spp., *Mentha* spp., *Momordica* spp., *Morus nigra, Musa* spp., *Nicotiana* spp., *Olea* spp., *Opuntia* spp., *Omithopus* spp., *Oryza* spp., *Panicum miliaceum, Passiflora edulis, Pastinaca sativa, Persea* spp., *Petroselinum crispum, Phaseolus* spp., *Phoenix* spp., *Physalis* spp., *Pinus* spp., *Pistacia vera, Pisum* spp., Poa spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Rubus* spp., *Saccharum* spp., *Sambucus* spp., *Secale cereale, Sesamum* spp., *Solanum* spp., *Sorghum bicolor, Spinacia* spp., *Syzygium* spp., *Tamarindus indica, Theobroma cacao, Trifolium* spp., *Tifficosecale rimpaui, Triticum* spp., *Vaccinium* spp., *Vicia* spp., *Vigna spp., Vitis* spp., Zea *mays, Zizania palustris, Ziziphus* spp., amongst others.

According to a preferred feature of the present invention, the plant is a crop plant such as soybean, sunflower, canola, alfalfa, rapeseed or cotton. Further preferably, the plant according to the present invention is a monocotyledonous plant such as sugarcane, most preferably a cereal, such as rice, maize, wheat, millet, barley, rye, oats or sorghum.

The activity of an HKT protein or a homologue thereof may be increased by increasing levels of the HKT polypeptide, for example by increasing the level of nucleic acids encoding an HKT protein. Alternatively, activity may also be increased when there is no change in levels of an HKT, or even when there is a reduction in levels of an HKT. This may occur when the intrinsic properties of the polypeptide are altered, for example, by making a mutant or selecting a variant that is more active than the wild type.

The term "HKT protein or homologue thereof as defined herein refers to a polypeptide with HKT activity and which polypeptide comprises four units of a transmembrane domain - pore forming domain - transmembrane domain. The HKT protein has a sequence as represented by SEQ ID NO: 2, or is a homologue thereof having in increasing order of preference at least 35%, 36%, 37%, 38%, 39%, 40%, 41%. 42%, 43%. 44%, 45%, 46%. 47%, 48%, 49%, 50%, 55%. 60%, 65%, 70%, 75%, 80%, 85%, 90%. 95% overall sequence identity to the amino acid represented by SEQ ID NO: 2 having cation transports activity. The overall sequence identity is determined using an alignment algorithm that can perform lobal alignments, such as the Needleman Wunsch algorithm in the program GAP (GCG Wisconsin Package, Accelrys).

An "HKT protein or a homologue thereof" falling within the above definition may readily be identified using routine techniques well known to persons skilled in the art. For example, HKT acitivity as cation transporter may readily be determined *in vitro* or *in vivo* using techniques well known in the art. For example, the complementation and the effect on K⁺ uptake upon cloning of an *HKT* gene in the *Escherichia coli* strain LB2003 (deficient for the K⁺ uptake systems Trk, Kup and Kdp) may be assayed as described by Uozumi et al. (2000). Similarly, the *Saccharomyces cerevisiae* strain CY162 (deficient for the high affinity uptake systems *trk1* and *trk2*) may be complemented by cloning an *HKT* gene, whereas the yeast strain G19 (deficient for the Na⁺ extruding ATPase genes *ENA1* to *ENA4*) or a wild type yeast strain will exhibit growth inhibition upon cloning of an HKT gene (Horie et al., 2001). Alternatively, a voltage damp assay may be performed using *Xenopus laevis* oocytes (see for example Horie et al., 2001) or a cation uptake/depletion test in roots or yeast as described by Garciadeblas et al. (Plant J. 34, 788-801, 2003) and Banuelos et al. (Plant Physiol. 130, 784-795, 2002). At least one of the above mentioned assays (or other assays known in the art) will demonstrate the cation transporting activity of an HKT protein or a homologue thereof. Alternatively, such "HKT protein or homologue thereof", when expressed under control of a WSI18 promoter in the *Oryza sativa* cultivar Nipponbare, increases seed yield compared to corresponding wild type plants. This increase in seed yield may be measured in several ways, for example as an increase of the number of filled seeds. Increased HKT activity thus encompasses at least one of increased levels of a nucleic acid encoding an HKT protein or a homologue thereof, increased levels of an HKT protein or a homologue thereof, increased transporter activity or increased seed yield.

Techniques for measuring increased levels of an HKT encoding nucleic acid are known in the art and include for example Northern Blotting, Real Time-PCR or Quantitative-PCR. Increased HKT protein levels may be determined using for example Western Blotting, by estimating band/spot intensity after gel electrophoresis of a crude protein sample, or by testing enzymatic activity (if applicable).

The various structural domains in an HKT protein may be identified using specialised databases e.g. SMART (Schultz et al. (1998) Proc. Natl. Acad. Sci. USA 95, 5857-5864; Letunic et al. (2002) Nucleic Acids Res 30, 242-244; http://smart.embl-heidelberg.de/), InterPro (Mulder et al., (2003) Nud. Acids. Res. 31, 315-318; http://www.ebi.ac.uk/interpro/), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAIPress, Menlo Park; Hulo et al., Nud. Acids. Res. 32:D134-D137, (2004), http://www.expasy.org/prosite/) or Pfam (Bateman et al., Nucleic Acids Research 30(1):276-280 (2002), http://www.sanger.ac.uk/Software/Pfam/).

The structural domains in an HKT protein are known in the art and the topological model is supported by experimental evidence (Kato et al., Proc. Natl. Acad. Sci. USA 98, 6488-6493, 2001). The HKT group of proteins consists of proteins comprising four "transmembrane domain - pore domain - transmembrane domain" units (MPM). Each of the four pore forming domains comprises a conserved Glycine or Serine residue. The conserved Glycine (or Serine) residue in the first pore forming domain (indicated by an asterisk in domain A in Figure 1) determines cation specificity. Each MPM unit comprises an MPM motif as characterised by Durell et al. (1999). For example, the fourth MPM unit in AtHKT1 (SEQ ID NO: 2) comprises the motif: (SEQ ID NO: 7), wherein the bold underlined parts of the sequence represent the transmembrane motif and the underlined part in italics indicates the pore-forming loop motif, with the conserved Glycine residue in bold italics.

HKT proteins, such as SEQ ID NO: 2, also comprise a TrkH domain (Pfam accession number PF02386, Interpro accession IPR003445) (starting at G145 and ending with Y502 for SEQ ID NO: 2), which is characteristic for a group of proteins comprising potassium transport proteins (Trk) and V-type sodium ATP synthase subunit J or translocating ATPase J.

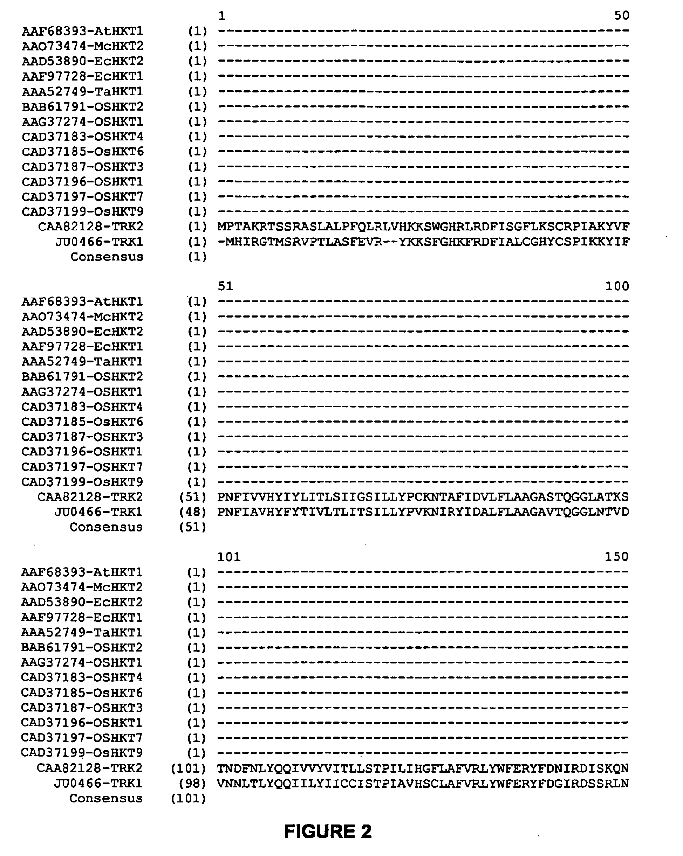

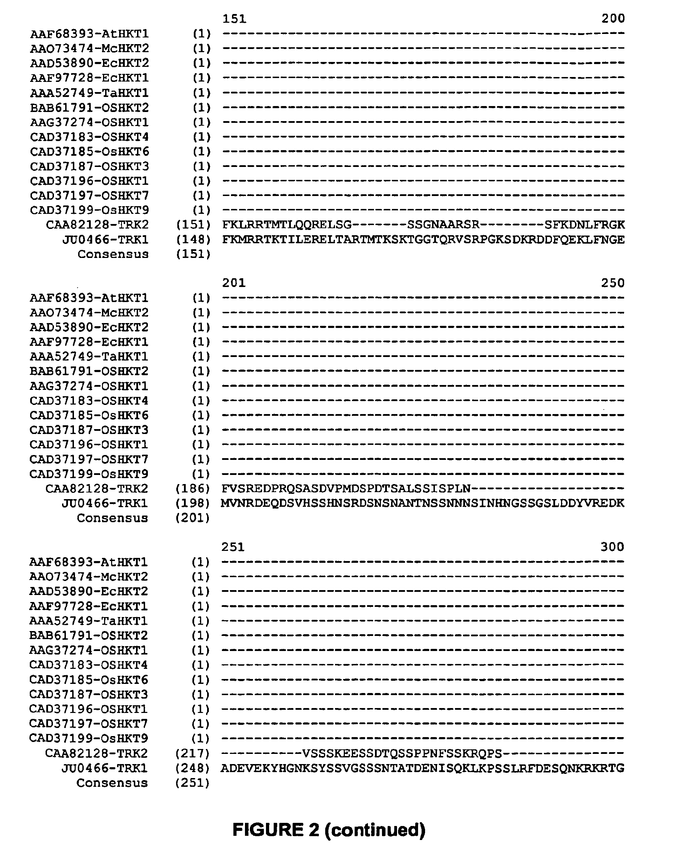

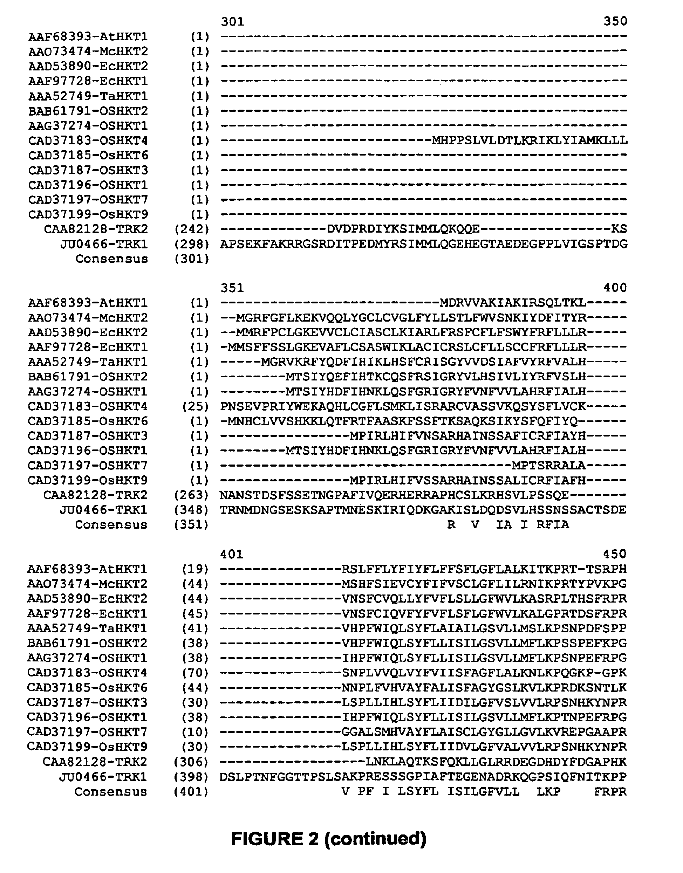

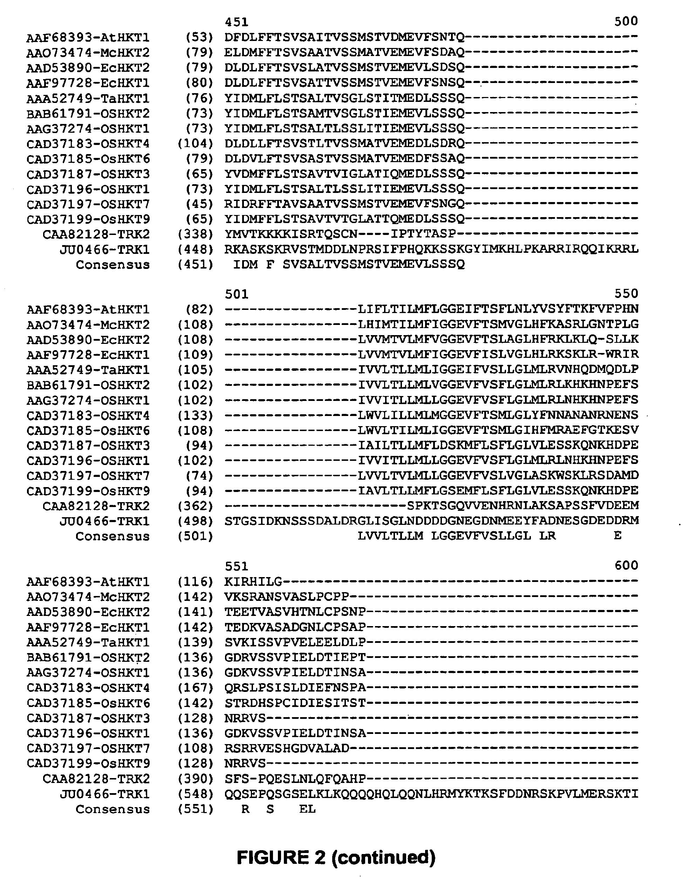

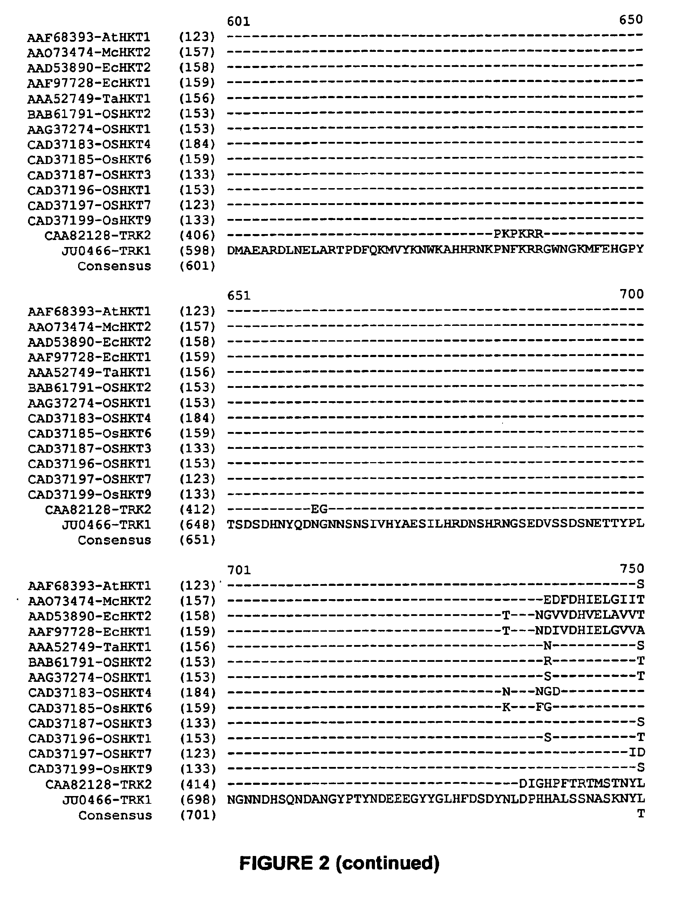

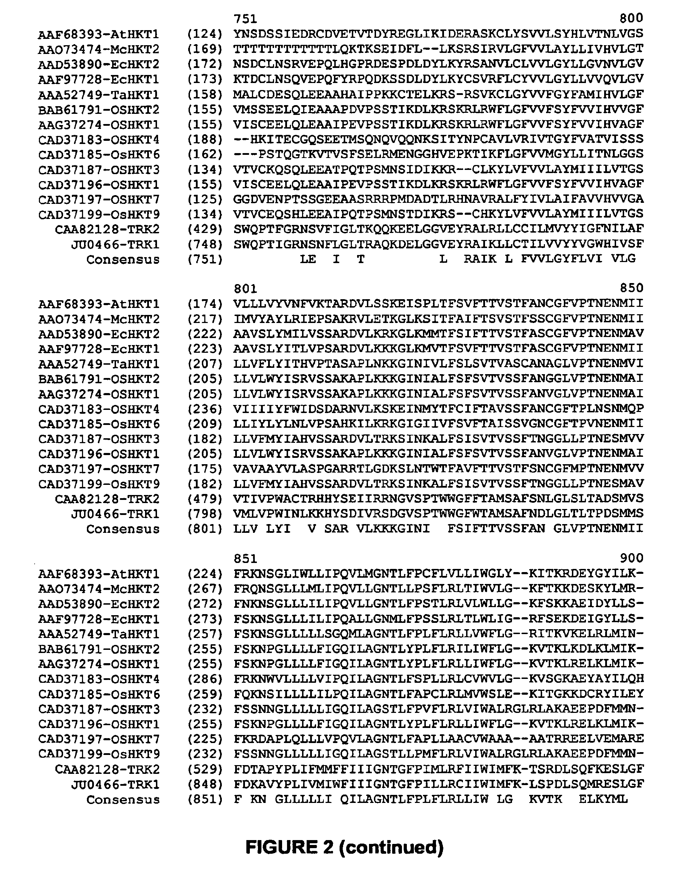

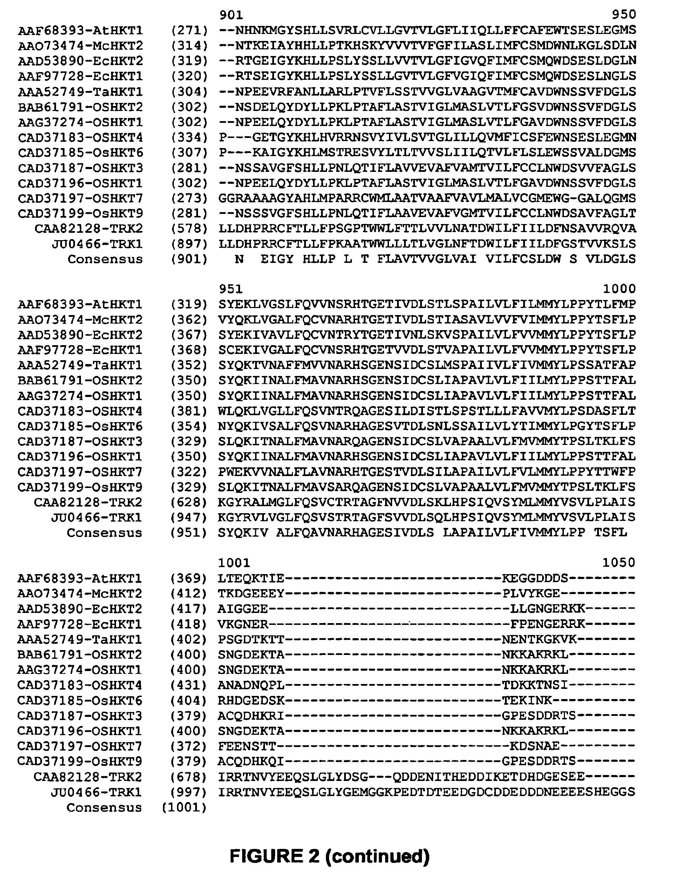

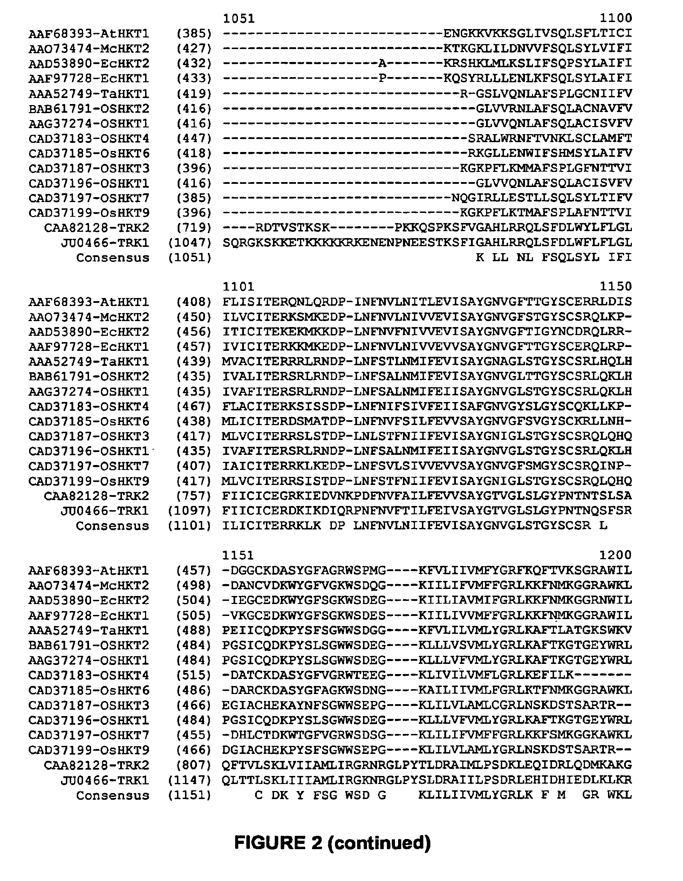

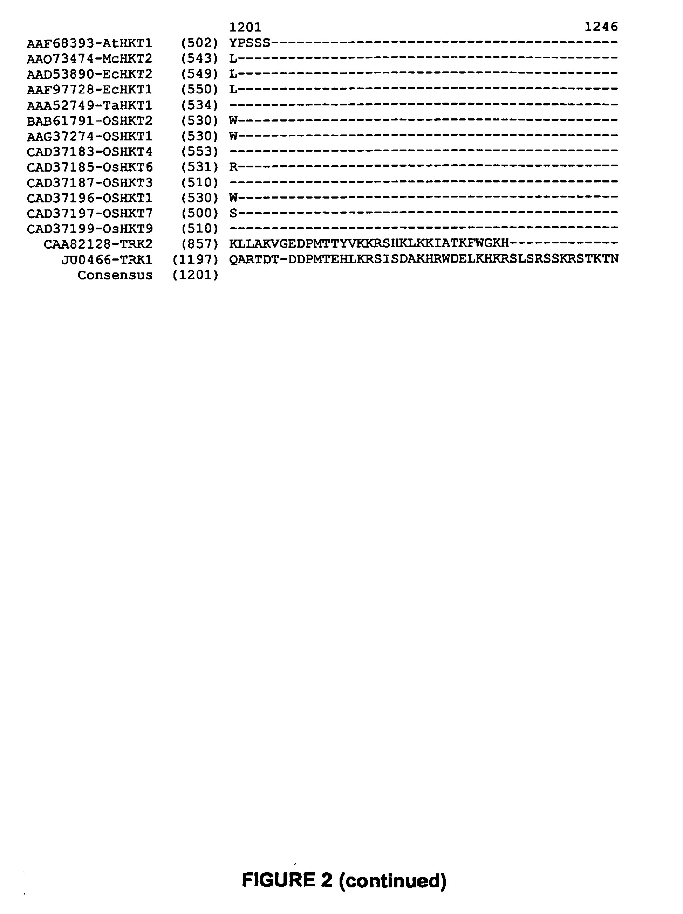

Preferably, the HKT protein useful in the present invention comprises in the fourth pore-forming domain a consensus sequence corresponding to **E**VI**SA**Y**G**NV**G**FTT**GY** (SEQ ID NO: 8) wherein the residues indicated in bold are invariably conserved and wherein the other residues may vary (see for example Figure 2). Preferably, the consensus sequence in the fourth pore-forming domain corresponds to
E(V,I) (I,V)SA(Y,F)G(N,T) (V,A,I)G(F,L,Y) (T,S) (T,I,L,V,M)GY (SEQ ID NO:9). More preferably, the consensus sequence in the fourth pore-forming domain corresponds to E(V,I) (I,V)SA(Y,F)GNVG(F,L,Y) (T,S) (T,L,V)GY.

Alternatively, the HKT protein useful in the methods of the present invention comprises in the fourth pore-forming domain an SA(Y,F)GN sequence signature and a DP(I,L)N(Y,F,L) sequence signature (SEQ ID NO: 10). Preferably, the signature sequence of SEQ ID NO: 10 **is** DP(I,L)NF.

Whether a polypeptide has at least 35% identity to the amino acid represented by SEQ ID NO: 2 may readily be established by sequence alignment Methods for the search and identification of HKT homologues would be well within the realm of persons skilled in the art. Such methods comprise comparison of the sequences represented by SEQ ID NO: 1 or SEQ ID NO: 2, in a computer readable format, with sequences that are available in public databases such as MIPS (http://mips.gsf.de/), GenBank (http:/www.ncbi.nlm.nih.gov/Genbank/index.html) or the EMBL Nucleotide Sequence Database (http://www.ebi.ac.uk/embl/index.html), using algorithms well known in the art for the alignment or comparison of sequences, such as GAP (Needleman and Wunsch, J. Mol. Biol. 48; 443-453 (1970)), BESTFIT (using the local homology algorithm of Smith and Waterman (Advances in Applied Mathematics 2; 482-489 (1981))), BLAST (Altschul, S.F., Gish, W., Miller, W., Myers, E.W. & Lipman, D.J., J. Mol. Biol. 215:403-410 (1990)), FASTA and TFASTA (W. R. Pearson and D. J. Lipman Proc.Nati.Acad.Sci. USA 85:2444- 2448 (1988)), or using, for example, the VNTI AlignX multiple alignment program, based on a modified Clustal W algorithm (InforMax, Bethesda, MD, http://www.informaxinc.com), with default settings for gap opening penalty of 10 and a gap extension of 0.05. The software for performing BLAST analysis is publicly available through the National Centre for Biotechnology information (NCBI). The homologues mentioned below were identified using BLAST default parameters (BLOSUM62 matrix, gap opening penalty 11 and gap extension penalty 1) and preferably the full-length sequences are used for analysis.

Examples of polypeptides falling under the definition of an "HKT or a homologue thereof" include two *Eucalyptus camaldulensis* homologues (SEQ ID NO: 16, GenBank Accession No. AAF97728 and SEQ ID NO: 18, GenBank Accession No. AAD53890); two homologues from *Mesembryanthemum crystallinum* (SEQ ID NO: 14, GenBank Accession No. AAK52962 and SEQ ID NO: 12, GenBank Accession No. AAO73474). Other homologues suitable for practising the method according to the invention include the rice homologues represented in GenBank Accessions AAG37274 (OsHKT1, SEQ ID NO: 20), BAB61791 (OsHKT2, SEQ ID NO: 22), CAD37187 (OsHKT3, SEQ ID NO: 24), CAD37183 (OsHKT4, SEQ ID NO: 26), CAD37185 (OsHKT6, SEQ ID NO: 28), CAD37197 (OsHKT7, SEQ ID NO: 30), BAB93392 (OsHKT8, SEQ ID NO: 32) and CAD37199 (OsHKT9, SEQ ID NO: 34), and a wheat homologue (GenBank Accession No. AAA52749, SEQ ID NO: 36). It may however be envisaged that HKT homologues with a sequence identity lower than 35% to SEQ ID NO: 2 may still be suitable in the methods of the present invention, examples of such proteins are a *Suaeda maritima* homologue (SEQ ID NO: 38, GenBank Accession No. AY530754), or non-plant homologues such as yeast TRK1 (SEQ ID NO: 40, GenBank Accession No. NP_012406) or TRK2 (SEQ ID NO: 42, GenBank Accession No. CAA82128) or *Sacharomyces uvarum* TRK1 (GenBank Accession No. JU0466 or AAA34661).

Table 1 lists examples of HKT proteins and homologues from other organisms, the length of the protein and their sequence identity to SEQ ID NO: 2.

**Table 1**

| | Protein sequence | Length aa | % identity to AAF68393-AtHKT1 |
|---|---|---|---|
| SEQ ID NO: 2 | AAF68393-AtHKT1 | 506 | 100.0 |
| SEQ ID NO: 12 | AAO73474-McHKT2 | 543 | 43.2 |
| SEQ ID NO: 14 | AAK52962-McHKT1 | 505 | 43.0 |
| SEQ ID NO: 16 | AAF97728-EcHKT1 | 550 | 46.0 |
| SEQ ID NO: 18 | AAD53890-EcHKT2 | 549 | 45.5 |
| SEQ ID NO: 36 | AAA52749-TaHKT1 | 533 | 36.9 |
| SEQ ID NO: 20 | AAG37274-OsHKT1 | 530 | 36.9 |
| SEQ ID NO: 22 | BAB61791-OsHKT2 | 530 | 37.0 |
| SEQ ID NO: 24 | CAD37187-OsHKT3 | 509 | 35.1 |
| SEQ ID NO: 26 | CAD37183-OsHKT4 | 552 | 37.1 |
| SEQ ID NO: 28 | CAD37185-OsHKT6 | 531 | 43.1 |
| SEQ ID NO: 30 | CAD37197-OsHKT7 | 500 | 42.2 |
| SEQ ID NO: 32 | BAB93392-OsHKT8 | 554 | 42.0 |
| SEQ ID NO: 34 | CAD37199-OsHKT9 | 509 | 35.1 |
| SEQ ID NO: 38 | AY530754-SmHKT1 | 485 | 33.5 |
| SEQ ID NO: 40 | NP_012406-ScTRK1 | 1235 | 11.3 |
| SEQ ID NO: 42 | CAA82128-ScTRK2 | 889 | 13.9 |
| SEQ ID NO: 44 | JU0466-SuTRK1 | 1241 | 9.7 |

At, *Arabidopsis thaliana*; Mc, *Mesembryanthemum crystallinum*; Ec, *Eucalyptus camaldulensis*; Ta, *Triticum aestivum*; Os, *Oryza sativa*; Sc, *Saccharomyces cerevisiae*; Sm, *Suaeda maritime*; Su, *Saccharomyces uvarum*

Despite what may appear to be a relatively low sequence homology to SEQ ID NO: 2 (as low as approximately 35%), HKT proteins are highly conserved in structure, with full-length proteins having four "transmembrane domain - pore domain - transmembrane domain" (MPM) units and preferably comprise in the fourth pore forming domain a consensus sequence corresponding to **E**VI**SA**Y**G**NV**G**FTT**GY** (SEQ ID NO: 8) wherein the residues indicated in bold are invariably conserved and wherein the other residues may vary. Preferably, the consensus sequence in the fourth pore-forming domain corresponds to SEQ ID NO: 9. More preferably, the consensus sequence in the fourth pore-forming domain corresponds to E(V,I)(I,V)SA(Y,F)GNVG(F,L,Y)(T,S)(T,L,V)GY. Alternatively, the HKT protein useful in the present invention comprises in the fourth pore-forming domain an SA(Y,F)GN sequence and a DP(I,L)N(Y,F,L) sequence. Furthermore, when HKT sequences from different sources are compared, conserved amino acids may be identified throughout the sequence (Figure 2, which does not represent a limiting list of HKT proteins) and sequence identity within the fourth MPM unit among different species can be as high as 70%. *HKT* genes may therefore easily be found in other plant species. It is therefore to be understood that the term "HKT polypeptide or a homologue thereof" is not limited to the sequences represented by SEQ ID NO: 2 nor to SEQ ID NO: 12 to 36 listed above, but that any polypeptide meeting the criteria of having HKT activity and having an HKT topology as outlined above and having at least 35% sequence identity to SEQ ID NO: 2 may be suitable for use in the methods of the invention. As pointed out above, it may however be envisaged that proteins having the HKT topology outlined above but having a sequence identity lower than 35% to SEQ ID NO: 2 may still be useful in the methods of the present invention.

The nucleic acid encoding an HKT polypeptide or a homologue thereof may be any natural or synthetic nucleic acid. An HKT polypeptide or a homologue thereof as defined hereinabove is encoded by an HKT nucleic acid/gene. Therefore the term "*HKT* nucleic acid/gene" as defined herein is any nucleic acid/gene encoding an HKT polypeptide or a homologue thereof as defined hereinabove. Examples of *HKT* nucleic acids include those encoding SEQ ID NO: 2 and the sequence represented by SEQ ID NO: 1, or nucleic acids encoding proteins represented by SEQ ID NO: 12 to 36 (with the GenBank accession numbers AAF97728, AAD53890, AAK52962, AA073474, AAG37274, BAB61791, CAD37187, CAD37183, CAD37185, CAD37197, BAB93392, CAD37199, or AAA52749. However nucleic acids encoding proteins having the HKT topology outlined above but having a sequence identity lower than 35% to SEQ ID NO: 2 may also be useful in the methods of the present invention; examples include SEQ ID NO: 38 to 44. *HKT* nucleic acids/genes and functional variants thereof may be suitable in practising the methods of the invention. Functional variant HKT nucleic acid/genes include portions of an *HKT* nucleic acid/gene and/or nucleic adds capable of hybridising with an *HKT* nucleic acid/gene. The term "functional" in the context of a functional variant refers to a variant (i.e. a portion or a hybridising sequence) which encodes a polypeptide having HKT activity and which polypeptide comprises four units of a transmembrane domain - pore forming domain - transmembrane domain, wherein the fourth pore forming domain preferably comprises a consensus sequence corresponding to **E**VI**SA**Y**G**NV**G**FTT**GY** (SEQ ID NO: 8) wherein the residues indicated in bold are invariably conserved and wherein the other residues may vary. Preferably, the consensus sequence in the fourth pore-forming domain corresponds to SEQ ID NO: 9. More preferably, the consensus sequence in the fourth pore-forming domain corresponds to E(V,I)(I,V)SA(Y,F)GNVG(F,L,Y)(T,S)(T,L,V)GY. Alternatively, the variant HKT protein may comprise in the fourth pore-forming domain an SA(Y,F)GN sequence and a DP(I,L)N(Y,F,L) sequence.

The term portion as defined herein refers to a piece of DNA comprising at least 80 nucleotides or more, preferably at least 330 nucleotides, which portion encodes a polypeptide having HKT activity and which polypeptide comprises at least one unit of a transmembrane domain - pore forming domain - transmembrane domain. A portion may be prepared, for example, by making one or more deletions to an *HKT* nucleic acid. The portions may be used in isolated form or they may be fused to other coding (or non coding) sequences in order to, for example, produce a protein that combines several activities, one of them being HKT activity, such as for example cation transporter activity. When fused to other coding sequences, the resulting polypeptide produced upon translation could be bigger than that predicted for the *HKT* fragment Preferably, the functional portion is a portion of a nucleic acid as represented by SEQ ID NO: 1, or a portion of nucleic acids encoding proteins as defined above, and which portion encodes a polypeptide having HKT activity.

Another type of variant *HKT* is a nucleic acid capable of hybridising under stringent conditions with an *HKT* nucleic acid/gene having SEQ ID NO:1 as hereinbefore defined, which hybridising sequence encodes a polypeptide having HKT activity and which polypeptide comprises at least one unit of a transmembrane domain - pore forming domain - transmembrane domain. The hybridising sequence is preferably at least 80 nucleotides in length, more preferably at least 330 nucleotides in length.

The term "hybridisation" as defined herein is a process wherein substantially homologous complementary nucleotide sequences anneal to each other, such that the sense strand of one species will anneal to the antisense strand of the other species. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids. The stringency of hybridisation is influenced by conditions such as temperature, salt concentration, ionic strength and hybridisation buffer composition.

"Stringent hybridisation conditions" and "stringent hybridisation wash conditions" in the context of nucleic acid hybridisation experiments such as Southern and Northern hybridisations are sequence dependent and are different under different environmental parameters. The skilled artisan is aware of various parameters which may be altered during hybridisation and washing and which will either maintain or change the stringency conditions.

The Tₘ is the temperature under defined ionic strength and pH, at which 50% of the target sequence hybridises to a perfectly matched probe. The Tₘ is dependent upon the solution conditions and the base composition and length of the probe. For example, longer sequences hybridise specifically at higher temperatures. The maximum rate of hybridisation is obtained from about 16°C up to 32°C below Tₘ. The presence of monovalent cations in the hybridisation solution reduce the electrostatic repulsion between the two nucleic acid strands thereby promoting hybrid formation; this effect is visible for sodium concentrations of up to 0.4M. Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tₘ decreases about 1°C per % base mismatch. The Tₘ may be calculated using the following equations, depending on the types of hybrids:
• DNA-DNA hybrids (Meinkoth and Wahl, Anal. Biochem., 138: 26T-284, 1984):
Tₘ= 81.5°C + 16.6xlog[Na⁺]^{a} + 0.41x%[G/C^{b}] - 500x[L^{c}]⁻¹ - 0.61x% formamide

• DNA-RNA or RNA-RNA hybrids:
Tₘ= T9.8 + 18.5 (log₁₀[Na⁺]^{a}) + 0.58 (%G/C^{b}) + 11.8 (%G/C^{b})² - 820/L^{c}

• oligo-DNA or oligo-RNA^{d} hybrids:

| | |
|---|---|
| For <20 nucleotides: | Tₘ= 2 (*I*ₙ) |
| For 20-35 nucleotides: | Tₘ= 22 + 1.46 (*I*ₙ) |

*^{a}* or for other monovalent cation, but only accurate in the 0.01-0.4 M range.
*^{b}* only accurate for %GC in the 30% to 75% range.
*^{c}* L = length of duplex in base pairs.
*^{d}* Oligo, oligonucleotide; *I*ₙ, effective length of primer = 2×(no. of G/C)+(no. of A/T).

*Note:* for each 1 % formamide, the Tₘ is reduced by about 0.6 to 0.7°C, while the presence of 6M urea reduces the Tₘ by about 30°C

Specificity of hybridisation is typically the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. Generally, suitable stringent conditions for nucleic acid hybridisation assays or gene amplification detection procedures are as set forth above. More or less stringent conditions may also be selected. Generally, low stringency conditions are selected to be about 50°C lower than the thermal melting point (Tₘ) for the specific sequence at a defined ionic strength and pH. Medium stringency conditions are when the temperature is 20°C below Tₘ, and high stringency conditions are when the temperature is 10°C below Tₘ. For example, stringent conditions are those that are at least as stringent as, for example, conditions A-L; and reduced stringency conditions are at least as stringent as, for example, conditions M-R. Non-specific binding may be controlled using any one of a number of known techniques such as, for example, blocking the membrane with protein containing solutions, additions of heterologous RNA, DNA, and SDS to the hybridisation buffer, and treatment with Rnase.

Examples of hybridisation and wash conditions are listed in table 2:

**Table 2:**

| Stringency Condition | Polynucleotide Hybrid ^{±} | Hybrid Length (bp)^{‡} | Hybridization Temperature and Buffer ^{†} | Wash Temperature and Buffer ^{†} |
|---|---|---|---|---|
| A | DNA:DNA | > or equal to 50 | 65°C 1×SSC; or 42°C, 1×SSC and 50% formamide | 65°C; 0.3×SSC |
| B | DNA:DNA | <50 | Tb*; 1×SSC | Tb*; 1×SSC |
| C | DNA:RNA | > or equal to 50 | 67°C 1×SSC; or 45°C, 1×SSC and 50% formamide | 67°C; 0.3×SSC |
| D | DNA:RNA | <50 | Td*; 1×SSC | Td*; 1×SSC |
| E | RNA:RNA | > or equal to 50 | 70°C 1×SSC; or 50°C, 1×SSC and 50% formamide | 70°C; 0.3×SSC |
| F | RNA:RNA | <50 | Tf*; 1×SSC | Tf*; 1×SSC |
| G | DNA:DNA | > or equal to 50 | 65°C 4×SSC; or 45°C, 4×SSC and 50% formamide | 65°C; 1×SSC |
| H | DNA:DNA | <50 | Th*; 4×SSC | Th*; 4×SSC |
| I | DNA:RNA | > or equal to 50 | 67°C 4×SSC; or 45°C, 4×SSC and 50% formamide | 67°C; 1×SSC |
| J | DNA:RNA | <50 | Tj*; 4×SSC | Tj*; 4×SSC |
| K | RNA:RNA | > or equal to | 70°C 4×SSC; or 40°C, 6×SSC 50 and 50% formamide | 67°C; 1×SSC |
| L | RNA:RNA | <50 | TI*; 2×SSC | TI*; 2×SSC |
| M | DNA:DNA | > or equal to 50 | 50°C 4×SSC; or 40°C, 6×SSC and 50% formamide | 50°C; 2×SSC |
| N | DNA:DNA | <50 | Tn*; 6×SSC | Tn*; 6×SSC |
| O | DNA:RNA | > or equal to 50 | 55°C 4×SSC; or 42°C, 6×SSC and 50% formamide | 55°C; 2×SSC |
| P | DNA:RNA | <50 | Tp*; 6×SSC | Tp*; 6×SSC |
| Q | RNA:RNA | > or equal to 50 | 60°C 4×SSC; or 45°C. 6×SSC and 50% formamide | 60°C.; 2×SSC |
| R | RNA:RNA | <50 | Tr*; 4 ×SSC | Tr*; 4×SSC |

| | | | | |
|---|---|---|---|---|
| ^{‡} The "hybrid length" is the anticipated length for the hybridising nucleic acid. When ucleic acids of known sequence are hybridised, the hybrid length may be determined by aligning the sequences and identifying the conserved regions described herein. ^{†} SSPE (1×SSPE is 0.15M NaCl, 10mM NaH₂PO₄, and 1.25mM EDTA, pH7.4) may be substituted for SSC (1×SSC is 0.15M NaCl and 15mM sodium citrate) in the hybridisation and wash buffers; washes are performed for 15 minutes after hybridisation is complete. The hybridisations and washes may additionally include 5 × Denhardt's reagent, 0.5-1.0% SDS, 100 µg/ml denatured, fragmented salmon sperm DNA, 0.5% sodium pyrophosphate, and up to 50% formamide. * Tb-Tr: The hybridisation temperature for hybrids anticipated to be less than 50 base pairs in length should be 5-10°C less than the melting temperature Tₘ of the hybrids; the Tₘ is determined according to the above-mentioned equations. ^{±} The present invention also encompasses the substitution of any one, or more DNA or RNA hybrid partners with either a PNA, or a modified nucleic acid. | | | | |

For the purposes of defining the level of stringency, reference can conveniently be made to Sambrook et al. (2001) Molecular Cloning: a laboratory manual, 3rd Edition Cold Spring Harbor Laboratory Press, CSH, New York or to Current Protocols in Molecular Biology, John Wiley & Sons, N.Y. (1989).

For example, a nucleic acid encoding SEQ ID NO: 2 or a homologue thereof may be used in a hybridisation experiment. Alternatively fragments thereof may be used as probes. Depending on the starting pool of sequences from which the HKT protein is to be identified, different fragments for hybridization may be selected. For example, when a limited number of homologues with a high sequence identity to HKT are desired, a less conserved fragment may be used for hybridisation. By aligning SEQ ID NO: 2 and homologues thereof, it is possible to design equivalent nucleic acid fragments useful as probes for hybridisation.

After hybridisation and washing, the duplexes may be detected by autoradiography (where radiolabeled probes are used) or by chemiluminescence, immunodetection, by fluorescent or chromogenic detection, depending on the type of probe labelling. Alternatively, a ribonuclease protection assay may be performed for detection of RNA: RNA hybrids

The *HKT* nucleic acid or variant thereof may be derived from any natural or artificial source. The nucleic acid/gene or variant thereof may be isolated from a microbial source, such as bacteria, yeast or fungi, or from a plant, algae or animal (including human) source. This nucleic acid may be modified from its native form in composition and/or genomic environment through deliberate human manipulation. The nucleic acid is preferably of plant origin, whether from the same plant species (for example to the one in which it is to be introduced) or whether from a different plant species. The nucleic acid may be isolated from a dicotyledonous species, preferably from the family Brassicaceae, further preferably from *Arabidopsis thaliana.* More preferably, the *HKT* isolated from *Arabidopsis thaliana* is represented by SEQ ID NO: 1 and the HKT amino acid sequence is as represented by SEQ ID NO: 2.

The activity of an HKT polypeptide or a homologue thereof may be increased by introducing a genetic modification (preferably in the locus of an *HKT* gene). The locus of a gene as defined herein is taken to mean a genomic region which includes the gene of interest and 10 kB up- or downstream of the coding region.

The genetic modification may be introduced, for example, by any one (or more) of the following methods: TDNA activation, TILLING, site-directed mutagenesis, homologous recombination, directed evolution or by introducing and expressing in a plant a nucleic acid encoding an HKT polypeptide or a homologue thereof, provided that each of the methods requires human intervention. Following introduction of the genetic modification there follows a step of selecting for increased activity of an HKT polypeptide, which increase in activity gives plants having improved growth characteristics.

T-DNA activation tagging (Hayashi *et al*. Science (1992) 1350-1353) involves insertion of T-DNA usually containing a promoter (may also be a translation enhancer or an intron), in the genomic region of the gene of interest or 10 kB up- or downstream of the coding region of a gene in a configuration such that such promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to overexpression of genes near to the inserted T-DNA. The resulting transgenic plants show dominant phenotypes due to overexpression of genes dose to the introduced promoter. The promoter to be introduced may be any promoter capable of directing expression of a gene in the desired organism, in this case a plant. For example, constitutive, tissue-specific, cell type-specific and inducible promoters are all suitable for use in T-DNA activation.

A genetic modification may also be introduced in the locus of an *HKT* gene using the technique of TILLING (Targeted Induced Local Lesions IN Genomes). This is a mutagenesis technology useful to generate and/or identify, and to eventually isolate mutagenised variants of an *HKT* nucleic acid capable of exhibiting HKT activity. TILLING also allows selection of plants carrying such mutant variants. These mutant variants may even exhibit higher HKT activity than that exhibited by the gene in its natural form. TILLING combines high-density mutagenesis with high-throughput screening methods. The steps typically followed in TILLING are: (a) EMS mutagenesis (Redei and Koncz (1992), In: C Koncz, N-H Chua, J Schell, eds, Methods in Arabidopsis Research. World Scientific, Singapore, pp 16-82; Feldmann et al., (1994) In: EM Meyerowitz, CR Somerville, eds, Arabidopsis. Cold Spring Harbor Laboratory Press, Cold Spring Harbor, NY, pp 137-172; Lightner and Caspar (1998), In: J Martinez-Zapater, J. Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum, Nat Biotechnol. 2000 Apr; 18(4):455-7, Stemple, Nature Rev. Genet 5, 145-150, 2004).

Site directed mutagenesis may be used to generated variants of *HKT* nucleic acids or portions thereof that retain HKT activity, for example cation transporter activity. Several methods are available to achieve site directed mutagenesis, the most common being PCR-based methods (See for example Ausubel et al., Current Protocols in Molecular Biology. Wiley Eds. http://www.4ulr.com/products/currentprotocols/index.html).

Directed evolution may be used to generate functional variants of *HKT* nucleic acid molecules encoding HKT polypeptides or homologues, or portions thereof having an increased biological activity as outlined above. Directed evolution consists of iterations of DNA shuffling followed by appropriate screening and/or selection (Castle et al., (2004) Science 304(5674): 1151-4; US patents 5,811,238 and 6,395,547).

TDNA activation, TILLING, site-directed mutagenesis and directed evolution are examples of technologies that enable the generation novel alleles and variants of *HKT* that retain HKT function and which are therefore useful in the methods of the invention.

Homologous recombination allows introduction in a genome of a selected nucleic acid at a defined selected position. Homologous recombination is a standard technology used routinely in biological sciences for lower organism such as yeast or the moss *Physcomitrella*. Methods for performing homologous recombination in plants have been described not only for model plants (Offringa et al. (1990) EMBO J. 9, 3077-3084) but also for crop plants, for example rice (Terada et al., (2002) Nature Biotechnol. 20, 1030-1034; or lida and Terada (2004) Curr. Opin. Biotechnol. 15, 132-138). The nucleic acid to be targeted (which may be an *HKT* nucleic acid molecule or variant thereof as hereinbefore defined) need not be targeted to the locus of an *HKT* gene, but may be introduced in, for example, regions of high expression. The nucleic acid to be targeted may be an improved allele used to replace the endogenous gene or may be introduced in addition to the endogenous gene.

According to a preferred embodiment of the invention, plant growth characteristics may be improved by introducing and expressing in a plant an isolated nucleic acid encoding an HKT polypeptide or a homologue thereof.

A preferred method for introducing a genetic modification (which in this case need not be in the locus of an *HKT* gene) is to introduce and express in a plant a nucleic acid encoding an HKT polypeptide or a homologue thereof. An HKT polypeptide or a homologue thereof as mentioned above is a polypeptide having HKT activity and four units of a transmembrane domain - pore forming domain - transmembrane domain. The HKT polypeptide has, in increasing order of preference, at least 35%, 36%, 37%, 38%, 39%, 40%, 41%, 42%, 43%, 44%, 45%, 46%, 47%, 48%, 49%, 50%, 55%, 60%, 65%, 70%, 75%, 80%, 85%, 90%, 95% sequence identity to the amino acid sequence represented by SEQ ID NO: 2, and has cation transporter activity or is as represented in SEQ ID NO: 2.

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

Also encompassed by the term "homologues" are two special forms of homology, which include orthologous sequences and paralogous sequences, which encompass evolutionary concepts used to describe ancestral relationships of genes. The term "paralogous" relates to gene-duplications within the genome of a species leading to paralogous genes. The term "orthologous" relates to homologous genes in different organisms due to speciation. Orthologues in, for example, monocot plant species may easily be found by performing a so-called reciprocal blast search. This may be done by a first blast involving blasting the sequence in question (for example, SEQ ID NO 1 or SEQ ID NO 2, being from *Arabidopsis thaliana)* against any sequence database, such as the publicly available NCBI database which may be found at: http://www.ncbi.nlm.nih.gov. If orthologues in rice were sought, the sequence in question would be blasted against, for example, the 28,469 full-length cDNA clones from *Oryza sativa* Nipponbare available at NCBI. BLASTn or tBLASTX may be used when starting from nucleotides or BLASTP or TBLASTN when starting from the protein, with standard default values. The blast results may be filtered. The full-length sequences of either the filtered results or the non-filtered results are then blasted back (second blast) against the sequences of the organism from which the sequence in question is derived, *in casu Arabidopsis thaliana.* The results of the first and second blasts are then compared. An orthologue is found when the results of the second blast give as hits with the highest similarity a query *HKT* nucleic acid or HKT polypeptide. If one of the hits in the first BLAST is from the same organism, then a paralogue has been found. Such paralogue is also considered a homologue of HKT, provided that this homologue has HKT activity and comprises four units of a transmembrane domain - pore forming domain - transmembrane domain and preferably also comprises the conserved sequences defined above. In the case of large families, ClustalW may be used, followed by the construction of a neighbour joining tree, to help visualize the clustering.

A homologue may be in the form of a "substitutionat variant" of a protein, i.e. where at least one residue in an amino acid sequence has been removed and a different residue inserted in its place. Amino acid substitutions are typically of single residues, but may be clustered depending upon functional constraints placed upon the polypeptide; insertions will usually be of the order of about 1 to 10 amino acid residues. Preferably, amino acid substitutions comprise conservative amino acid substitutions (Table 3). To produce such homologues, amino acids of the protein may be replaced by other amino acids having similar properties (such as similar hydrophobicity, hydrophilicity, antigenicity, propensity to form or break α-helical structures or β-sheet structures). Conservative substitution tables are well known in the art (see for example Creighton (1984) Proteins. W.H. Freeman and Company).

**Table 3: Examples of conserved amino acid substitutions:**

| **Residue** | **Conservative Substitutions** | **Residue** | **Conservative Substitutions** |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Less conserved substitutions may be made in case the above-mentioned amino acid properties are not so critical.

A homologue may also be in the form of an "insertional variant" of a protein, i.e. where one or more amino acid residues are introduced into a predetermined site in a protein. Insertions may comprise amino-terminal and/or carboxy-terminal fusions as well as intra-sequence insertions of single or multiple amino acids. Generally, insertions within the amino acid sequence will be smaller than amino- or carboxy-terminal fusions, of the order of about 1 to 10 residues. Examples of amino- or carboxy-terminal fusion proteins or peptides include the binding domain or activation domain of a transcriptional activator as used in the yeast two-hybrid system, phage coat proteins, (histidine)6-tag, glutathione S-transferase-tag, protein A, maltose-binding protein, dihydrofolate reductase, Tag 100 epitope, c-myc epitope, **FLAG^{®}-epitope,** lacZ, CMP (calmodulin-binding peptide), HA epitope, protein C epitope and VSV epitope.

Homologues in the form of "deletion variants" of a protein are characterised by the removal of one or more amino acids from a protein.

Amino acid variants of a protein may readily be made using peptide synthetic techniques well known in the art, such as solid phase peptide synthesis and the like, or by recombinant DNA manipulations. Methods for the manipulation of DNA sequences to produce substitution, insertion or deletion variants of a protein are well known in the art. For example, techniques for making mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

The HKT polypeptide or homologue thereof may be a derivative. "Derivatives" include peptides, oligopeptides, polypeptides, proteins and enzymes which may comprise substitutions, deletions or additions of naturally and non-naturally occurring amino acid residues compared to the amino acid sequence of a naturally-occurring form of the protein, for example, as presented in SEQ ID NO 2. "Derivatives" of a protein encompass peptides, oligopeptides, polypeptides, proteins and enzymes which may comprise naturally occurring altered, glycosylated, acylated or non-naturally occurring amino acid residues compared to the amino acid sequence of a naturally-occurring form of the polypeptide. A derivative may also comprise one or more non-amino acid substituents compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein.

The HKT polypeptide or homologue thereof may be encoded by an alternative splice variant of an *HKT* nucleic acid molecule or gene. The term "alternative splice variant" as used herein encompasses variants of a nucleic acid sequence in which selected introns and/or exons have been excised, replaced or added. Such variants will be ones in which the biological activity of the protein is retained, which may be achieved by selectively retaining functional segments of the protein. Such splice variants may be found in nature or may be manmade. Methods for making such splice variants are well known in the art. Preferred splice variants are splice variants derived from the nucleic acid represented by SEQ ID NO: 3. Further preferred are splice variants encoding a polypeptide having HKT activity and comprising at least one, and preferably four unit(s) of a transmembrane domain - pore forming domain - transmembrane domain. A preferred splice variant is represented by SEQ ID NO: 1.

The homologue may also be encoded by an allelic variant of a nucleic acid encoding an HKT polypeptide or a homologue thereof, preferably an allelic variant of the nucleic acid represented by SEQ ID NO 1. Further preferably, the polypeptide encoded by the allelic variant has HKT activity and comprises at least one, and preferably four unit(s) of a transmembrane domain - pore forming domain - transmembrane domain. The homologue may also be encoded by an allelic variant of a nucleic acid represented by SEQ ID NO: 11, 13, 15, 17, 19, 21, 23, 25, 27, 29, 35 or 37. Allelic variants exist in nature and encompassed within the methods of the present invention is the use of these natural alleles. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

According to a preferred aspect of the present invention, enhanced or increased expression of the *HKT* nucleic acid molecule or variant thereof is envisaged. Methods for obtaining enhanced or increased expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of an *HKT* nucleic acid or variant thereof. For example, endogenous promoters may be altered *in vivo* by mutation, deletion, and/or substitution (see Kmiec, U.S. Pat No. 5,565,350; Zarling et al., PCT/US93/03868), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg, Mol. Cell Biol. 8,4395-4405 (1988); Callis et al., Genes Dev. 1, 1183-1200 (1987)). Such intron enhancement of gene expression is typically greatest when placed near the 5' end of the transcription unit Use of the maize introns Adh1-S intron 1, 2, and 6, the Bronze-1 intron are known in the art. See generally, The Maize Handbook, Chapter 116, Freeling and Walbot, Eds., Springer, N.Y. (1994).

The disclosure also provides genetic constructs and vectors to facilitate introduction and/or expression of the nucleotide sequences useful in the methods according to the invention.

Therefore, there is provided a gene construct comprising:
(i) an isolated *HKT* nucleic acid molecule or functional variant thereof;
(ii) one or more control sequence(s) capable of driving expression of the nucleic add sequence of (i); and optionally
(iii) a transcription termination sequence.

Preferably, the control sequence used in the gene construct is a seed-specific control sequence.

Constructs useful in the methods according to the present invention may be constructed using recombinant DNA technology well known to persons skilled in the art. The gene constructs may be inserted into vectors, which may be commercially available, suitable for transforming into plants and suitable for expression of the gene of interest in the transformed cells.

Plants are transformed with a vector comprising the sequence of interest (i.e., an *HKT* nucleic acid or variant thereof). The sequence of interest is operably linked to one or more control sequences (at least to a promoter). The terms "regulatory element", "control sequence" and "promoter" are all used interchangeably herein and are to be taken in a broad context to refer to regulatory nucleic acid sequences capable of effecting expression of the sequences to which they are ligated. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli (in case of an inducible promoter), or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative which confers, activates or enhances expression of a nucleic acid molecule in a cell, tissue or organ. The term "operably linked" as used herein refers to a functional linkage between the promoter sequence and the gene of interest, such that the promoter sequence is able to initiate transcription of the gene of interest.

Advantageously, any type of promoter may be used to drive expression of the nucleic acid sequence. The promoter may be an inducible promoter, i.e. having induced or increased transcription initiation in response to a developmental, chemical, environmental or physical stimulus. An example of such a promoter is a stress-inducible promoter. Additionally or alternatively, the promoter may be a constitutive promoter. The term "constitutive" as defined herein refers to a promoter that is expressed predominantly in at least one tissue or organ and predominantly at any life stage of the plant. Preferably the constitutive promoter is expressed predominantly throughout the plant. Additionally or alternatively, the promoter may be a tissue-specific promoter, i.e. one that is capable of preferentially initiating transcription in certain tissues, such as the leaves, roots, seed tissue etc.

Preferably, the isolated *HKT* nucleic acid or variant thereof is operably linked to a seed-specific promoter. Further preferably, the seed-specific promoter is embryo- and aleurone-specfic and mainly active during the late embryogenic stages. The term "seed-specific" as defined herein refers to a promoter that is expressed predominantly in the seeds of the plant. Preferably the promoter is expressed predominantly in the embryo and/or aleurone layer. More preferably, the seed-specific promoter has a comparable expression profile to the WSI18 promoter. Most preferably, the seed-specific promoter is the WSI18 promoter from rice (WO2004/070039), as given in SEQ ID NO: 45 (corresponding to nucleotides 1 to 1828 of SEQ ID NO: 4). The WSI18 promoter from rice is responsive to abscisic acid (ABA) and highly induced upon conditions that involve ABA, such as seed desiccation. Therefore, a preferred promoter for use in the present invention may also be any other promoter induced by ABA and/or by stress conditions such as drought. It should be dear however that the applicability of the present invention is not restricted to the *HKT* nucleic add represented by SEQ ID NO: 1, nor is the applicability of the invention restricted to expression of an *HKT* nucleic acid when driven by a WSI18 promoter. Examples of other seed-specific promoters that may also be used to drive expression of an *HKT* nucleic acid are listed in Table 4.

**Table 4: Examples of seed-specific promoters for use in the performance of the present invention:**

| GENE SOURCE | EXPRESSION PATTERN | REFERENCE |
|---|---|---|
| seed-specific genes | seed | Simon, et al., Plant Mol. Biol. 5: 191, 1985; Scofield, et al., J. Biol. Chem. 262: 12202, 1987.; Baszczynski, et al., Plant Mol. Biol. 14: 633, 1990. |
| Brazil Nut albumin | seed | Pearson, et al., Plant Mol. Biol. 18: 235-245, 1992. |
| legumin | seed | Ellis, et al., Plant Mol. Biol. 10: 203-214, 1988. |
| glutelin (rice) | seed | Takaiwa, et al., Mol. Gen. Genet. 208: 15-22, 1986; Takaiwa, et al., FEBS Letts. 221: 43-47, 1987. |
| zein | seed | Matzke et al Plant Mol Biol, 14(3):323-321990 |
| napA | seed | Stalberg, et al, Planta 199: 515-519, 1996. |
| wheat LMW and HMW glutenin-1 | endosperm | Mol Gen Genet 216:81-90, 1989; NAR 17:461-2, 1989 |
| wheat SPA | seed | Albani et al, Plant Cell, 9: 171-184, 1997 |
| wheat α, β, γ-gliadins | endosperm | EMBO J. 3:1409-15, 1984 |
| barley *Itr1* promoter | endosperm | |
| barley B1, C, D, hordein | endosperm | Theor Appl Gen 98:1253-62, 1999; Plant J 4:343-55, 1993; Mol Gen Genet 250:750-60, 1996 |
| barley DOF | endosperm | Mena et al, The Plant Journal, 116(1): 53-62, 1998 |
| *blz2* | endosperm | EP99106056.7 |
| synthetic promoter | endosperm | Vicente-Carbajosa et al., Plant J. 13: 629-640, 1998. |
| rice prolamin NRP33 | endosperm | Wu et al, Plant Cell Physiology 39(8) 885-889, 1998 |
| rice α-globulin Glb-1 | endosperm | Wu et al, Plant Cell Physiology 39(8) 885-889, 1998 |
| rice OSH1 | embryo | Sato et al, Proc. Natl. Acad. Sci. USA, 93: 8117-8122, 1996 |
| rice α-globulin REB/OHP-1 | endosperm | Nakase *et al*. Plant Mol. Biol. 33: 513-522, 1997 |
| rice ADP-glucose PP | endosperm | Trans Res 6:157-68, 1997 |
| maize ESR gene family | endosperm | Plant J 12:235-46, 1997 |
| sorgum γ-kafirin | endosperm | PMB 32:1029-35, 1996 |
| KNOX | embryo | Postma-Haarsma et al, Plant Mol. Biol. 39:257-71, 1999 |
| rice oleosin | embryo and aleuron | Wu et at, J. Biochem., 123:386, 1998 |
| sunflower oleosin | seed (embryo and dry seed) | Cummins, et al., Plant Mol. Biol. 19: 873-876, 1992 |
| PRO0117, putative rice 40S ribosomal protein | weak in endosperm | WO2004/070039 |
| PRO0135, rice alpha-globulin | strong in endosperm | |
| PRO0136, rice alanine aminotransferase | weak in endosperm | |
| PRO0147, trypsin inhibitor ITR1 (barley) | weak in endosperm | |
| PRO0175, rice RAB21 | embryo + stress | WO2004/070039 |
| PRO0218, rice oleosin 18kd | aleurone + embryo | |

Optionally, one or more terminator sequences may also be used in the construct introduced into a plant. The term "terminator" encompasses a control sequence which is a DNA sequence at the end of a transcriptional unit which signals 3' processing and polyadenylation of a primary transcript and termination of transcription. Additional regulatory elements may include transcriptional as well as translational enhancers. Those skilled in the art will be aware of terminator and enhancer sequences which may be suitable for use in performing the invention. Such sequences would be known or may readily be obtained by a person skilled in the art.

The genetic constructs may further include an origin of replication sequence, which is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

The genetic construct may optionally comprise a selectable marker gene. As used herein, the term "selectable marker gene" includes any gene which confers a phenotype on a cell in which it is expressed to facilitate the identification and/or selection of cells which are transfected or transformed with a nucleic acid construct disclosed herein. Suitable markers may be selected from markers that confer antibiotic or herbicide resistance, that introduce a new metabolic trait or that allow visual selection. Examples of selectable marker genes include genes conferring resistance to antibiotics (such as *npt*II that phosphorylates neomycin and kanamycin, or *hpt,* phosphorylating hygromycin), to herbicides (for example *bar* which provides resistance to Basta; *aro*A or gox providing resistance against glyphosate), or genes that provide a metabolic trait (such as *manA* that allows plants to use mannose as sole carbon source). Visual marker genes result in the formation of colour (for example β-glucuronidase, GUS), luminescence (such as luciferase) or fluorescence (Green Fluorescent Protein, GFP, and derivatives thereof).

In a preferred embodiment, the genetic construct as mentioned above, comprises an *HKT* nucleic acid in sense orientation coupled to a promoter that is preferably a seed-specific promoter, such as for example the rice WSI18 promoter. Therefore, another aspect of the present disclosure is a vector construct carrying an expression cassette essentially similar to SEQ ID NO 4, comprising a WSI18 promoter, the rice *HKT* gene and the T-zein + T-rubisco deltaGA transcription terminator sequence. A sequence essentially similar to SEQ ID NO 4 encompasses a first nucleic acid sequence encoding a protein homologous to SEQ ID NO 2 or hybridising to SEQ ID NO 1, which first nucleic acid is operably linked to a WSI18 promoter or a promoter with a similar expression pattern, additionally or alternatively the first nucleic acid is linked to a transcription termination sequence.

The present disclosure also encompasses plants or parts (including plant cells) thereof obtainable by the methods according to the present invention. The present disclsoure therefore provides plants or parts thereof (including plant cells) obtainable by the method according to the present invention, which plants have introduced therein an isolated *HKT* nucleic acid or variant thereof, or which plants have introduced therein a genetic modification, preferably in the locus of an *HKT* gene.

The invention also provides a method for increasing yield of a plant as described in claim 4.

More specifically, the present invention provides a method for the production of transgenic plants having increased yield as described in claim 10.

The nucleic acid may be introduced directly into a plant cell or into the plant itself (including introduction into a tissue, organ or any other part of a plant). According to a preferred feature of the present invention, the nucleic acid is preferably introduced into a plant by transformation.

The term "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct as disclosed herein and a whole plant regenerated therefrom. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

Transformation of plant species is now a fairly routine technique. Advantageously, any of several transformation methods may be used to introduce the gene of interest into a suitable ancestor cell. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens et al. (1982) Nature 296, 72-74; Negrutiu et al. (1987) Plant Mol. Biol. 8, 363-373); electroporation of protoplasts (Shillito et al. (1985) Bio/Technol 3, 1099-1102); microinjection into plant material (Crossway et al. (1986) Mol. Gen. Genet 202, 179-185); DNA or RNA-coated particle bombardment (Klein et al. (1987) Nature 327, 70) infection with (non-integrative) viruses and the like. Transgenic rice plants expressing an HKT protein are preferably produced via *Agrobacterium*-mediated transformation using any of the well known methods for rice transformation, such as described in any of the following: published European patent application EP 1198985 A1, Aldemita and Hodges (Planta 199, 612-617, 1996); Chan et al. (Plant Mol. Biol. 22, 491-506, 1993), Hiei et al. (Plant J. 6, 271-282, 1994), which disclosures are incorporated by reference herein as if fully set forth. In the case of corn transformation, the preferred method is as described in either Ishida et al. (Nature Biotechnol. 14, 745-50, 1996) or Frame et al. (Plant Physiol. 129, 13-22, 2002), which disclosures are incorporated by reference herein as if fully set forth.

Generally after transformation, plant cells or cell groupings are selected for the presence of one or more markers which are encoded by plant-expressible genes co-transferred with the gene of interest, following which the transformed material is regenerated into a whole plant.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art. The cultivation of transformed plant cells into mature plants may thus encompass steps of selection and/or regeneration and/or growing to maturity.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present disclosure clearly extends to any plant cell or plant produced by any of the methods described herein, and to all plant parts and propagules thereof. The present disclosure extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced in the parent by the methods according to the invention. The disclosure also includes host cells containing an isolated *HKT* nucleic acid or variant thereof. Preferred host cells according to the disclosure are plant cells. The disclosure also extends to harvestable parts of a plant as disclosed herein, such as but not limited to seeds, leaves, fruits, flowers, stems, rhizomes, tubers and bulbs. The disclosure furthermore relates to products directly derived from a harvestable part of such a plant, such as dry pellets or powders, oil, fat and fatty acids, starch or proteins.

The present invention also encompasses the use of the afore mentioned *HKT* nucleic acids or variants thereof and to the use of HKT polypeptides or homologues thereof for increasing yield of plants.

The seed yield may include one or more of the following: increased number of (filled) seeds, increased seed weight, increased harvest index, increased thousand kernel weight, seed filling rate, among others.

The afore mentioned *HKT* nucleic acids or variants thereof or HKT polypeptides or homologues thereof may find use in breeding programmes in which a DNA marker is identified which may be genetically linked to an *HKT* gene or variant thereof. The said *HKT* or variants thereof or HKT or homologues thereof may be used to define a molecular marker for identifying plants having increased yield.

Allelic variants of an *HKT* may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place by, for example, PCR. This is followed by a selection step for selection of superior allelic variants of the sequence in question and which give rise to improved growth characteristics in a plant. Selection is typically carried out by monitoring growth performance of plants containing different allelic variants of the sequence in question, for example, different allelic variants of SEQ ID NO: 1, or of nucleic acids encoding any of the above mentioned plant homologues. Growth performance may be monitored in a greenhouse or in the field. Further optional steps include crossing plants, in which the superior allelic variant was identified, with another plant. This could be used, for example, to make a combination of interesting phenotypic features.

*HKT* nucleic acids or variants thereof as referred to above may also be used as probes for genetically and physically mapping the genes that they are a part of, and as markers for traits linked to those genes. Such information may be useful in plant breeding in order to develop lines with desired phenotypes. Such use of *HKT* nucleic acids or variants thereof requires only a nucleic acid sequence of at least 10 nucleotides in length. The *HKT* nucleic acids or variants thereof may be used as restriction fragment length polymorphism (RFLP) markers. Southern blots of restriction-digested plant genomic DNA may be probed with the *HKT* nucleic acids or variants thereof. The resulting banding patterns may then be subjected to genetic analyses using computer programs such as MapMaker (Lander et al. (1987) Genomics 1, 174-181) in order to construct a genetic map. In addition, the nucleic acids may be used to probe Southern blots containing restriction endonudease-treated genomic DNAs of a set of individuals representing parent and progeny of a defined genetic cross. Segregation of the DNA polymorphisms is noted and used to calculate the position of the *HKT* nucleic acid or variant thereof in the genetic map previously obtained using this population (Botstein et al. (1980) Am. J. Hum. Genet. 32, 314-331).

The production and use of plant gene-derived probes for use in genetic mapping is described in Bematzky and Tanksley (Plant Mol. Biol. Reporter 4, 37-41, 1986). Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Nonmammalian Genomic Analysis: A Practical Guide, Academic press 1996, pp. 319-346, and references cited therein).

The nucleic acid probes may be used in direct fluorescence *in situ* hybridization (FISH) mapping (Trask (1991) Trends Genet. 7, 149-154). Although current methods of FISH mapping favour use of large clones (several to several hundred kb; see Laan et al. (1995) Genome Res. 5, 13-20), improvements in sensitivity may allow performance of FISH mapping using shorter probes.

A variety of nucleic acid amplification-based methods of genetic and physical mapping may be carried out using the nucleic acids. Examples include allele-specific amplification (Kazazian (1989) J. Lab. Clin. Med. 11, 95-96), polymorphism of PCR-amplified fragments (CAPS; Sheffield et al. (1993) Genomics 16, 325-332), allele-specific ligation (Landegren et al. (1988) Science 241, 1077-1080), nucleotide extension reactions (Sokolov (1990) Nucleic Acid Res. 18, 3671), Radiation Hybrid Mapping (Walter et al. (1997) Nat. Genet. 7, 22-28) and Happy Mapping (Dear and Cook (1989) Nucleic Acid Res. 17, 6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

In this way, generation, identification and/or isolation of plants with increased HKT activity displaying increased yield can be performed.

*HKT* nucleic acids or variants thereof or HKT polypeptides or homologues thereof may also find use as growth regulators. Since these molecules have been shown to be useful in improving the growth characteristics of plants, they would also be useful growth regulators, such as herbicides or growth stimulators. The present invention therefore provides a composition comprising an *HKT* or variant thereof or an HKT polypeptide or homologue thereof, together with a suitable carrier, diluent or excipient, for use as a growth regulator, preferably as a growth promoter.

Performance of the methods according to the present invention result in plants having increased yield, as described hereinbefore. These advantageous growth characteristics may also be combined with other economically advantageous traits, such as further yield-enhancing traits, tolerance to various stresses, traits modifying various architectural features and/or biochemical and/or physiological features, with the proviso that the sequences represented in GenBank Acc nr U16709 are not used for modifying salt tolerance of a plant or for accumulating alkali metals.

### Description of figures

The present invention will now be described with reference to the following figures in which:
**Fig. 1** Schematic representation of an HKT protein (Maser et al., 2002). The transmembrane domains are labelled with roman numerals I to VIII and the four pore forming domains are indicated with the letters A to D. The alignment shows the pore forming domain A of various plant HKTs compared with Trk1 from *S. cerevisiae* (M21328), TrkH from *Pseudomonas aeruginosa* (AAG06598), KtrB from *Vibrio alginolyticus* (BAA32063), and to the pore forming domain of the *Drosophila* Shaker channel (S00479). The residue corresponding to the first glycine of the K+ channel GYG motif is marked with an asterisk.
**Fig. 2** Multiple alignment of various HKT protein sequences. Database accession numbers are given, abbreviations used: At, *Arabidopsis thaliana;* Mc, *Mesembryanthemum crystallinum*; Ec, *Eucalyptus camaldulensis*; Os, *Oryza sativa.* JU0466 represents the TRK1 sequence from *Saccharomyces uvarum* and CAA82128 is the TRK2 sequence from *Saccharomyces cerevisiae.*

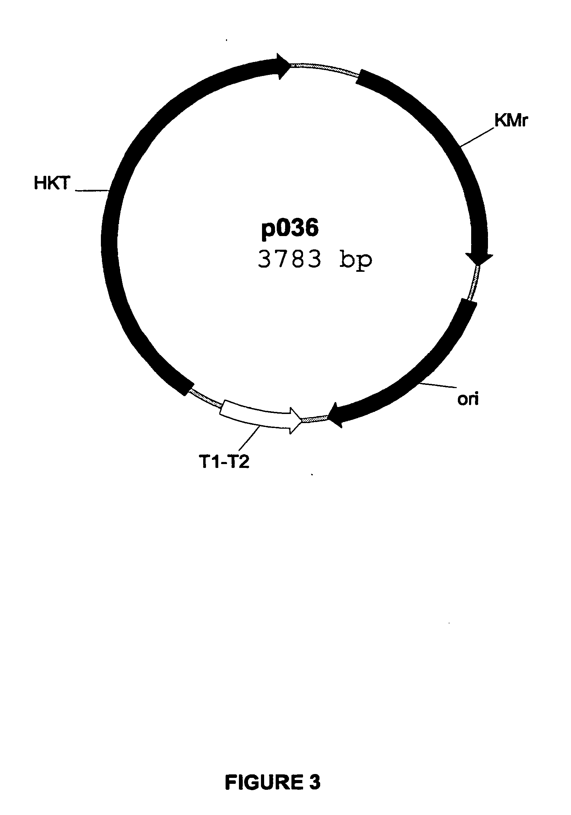

**Fig. 3** Schematic presentation of the entry done p036, containing CDS1532 within the AttL1 and AttL2 sites from Gateway^{®} cloning in the pDONR201 backbone. CDS1532 is the internal code for the *Arabidopsis* HKT encoding sequence. This vector contains also a bacterial kanamycin-resistance cassette and a bacterial origin of replication.

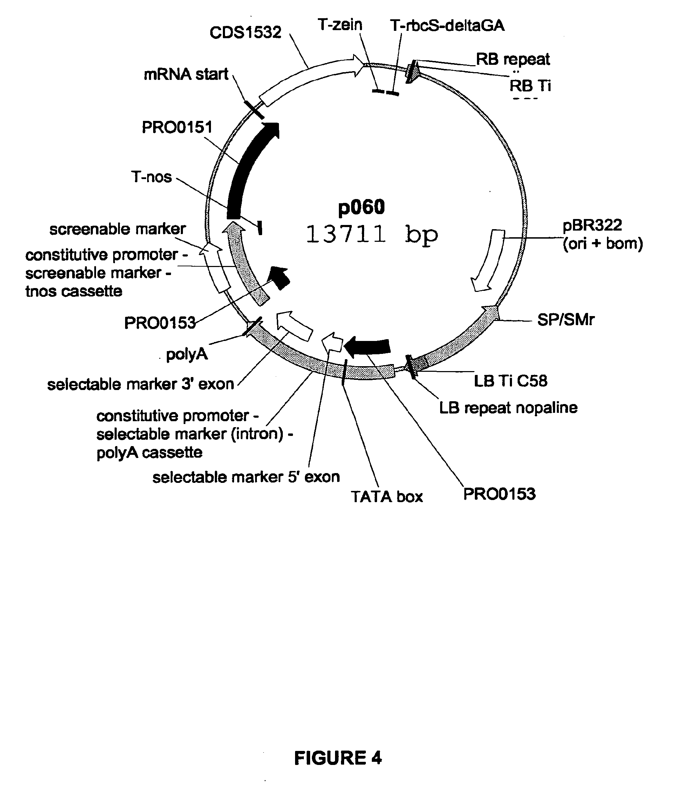

**Fig. 4** shows a binary vector for expression in *Oryza sativa* of the *Arabidopsis HKT* gene (internal reference CDS1532) under the control of the rice WSI18 promoter (internal reference PRO0151). This vector contains T-DNA derived from the Ti Plasmid, limited by a left border (LB repeat, LB Ti C58) and a right border (RB repeat, RB Ti C58)). From the left border to the right border, this T-DNA contains: a cassette for antibiotic selection of transformed plants; a constitutive promoter-selectable marker-NOS terminator cassette for visual screening of transformed plants; the PRO0151-CDS1532 construct-zein and rbcS-deltaGA double terminator cassette for expression of the *Arabidopsis HKT* gene. This vector also contains an origin of replication from pBR322 for bacterial replication and a selectable marker (Spe/SmeR) for bacterial selection with spectinomycin and streptomycin.

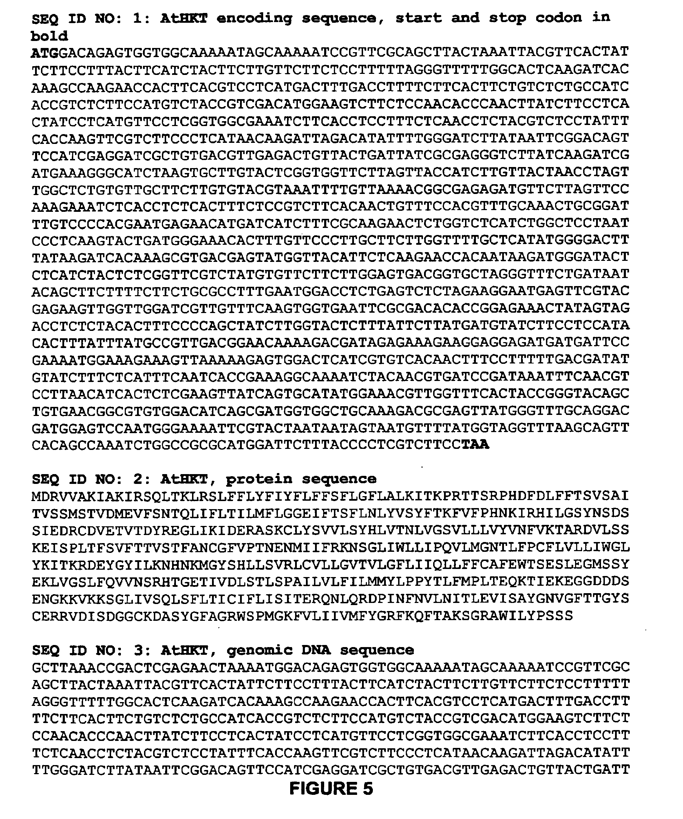

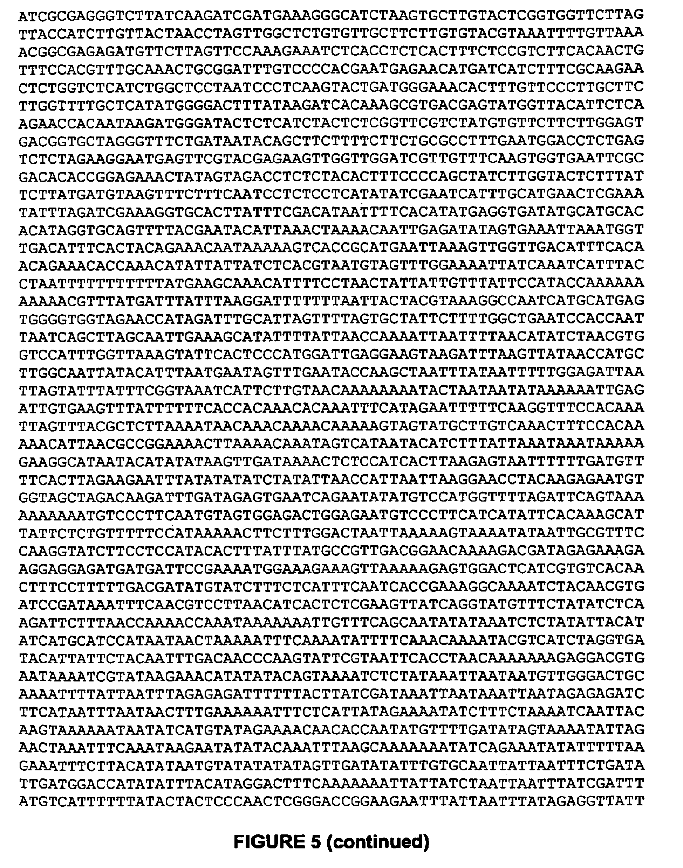

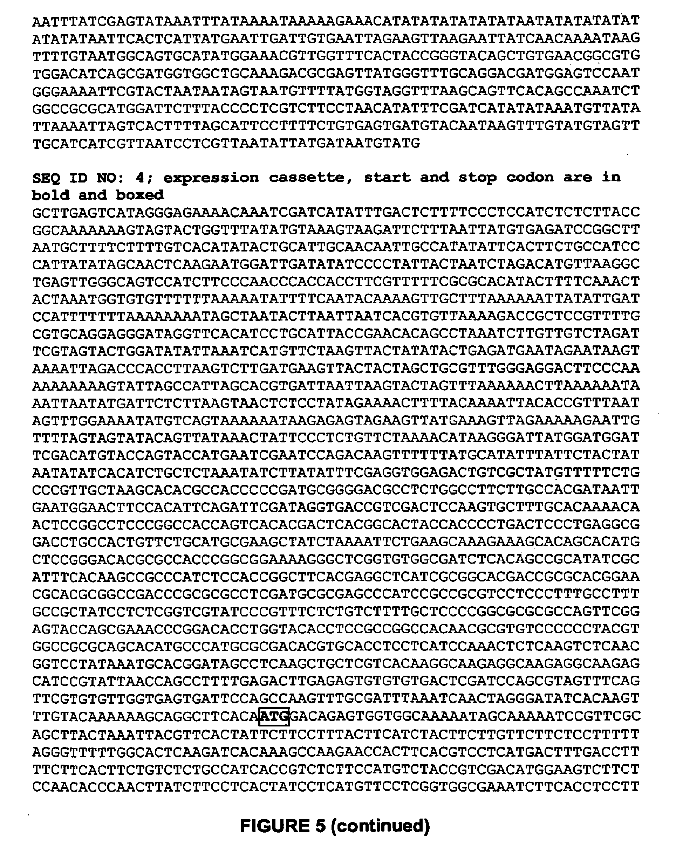

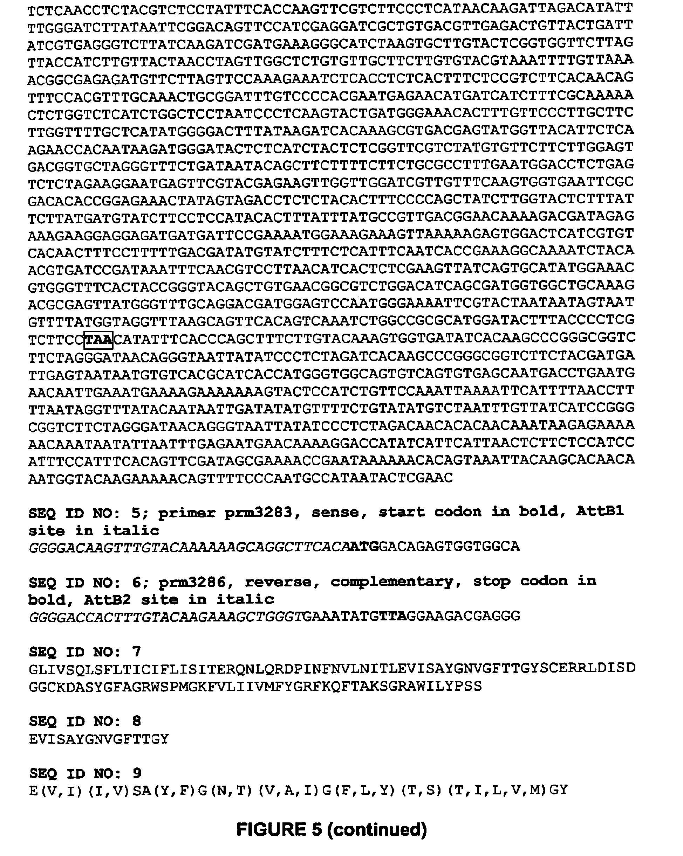

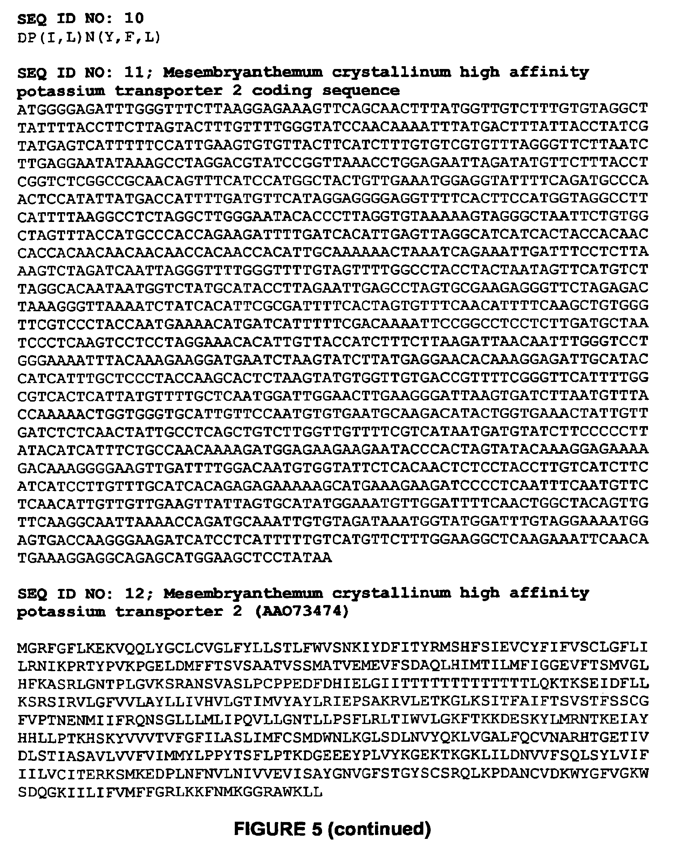

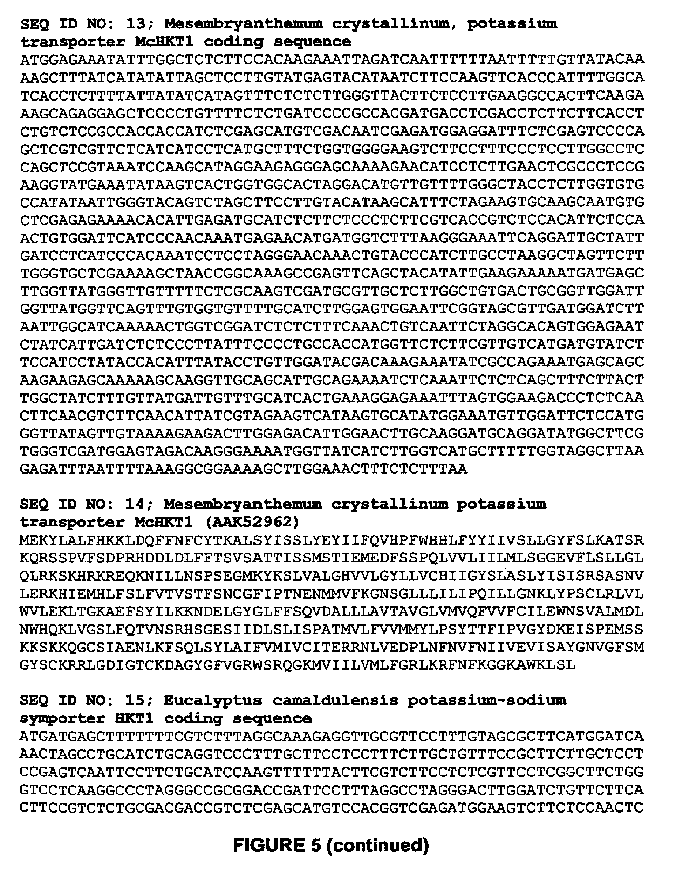

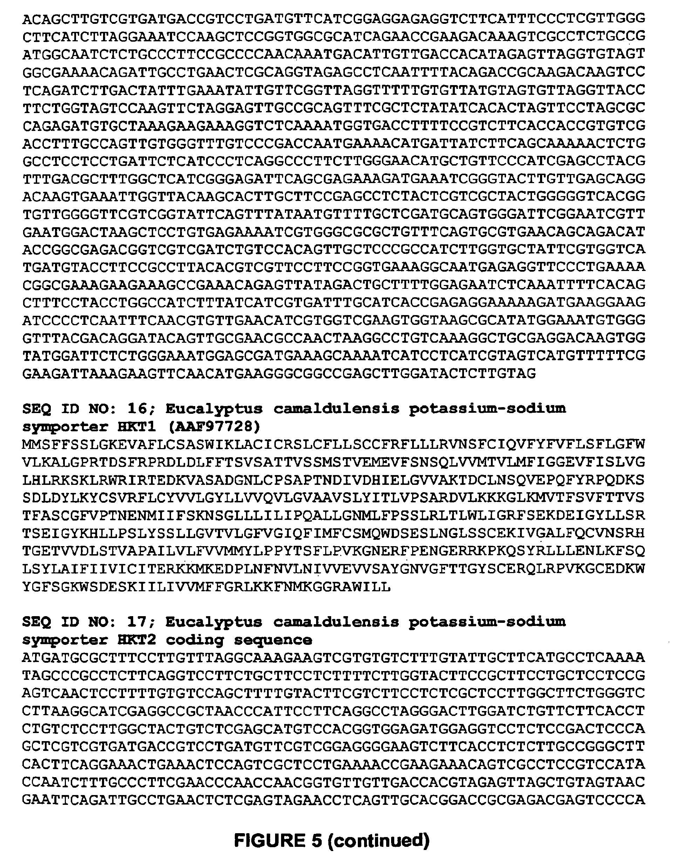

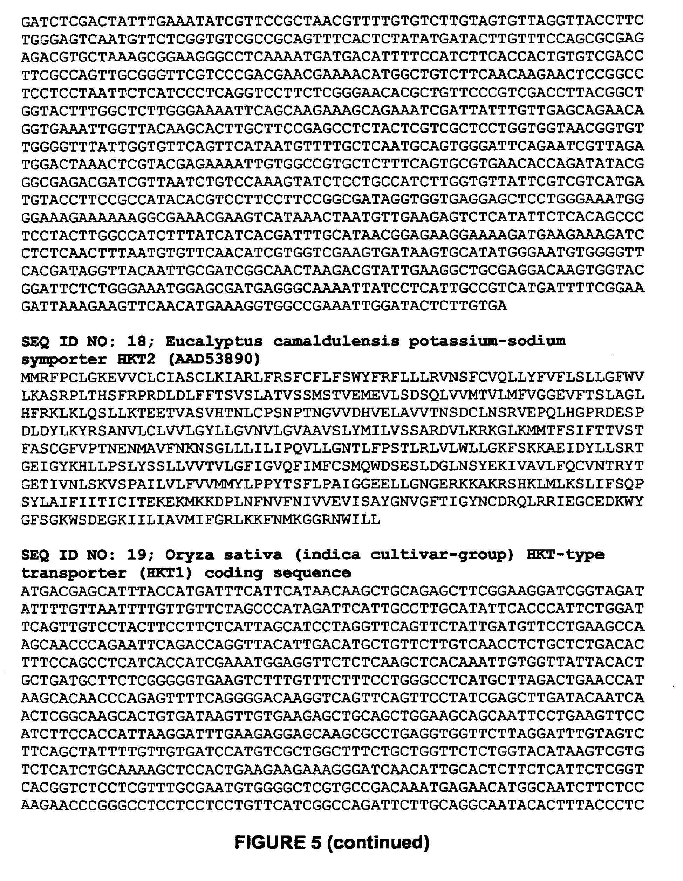

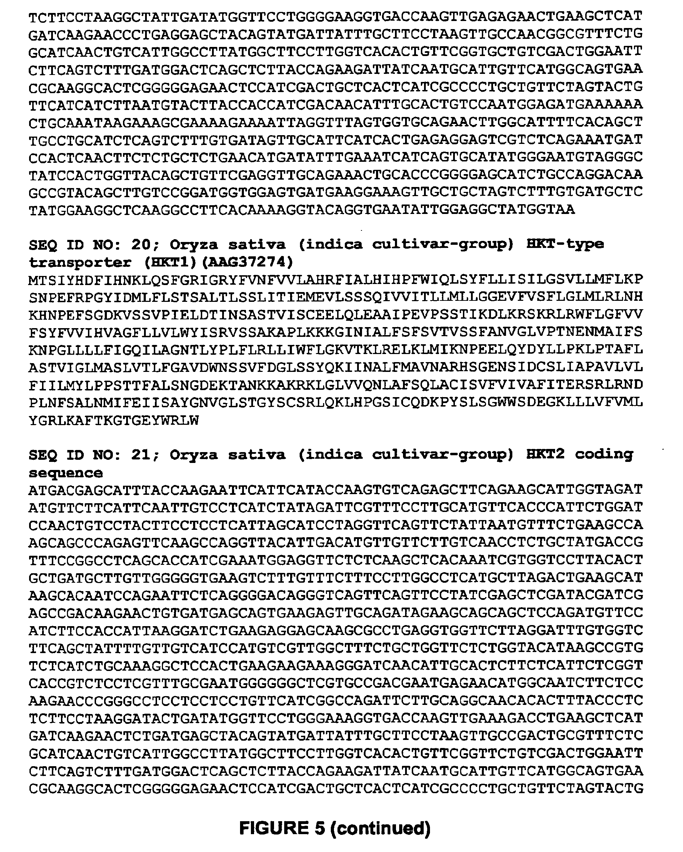

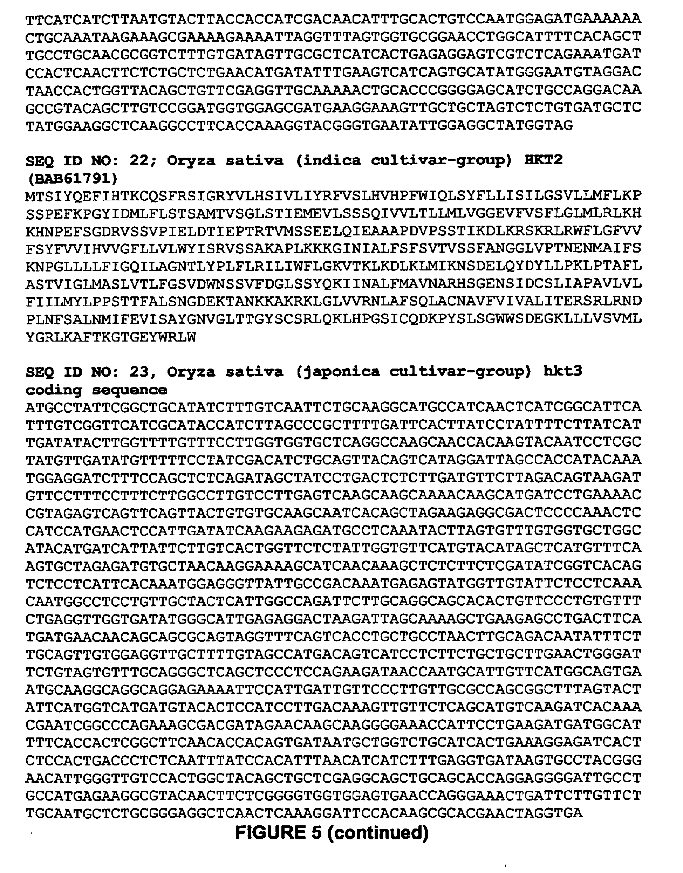

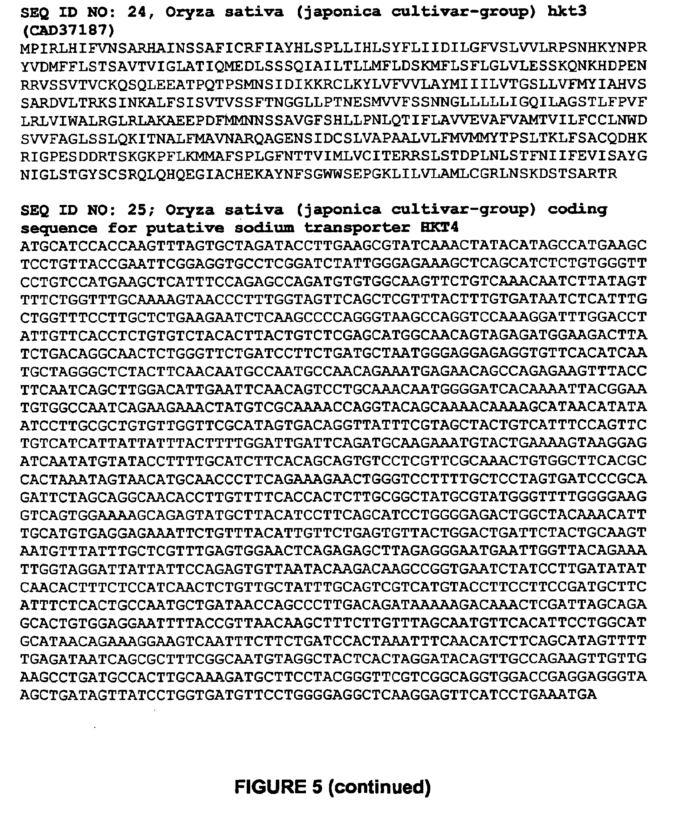

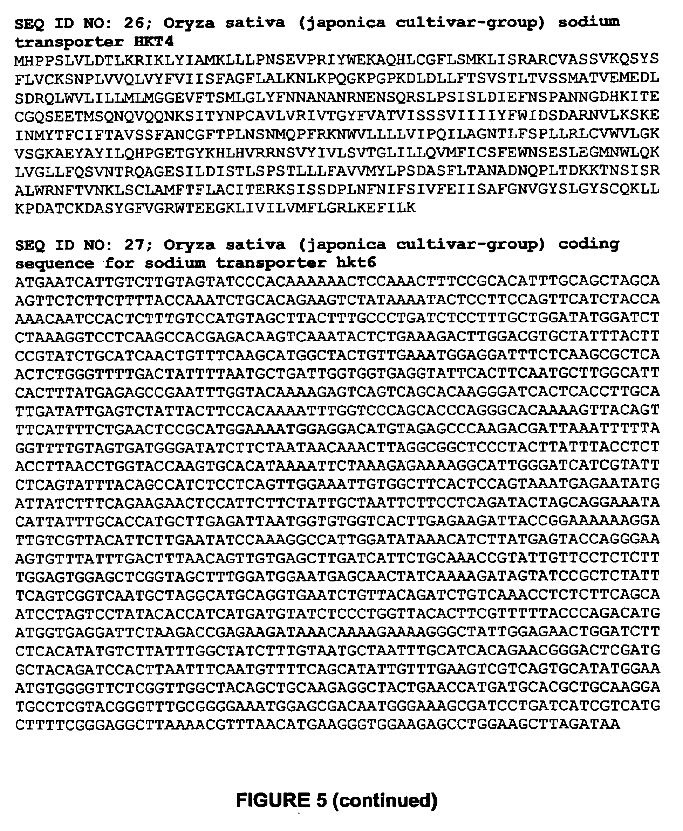

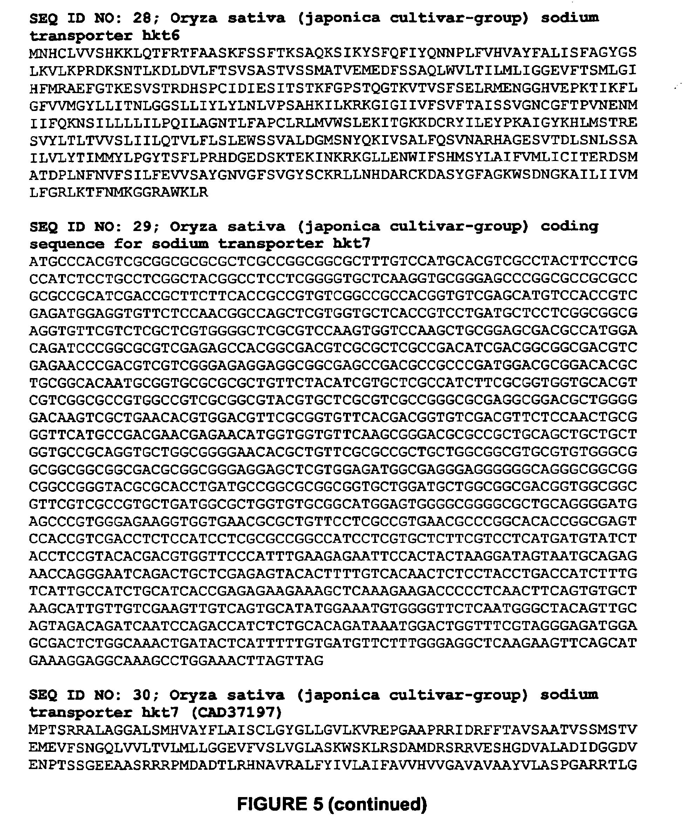

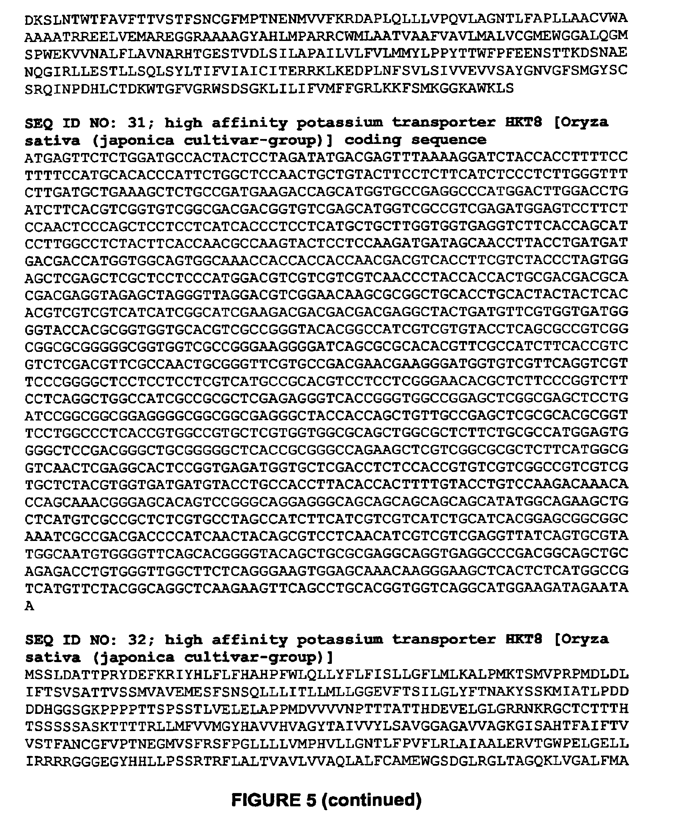

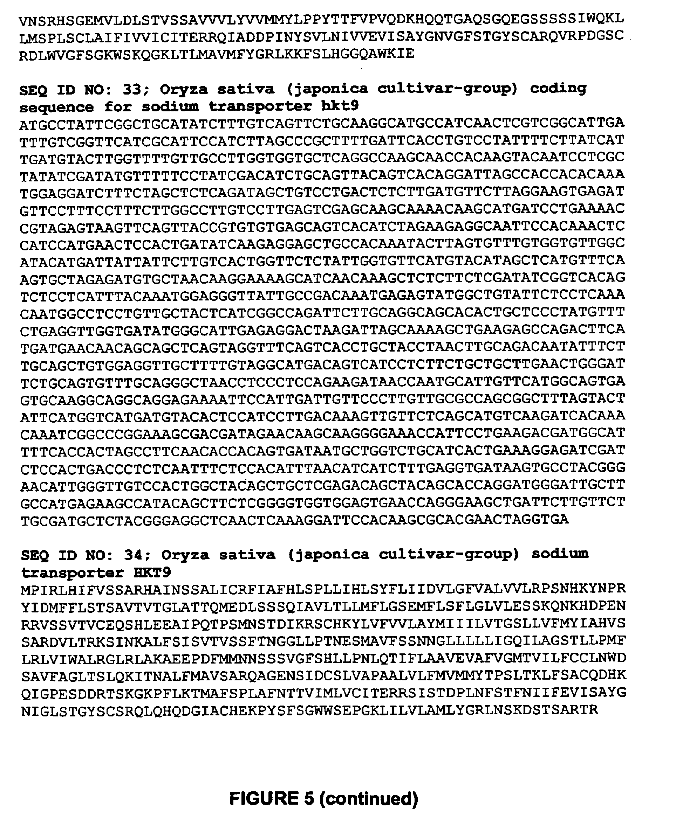

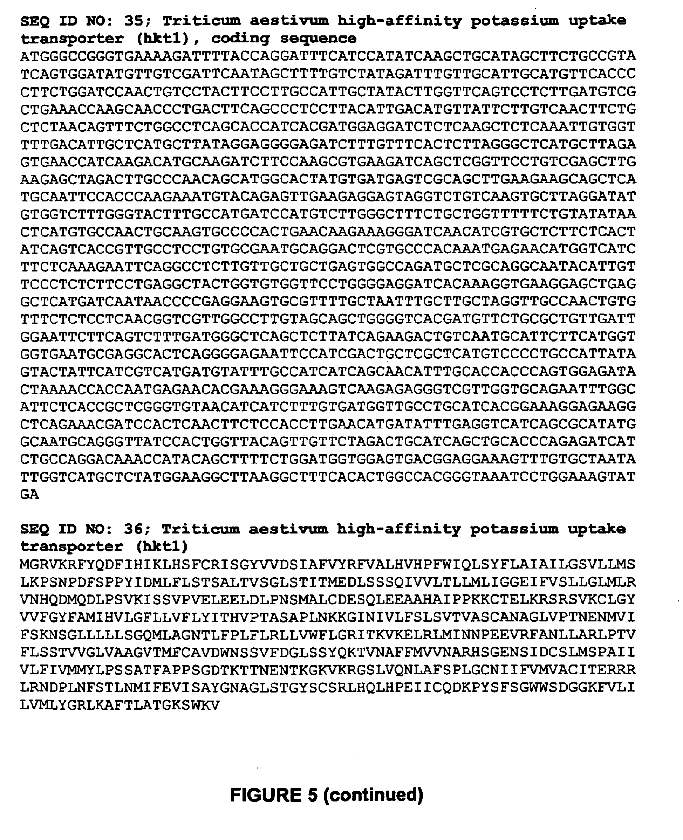

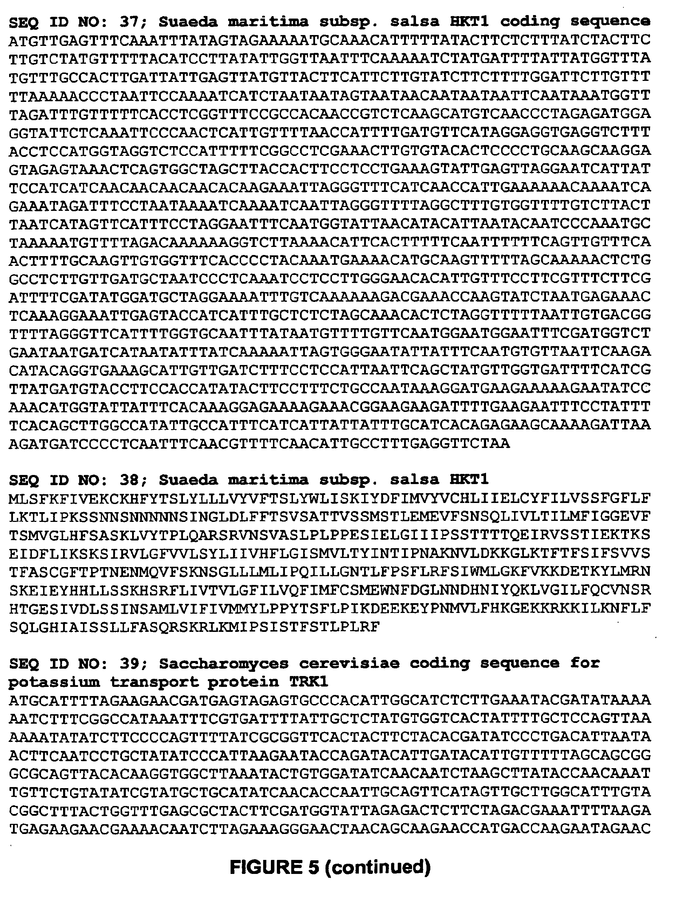

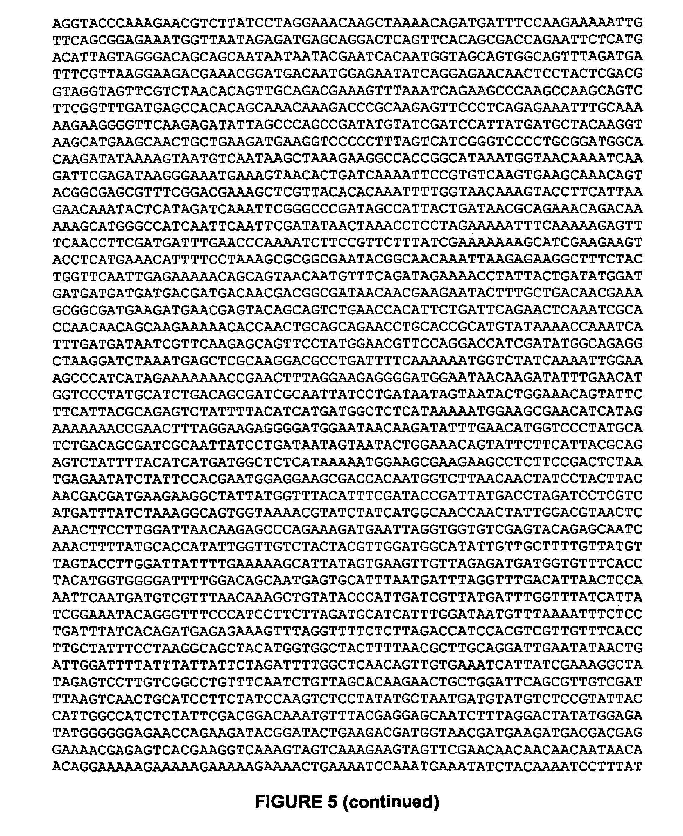

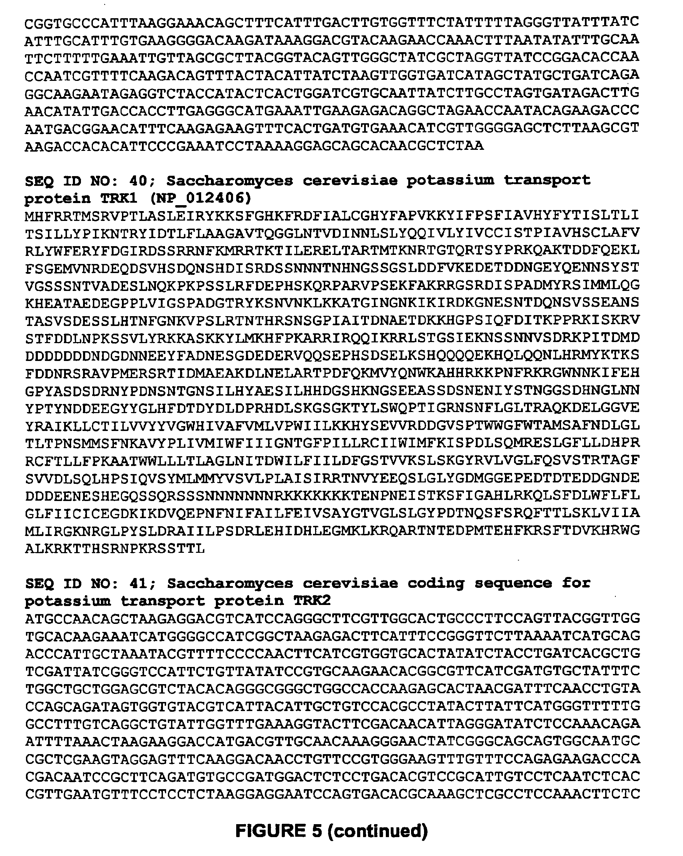

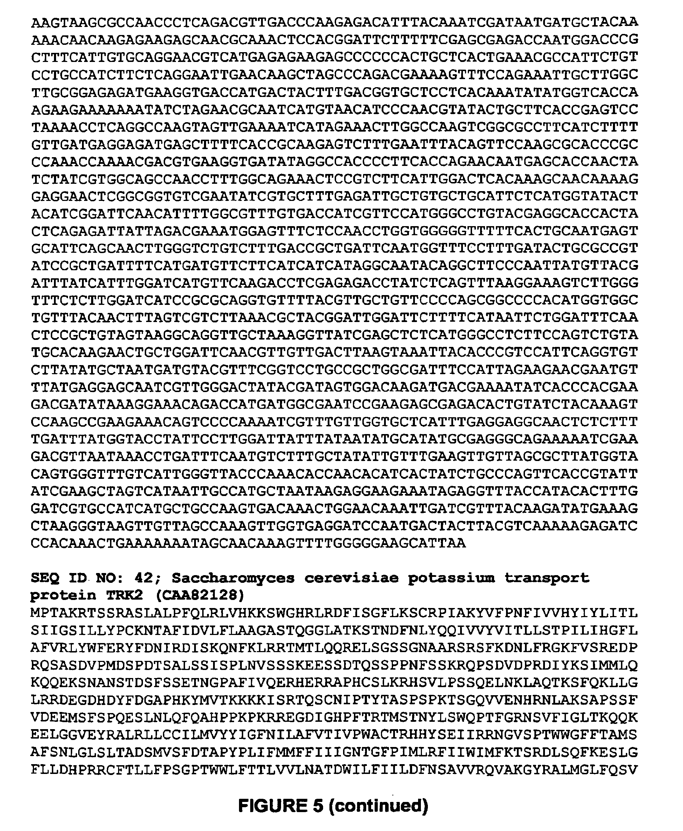

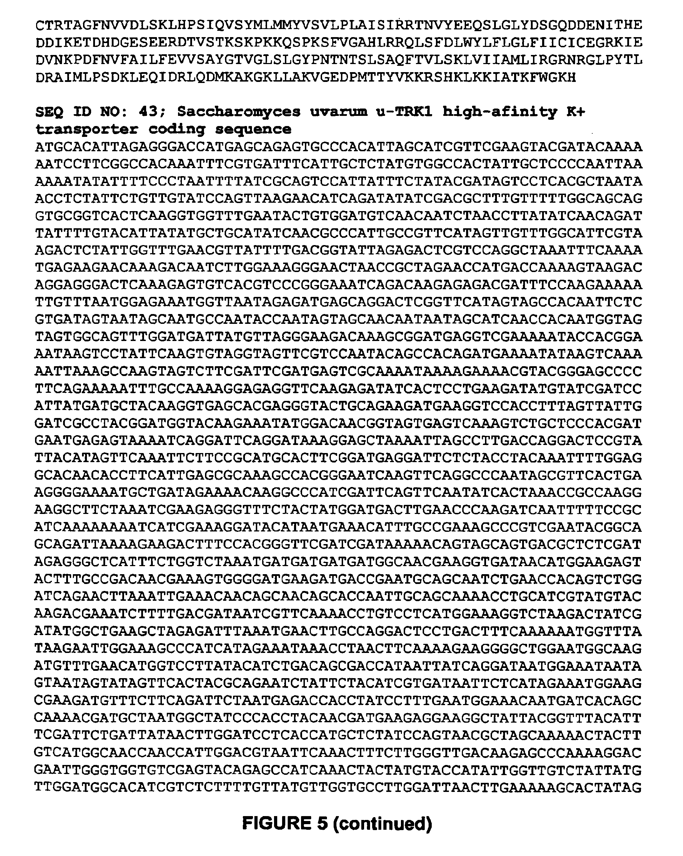

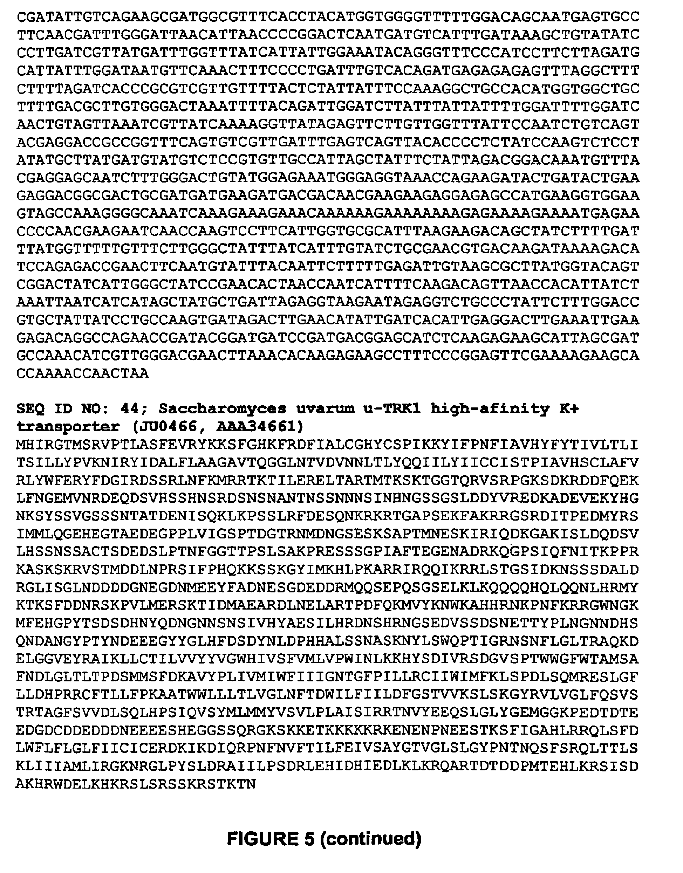

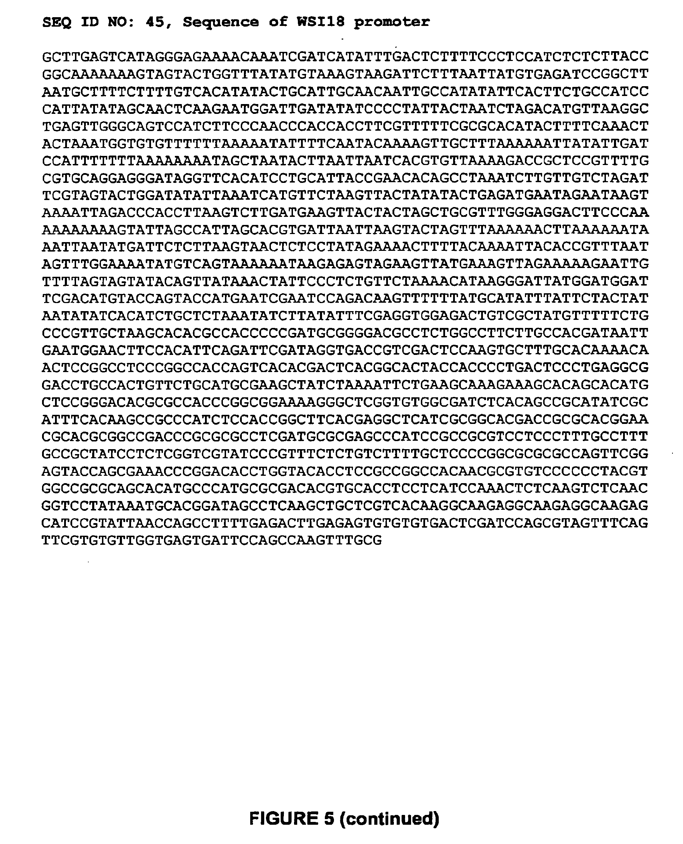

**Fig. 5** details examples of sequences useful in performing the methods according to the present invention.

### Examples

The present invention will now be described with reference to the following examples, which are by way of illustration alone.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

### Example 1: Gene Cloning

The *Arabidopsis* HKT (internal reference CDS1532) was amplified by PCR using as template an *Arabidopsis thaliana* seedling cDNA library (Invitrogen, Paisley, UK). After reverse transcription of RNA extracted from seedlings, the cDNAs were cloned into pCMV Sport 6.0. Average insert size of the bank was 1.5 kb, and original number of clones was of 1.59x10⁷ cfu. The original titer was determined to be 9.6x10⁵ cfu/ml, and became after a first amplification 6x10¹¹ cfu/ml. After plasmid extraction, 200 ng of template was used in a 50 µl PCR mix. Primers prm3283 (SEQ ID NO: 5) and prm8443 (SEQ ID NO: 6), which include the AttB sites for Gateway recombination, were used for PCR amplification. PCR was performed using Hifi Taq DNA polymerase under standard conditions. A PCR fragment of 1613 bp was amplified and purified also using standard methods. The first step of the Gateway procedure, the BP reaction, was then performed, during which the PCR fragment recombines *in vivo* with the pDONR201 plasmid to produce, according to the Gateway terminology, an "entry clone", p036 (Fig. 3). Plasmid pDONR201 was purchased from Invitrogen, as part of the Gateway^{®} technology.

### Example 2: Vector Construction

The entry done p036 was subsequently used in an LR reaction with p56, a destination vector used for *Oryza sativa* transformation. This vector contains as functional elements within the T-DNA borders: a plant selectable marker; a visual marker, and a Gateway cassette intended for LR *in vivo* recombination with the sequence of interest already cloned in the entry done. A rice WSI18 promoter for constitutive expression (PR00151) was located upstream of this Gateway cassette. After the LR recombination step, the resulting expression vector p060 (Fig. 4) comprising the expression cassette of SEQ ID NO: 4 can be transformed into the *Agrobacterium* strain LBA4404 and subsequently to *Oryza sativa* plants. Transformed rice plants were allowed to grow and were then examined for the parameters described in Example 3.

### Example 3: Evaluation of transformants:

Approximately 15 to 20 independent T0 transformants were generated. The primary transformants were transferred from tissue culture chambers to a greenhouse for growing and harvest of T1 seed. Five events of which the T1 progeny segregated 3:1 for presence/absence of the transgene were retained. For each of these events, 10 T1 seedlings containing the transgene (hetero- and homo-zygotes), and 10 T1 seedlings lacking the transgene (nullizygotes), were selected by visual marker screening. The selected T1 plants were transferred to a greenhouse. Each plant received a unique barcode label to link unambiguously the phenotyping data to the corresponding plant. The selected T1 plants were grown on soil in 10 cm diameter pots under the following environmental settings: photoperiod= 11.5 h, daylight intensity= 30,000 lux or more, daytime temperature= 28°C or higher, night time temperature= 22°C, relative humidity= 60-70%. Transgenic plants and the corresponding nullizygotes were grown side-by-side at random positions. From the stage of sowing until the stage of maturity, the plants were passed several times through a digital imaging cabinet. At each time point digital images (2048x1536 pixels, 16 million colours) were taken of each plant from at least 6 different angles.

The mature primary panicles were harvested, bagged, barcode-labelled and then dried for three days in the oven at 37°C. The panicles were then threshed and all the seeds collected. The filled husks were separated from the empty ones using an air-blowing device. After separation, both seed lots were then counted using a commercially available counting machine. The empty husks were discarded. The filled husks were weighed on an analytical balance and the cross-sectional area of the seeds was measured using digital imaging. This procedure resulted in the set of seed-related parameters described below.

These parameters were derived in an automated way from the digital images using image analysis software and were analysed statistically. A two factor ANOVA (analyses of variance) corrected for the unbalanced design was used as statistical model for the overall evaluation of plant phenotypic characteristics. An F-test was carried out on all the parameters measured of all the plants of all the events transformed with that gene. The F-test was carried out to check for an effect of the gene over all the transformation events and to verify for an overall effect of the gene, also named herein "global gene effect". If the value of the F test shows that the data are significant, than it is concluded that there is a "gene" effect, meaning that not only presence or the position of the gene is causing the effect The threshold for significance for a true global gene effect is set at 5% probability level for the F test.

To check for an effect of the genes within an event, i.e., for a line-specific effect, a t-test was performed within each event using data sets from the transgenic plants and the corresponding null plants. "Null plants" or null segregants" or "nullizygotes" are the plants treated in the same way as the transgenic plant, but from which the transgene has segregated. Null plants can also be described as the homozygous negative transformed plants. The threshold for significance for the t-test is set at 10% probability level. The results for some events can be above or below this threshold. This is based on the hypothesis that a gene might only have an effect in certain positions in the genome, and that the occurrence of this position-dependent effect is not uncommon. This kind of gene effect is also named herein a "line effect of the gene". The p-value is obtained by comparing the t-value to the t-distribution or alternatively, by comparing the F-value to the F-distribution. The p-value then gives the probability that the null hypothesis (i.e., that there is no effect of the transgene) is correct.

The data obtained in the first experiment were confirmed in a second experiment with T2 plants. Three lines that had the correct expression pattern were selected for further analysis. Seed batches from the positive plants (both hetero- and homozygotes) in T1, were screened by monitoring marker expression. For each chosen event, the heterozygote seed batches were then retained for T2 evaluation. Within each seed batch an equal number of positive and negative plants were grown in the greenhouse for evaluation.

A total number of 120 *HKT* transformed plants were evaluated in the T2 generation, that is 30 plants per event of which 15 positives for the transgene, and 15 negatives.

Because two experiments with overlapping events have been carried out, a combined analysis was performed. This is useful to check consistency of the effects over the two experiments, and if this is the case, to accumulate evidence from both experiments in order to increase confidence in the conclusion. The method used was a mixed-model approach that takes into account the multilevel structure of the data (i.e. experiment - event - segregants). P-values are obtained by comparing likelihood ratio test to chi square distributions.

### Example 4: Evaluation of transformants: measurement of yield related parameters

Upon analysis of the seeds as described above, the inventors found that plants transformed with the *AtHKT* gene construct scored better for several yield parameters, including the number of filled seeds, total yield and harvest index when compared to the nullizygous plants. The total seed yield was measured by weighing all filled husks harvested from a plant. The number of filled seeds was determined by counting the number of filled husks that remained after the separation step. The harvest index is defined in the present invention as the ratio between the total seed yield and the above ground area (in mm²).

Four events were selected for an evaluation in T2 plants. The results for increased yield were also present in the T2 generation. The increases for the individual lines varied between 8 to 20% for harvest index, between 12 to 24% for the total weight of seeds and was around 15% for the number of filled seeds.

## Claims

1. Method for increasing yield of a plant, relative to corresponding wild type plants, comprising increasing activity in a plant of a HKT protein and selecting for plants having increased yield relative to a corresponding wild type plant, said HKT protein being selected from the group consisting of
a) a protein having an amino acid sequence as shown in SEQ ID No. 2;
b) a protein having an amino acid sequence being at least 35 % identical to the amino acid sequence as shown in SEQ ID No. 2 having cation transporter activity;
c) a protein having an amino acid sequence being at least 35 % identical to the amino acid sequence as shown in SEQ ID No. 2 having cation transporter activity and comprising a consensus sequence as shown in SEQ ID No. 9;
d) a protein encoded by the nucleic acid sequence shown in SEQ ID No. 1; and
e) a protein encoded by a nucleic acid sequence which hybridizes under stringent conditions to the nucleic acid sequence as shown in SEQ ID No. 1, wherein the protein has cation transporter activity.

2. Method of claim 1, wherein said increased activity is effected by introducing a genetic modification in the locus of a gene encoding the HKT protein.

3. Method of claim 2, wherein said genetic modification is effected by on of site-directed mutagenesis, directed evolution, homologous recombination, TILLING and T-DNA activation.

4. Method for increasing yield of a plant relative to corresponding wild type plants, comprising introducing and expressing in a plant an HKT nucleic acid molecule being selected from the group consisting of:
a) a nucleic acid molecule having a sequence as shown in SEQ ID No.1;
b) a nucleic acid molecule being capable of hybridizing under stringent conditions to the nucleic acid sequence shown in SEQ ID No. 1, when said nucleic acid molecule encodes a protein having cation transporter activity.

5. Method according to claim 4, wherein said HKT nucleic acid molecule is operably linked to a seed specific promoter.

6. Method according to claim 4 or 5, wherein said HKT nucleic acid molecule is operably linked to an embryo- and/or aleurone-specific promoter.

7. Method according to claim 5 or 6, wherein said promoter has a comparable expression profile to a WSI18 promotor.

8. Method according to any one of claims 1 to 7 wherein said increased yield is increased biomass and/or increased seed yield.

9. Method according to claim 8, wherein said increased seed yield is selected from one of more of (i) increased seed biomass; (ii) increased number of seeds; (iii) increased number of filled seeds; (iv) increased seed size; (v) increased seed volume; (vi) increased harvest index (HI); and (vii) increased thousand kernel weight (TKW).

10. Method for the production of a transgenic plant having increased yield which method comprises:
(i) introduction into a plant a nucleic acid molecule as defined in claim 4;
(ii) cultivating the plant cell under conditions promoting plant growth and development.

11. Use of a nucleic acid molecule as defined in claim 4 or a protein as defined in claim 1 for increasing yield of plants.

12. Use according to claim 11, wherein said increased yield comprises at least increased number of filled seeds.

13. Use of a nucleic acid molecule as defined in claim 4 as a molecular marker for identifying plants exhibiting increased yield.

## Patentansprüche

1. Verfahren zum Steigern des Ertrags einer Pflanze gegenüber den entsprechenden Wildtyp-Pflanzen, umfassend das Steigern der Aktivität eines HKT-Proteins in einer Pflanze und Selektieren auf Pflanzen mit erhöhtem Ertrag gegenüber einer entsprechenden Wildtyp-Pflanze, wobei das HKT-Protein aus der Gruppe ausgewählt ist, die aus Folgendem besteht:
a) einem Protein mit einer Aminosäuresequenz, wie sie in SEQ ID NO: 2 gezeigt ist;
b) einem Protein mit einer Aminosäuresequenz, die zu der in SEQ ID NO: 2 gezeigten Aminosäuresequenz mindestens zu 35% identisch ist, und Kationentransporteraktivität aufweist;
c) einem Protein mit einer Aminosäuresequenz, die zu der in SEQ ID NO: 2 gezeigten Aminosäuresequenz mindestens zu 35% identisch ist, und Kationentransporteraktivität aufweist und eine Konsensussequenz, wie sie in der SEQ ID NO: 9 gezeigt ist, umfasst;
d) einem Protein, das von der in SEQ ID NO: 1 gezeigten Nukleinsäuresequenz codiert wird, und
e) einem Protein, das von der Nukleinsäuresequenz codiert wird, die unter stringenten Bedingungen an die in SEQ ID NO: 1 gezeigte Nukleinsäuresequenz hybridisiert, wobei das Protein Kationentransporteraktivität hat.

2. Verfahren nach Anspruch 1, wobei die erhöhte Aktivität durch Einbringen einer genetischen Modifikation in den Locus eines Gens, das das HKT-Protein codiert, herbeigeführt wird.

3. Verfahren nach Anspruch 2, wobei die genetische Modifikation durch ein Verfahren aus ortsgerichteter Mutagenese, gerichteter Evolution, homologer Rekombination, TILLING und T-DNA-Aktivierung herbeigeführt wird.

4. Verfahren zum Steigern des Ertrags einer Pflanze gegenüber den entsprechenden Wildtyp-Pflanzen, umfassend das Einbringen und Exprimieren eines HKT-Nukleinsäuremoleküls, ausgewählt aus der Gruppe, bestehend aus:
a) einem Nukleinsäuremolekül mit einer Sequenz, wie sie in der SEQ ID NO: 1 gezeigt ist;
b) ein Nukleinsäuremolekül, das unter stringenten Bedingungen an die in SEQ ID NO: 1 gezeigte Nukleinsäuresequenz hybridisieren kann, wenn das Nukleinsäuremolekül ein Protein mit Kationentransporteraktivität codiert,
in einer Pflanze.

5. Verfahren nach Anspruch 4, wobei das HKT-Nukleinsäuremolekül funktionsfähig mit einem samenspezifischen Promotor verbunden ist.

6. Verfahren nach Anspruch 4 oder 5, wobei das HKT-Nukleinsäuremolekül funktionsfähig mit einem embryo- und/oder aleuronspezifischen Promotor verbunden ist.

7. Verfahren nach Anspruch 5 oder 6, wobei der Promotor ein vergleichbares Expressionsprofil wie ein WSI18-Promotor hat.

8. Verfahren nach einem der Ansprüche 1 bis 7, wobei der erhöhte Ertrag eine erhöhte Biomasse und/oder ein erhöhter Samenertrag ist.

9. Verfahren nach Anspruch 8, wobei der erhöhte Samenertrag aus einem oder mehreren aus (i) erhöhter Samenbiomasse; (ii) erhöhter Anzahl Samen; (iii) erhöhter Anzahl gefüllter Samen; (iv) erhöhter Samengröße; (v) erhöhtem Samenvolumen; (vi) erhöhtem Ernteindex (HI) und (vii) erhöhtem Tausendkorngewicht (TKW) ausgewählt ist.

10. Verfahren zur Produktion einer transgenen Pflanze mit erhöhtem Ertrag, wobei das Verfahren umfasst:
(i) Einbringen eines Nukleinsäuremoleküls nach Anspruch 4 in eine Pflanze;
(ii) Züchten der Pflanzenzelle unter Bedingungen, die das Pflanzenwachstum und die Entwicklung fördern.

11. Verwendung eines Nukleinsäuremoleküls nach Anspruch 4 oder eines Proteins nach Anspruch 1 zum Steigern des Ertrags von Pflanzen.

12. Verwendung nach Anspruch 11, wobei der erhöhte Ertrag mindestens eine erhöhte Anzahl gefüllter Samen umfasst.

13. Verwendung eines Nukleinsäuremoleküls nach Anspruch 4 als molekularen Marker zum Identifizieren von Pflanzen, die einen erhöhten Ertrag aufweisen.

## Revendications

1. Méthode d'augmentation du rendement d'une plante par rapport aux plantes de type sauvage correspondantes, comprenant l'augmentation de l'activité dans une plante d'une protéine HKT et la sélection de plantes ayant un rendement accru par rapport à une plante de type sauvage correspondante, ladite protéine HKT étant choisie dans le groupe constitué par .
a) une protéine ayant une séquence d'acides aminés telle qu'illustrée dans SEQ ID n°2 ;
b) une protéine ayant une séquence d'acides aminés ayant une identité d'au moins 35% par rapport à la séquence d'acides aminés telle qu'illustrée dans SEQ ID n°2 ayant une activité de transporteur de cations ;
c) une protéine ayant une séquence d'acides aminés ayant une identité d'au moins 35% par rapport à la séquence d'acides aminés telle qu'illustrée dans SEQ ID n°2 ayant une activité de transporteur de cations et comprenant une séquence consensus telle qu'illustrée dans SEQ ID n°9 ;
d) une protéine codée par une séquence d'acide nucléique illustrée dans SEQ ID n°1 ; et
e) une protéine codée par une séquence d'acide nucléique qui s'hybride dans des conditions stringentes à la séquence d'acide nucléique telle qu'illustrée dans SEQ ID n°1, où la protéine possède une activité de transporteur de cations.

2. Méthode selon la revendication 1, dans laquelle ladite activité accrue est obtenue par l'introduction d'une modification génétique dans le locus d'un gène codant pour la protéine HKT.

3. Méthode selon la revendication 2, dans laquelle ladite modification génétique est obtenue par mutagenèse dirigée, évolution dirigée, recombinaison homologue, TILLING ou bien activation d'ADN-T.

4. Méthode d'augmentation du rendement d'une plante par rapport aux plantes de type sauvage correspondantes, comprenant l'introduction et l'expression dans une plante d'une molécule d'acide nucléique HKT étant choisie dans le groupe constitué par :
a) une molécule d'acide nucléique ayant une séquence telle qu'illustrée dans SEQ ID n°1 ;
a) une molécule d'acide nucléique capable de s'hybrider dans des conditions stringentes à la séquence d'acide nucléique illustrée dans SEQ ID n°1, où ladite molécule d'acide nucléique code pour une protéine possédant une activité de transporteur de cations.

5. Méthode selon la revendication 4, dans laquelle ladite molécule d'acide nucléique HKT est liée de manière opérante à un promoteur spécifique des graines.

6. Méthode selon la revendication 4 ou 5, dans laquelle ladite molécule d'acide nucléique HKT est liée de manière opérante à un promoteur spécifique de l'embryon et/ou de l'aleurone.

7. Méthode selon la revendication 5 ou 6, dans laquelle ledit promoteur possède un profil d'expression comparable à un promoteur WSI 18.

8. Méthode selon l'une quelconque des revendications 1 à 7, dans laquelle ledit rendement accru est une biomasse accrue et/ou un rendement accru en graines.

9. Méthode selon la revendication 8, dans laquelle ledit rendement accru en graines est choisi parmi un ou plusieurs parmi (i) une biomasse accrue en graines ; (ii) un nombre accru de graines ; (iii) un nombre accru de graines remplies ; (iv) une taille accrue des graines ; (v) un volume accru des graines ; (vi) un indice de moisson accru ; et (vii) un poids de mille grains accru.

10. Méthode de production d'une plante transgénique ayant un rendement accru, laquelle méthode comprend .
i) l'introduction dans une plante d'une molécule d'acide nucléique telle que définie selon la revendication 4 ;
ii) la culture de la cellule végétale dans des conditions favorisant la croissance et le développement de la plante.

11. Utilisation d'une molécule d'acide nucléique telle que définie selon la revendication 4 ou d'une protéine telle que définie selon la revendication 1, pour l'augmentation du rendement de plantes.

12. Utilisation selon la revendication 11, dans laquelle ledit rendement accru comprend au moins 1'augmentation du nombre de graines remplies.

13. Utilisation d'une molécule d'acide nucléique telle que définie selon la revendication 4, comme marqueur moléculaire pour l'identification de plantes présentant un rendement accru.
